# EUROPEAN PATENT APPLICATION

(11) **EP 4 265 619 A1**
(43) Date of publication of application: **25.10.2023**
(21) Application number: 21911280.2
(22) Date of filing: 29.11.2021
(51) Int. Cl.: C07D 495/04, H01L 51/00, H01L 51/50

(54) **HETEROCYCLIC COMPOUND AND ORGANIC LIGHT-EMITTING DEVICE COMPRISING SAME**

(30) Priority: 21.12.2020 KR 20200180272
(71) Applicant: LT Materials Co., Ltd., Yongin-si, Gyeonggi-do 17118 (KR)
(72) Inventor: CHAE, Woo-Jeong, Yongin-si, Gyeonggi-do 17118 (KR); MO, Jun-Tae, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/017701
(87) International publication number: WO 2022/139213

(57) **Abstract**

The present application provides a heterocyclic compound and an organic light emitting device in which the heterocyclic compound is contained in an organic material layer.

## Description

### [Technical Field]

This application claims priority to and the benefits of Korean Patent Application No. 10-2020-0180272, filed with the Korean Intellectual Property Office on December 21, 2020, the entire contents of which are incorporated herein by reference.

The present specification relates to a heterocyclic compound and an organic light emitting device comprising the same.

### [Background Art]

An electroluminescent device is a type of self-emission type display device, and there are advantages in that it not only has a wide viewing angle and excellent contrast, but also has fast response speed.

An organic light emitting device has a structure in which an organic thin film is disposed between two electrodes. When a voltage is applied to the organic light emitting device having such a structure, electrons and holes injected from the two electrodes combine in the organic thin film to form a pair, and then emit light while being disappeared. The organic thin film may be composed of a single layer or multiple layers as needed.

A material of the organic thin film may have a light emitting function as needed. For example, as the organic thin film material, a compound capable of forming the light emitting layer by itself may be used, or a compound capable of serving as a host or dopant of the host-dopant based light emitting layer may also be used. In addition, compounds capable of performing the roles of hole injection, hole transport, electron blocking, hole blocking, electron transport, electron injection, etc. may also be used as a material of the organic thin film.

In order to improve the performance, lifespan, or efficiency of the organic light emitting device, the development of the material of the organic thin film is continuously required.

### Prior Art Documents

### Patent Documents

(Patent Document 1) US Patent No. 4,356,429

### [Disclosure]

### [Technical Problem]

An object of the present specification is to provide a heterocyclic compound and an organic light emitting device comprising the same.

### [Technical Solution]

An embodiment of the present application provides a heterocyclic compound represented by Chemical Formula 1 below.

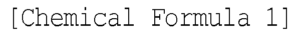

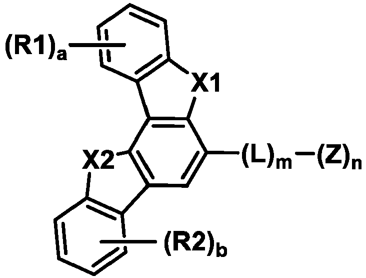

In Chemical Formula 1,
X1 and X2 are each independently O; or S, wherein X1 and X2 are different from each other,
L is a direct bond; a substituted or unsubstituted C₆-C₆₀ arylene group; or a substituted or unsubstituted C₂-C₆₀ heteroarylene group,
Z is a substituted or unsubstituted amine group; or a substituted or unsubstituted C₂-C₆₀ heteroaryl group,
R1 and R2 are the same as or different from each other, and are each independently hydrogen; heavy hydrogen; a cyano group; a nitro group; a substituted or unsubstituted C₁-C₆₀ alkyl group; a substituted or unsubstituted C₁-C₂₀ alkoxy group; a substituted or unsubstituted C₃-C₆₀ cycloalkyl group; a substituted or unsubstituted C₂-C₆₀ alkenyl group; a substituted or unsubstituted C₂-C₆₀ alkynyl group; a substituted or unsubstituted C₁-C₆₀ aryl group; a substituted or unsubstituted C₂-C₆₀ heterocycloalkyl group; a substituted or unsubstituted C₂-C₆₀ heteroaryl group; a substituted or unsubstituted phosphine oxide group; or a substituted or unsubstituted silyl group,
a and b are each independently an integer of 0 to 4, and when a and b are each 2 or more, the substituents in parentheses are the same as or different from each other, and
m and n are each independently an integer of 0 to 6, and when m and n are each 2 or more, the substituents in parentheses are the same as or different from each other.

Furthermore, in an embodiment of the present application, there is provided an organic light emitting device comprising: a first electrode; a second electrode; and an organic material layer with one or more layers provided between the first electrode and the second electrode, wherein one or more layers of the organic material layer comprise the heterocyclic compound represented by Chemical Formula 1 above.

### [Advantageous Effects]

The heterocyclic compound described in the present specification may be used as a material of an organic material layer of an organic light emitting device. The heterocyclic compound may serve as a hole injection material, a hole transport material, a light emitting material, an electron transport material, an electron injection material, etc. in the organic light emitting device.

Specifically, when the heterocyclic compound represented by Chemical Formula 1 above is used in the organic material layer of the organic light emitting device, it is possible to lower the driving voltage of the device, improve the light efficiency, and improve the lifespan characteristics of the device.

### [Description of Drawings]

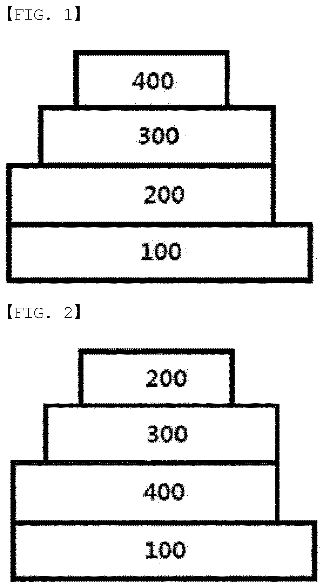

FIGS. 1 to 4 are diagrams each exemplarily illustrating a lamination structure of an organic light emitting device according to an embodiment of the present application.

### [Mode for Disclosure]

Hereinafter, the present specification will be described in more detail.

In the present specification, if a prescribed part "includes" a prescribed element, this means that another element can be further included instead of excluding other elements unless any particularly opposite description exists.

In the present specification, 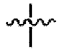 in the chemical formula means a bonding position.

The term "substitution" means that a hydrogen atom bonded to a carbon atom of a compound is changed to another substituent, and the position to be substituted is not limited as long as it is a position at which the hydrogen atom is substituted, that is, a position where the substituent is substitutable, and when two or more substituents are substituted, two or more substituents may be the same as or different from each other.

In the present specification, "substituted or unsubstituted" means that it is substituted or unsubstituted with one or more substituents selected from the group consisting of: a C₁-C₆₀ linear or branched alkyl group; a C₂-C₆₀ linear or branched alkenyl group; a C₂-C₆₀ linear or branched alkynyl group; a C₃-C₆₀ monocyclic or polycyclic cycloalkyl group; a C₂-C₆₀ monocyclic or polycyclic heterocycloalkyl group; a C₆-C₆₀ monocyclic or polycyclic aryl group; a C₂-C₆₀ monocyclic or polycyclic heteroaryl group; a silyl group; a phosphine oxide group; and an amine group, or is substituted or unsubstituted with a substituent to which two or more substituents selected from the above-exemplified substituents are connected.

More specifically, in the present specification, "substituted or unsubstituted" may mean that it is substituted or unsubstituted with one or more substituents selected from the group consisting of: a monocyclic or polycyclic C₆-C₆₀ aryl group; and a monocyclic or polycyclic C₂-C₆₀ heteroaryl group.

In the present specification, the halogen may be fluorine, chlorine, bromine, or iodine.

In the present specification, the alkyl group may include a linear or branched chain having 1 to 60 carbon atoms, and may be further substituted by other substituents. The number of carbon atoms of the alkyl group may be 1 to 60, specifically 1 to 40, and more specifically 1 to 20. Specific examples of the alkyl group may include a methyl group, an ethyl group, a propyl group, an n-propyl group, an isopropyl group, a butyl group, an n-butyl group, an isobutyl group, a tert-butyl group, a sec-butyl group, a 1-methylbutyl group, an 1-ethyl-butyl group, a pentyl group, an n-pentyl group, an isopentyl group, a neopentyl group, a tert-pentyl group, a hexyl group, an n-hexyl group, an 1-methylpentyl group, a 2-methylpentyl group, a 4-methyl-2-pentyl group, a 3,3-dimethylbutyl group, a 2-ethylbutyl group, a heptyl group, an n-heptyl group, an 1-methylhexyl group, a cyclopentylmethyl group, a cyclohexylmethyl group, an octyl group, an n-octyl group, a tert-octyl group, an 1-methylheptyl group, a 2-ethylhexyl group, a 2-propylpentyl group, an n-nonyl group, a 2,2-dimethylheptyl group, an 1-ethyl-propyl group, an 1,1-dimethyl-propyl group, an isohexyl group, a 2-methylpentyl group, a 4-methylhexyl group, a 5-methylhexyl group, etc., but the present application is not limited thereto.

In the present specification, a haloalkyl group refers to an alkyl group substituted with a halogen group, and specific examples of the haloalkyl group may include -CF₃, -CF₂CF₃, etc., but the present application is not limited thereto.

In the present specification, the alkenyl group may include a linear or branched chain having 2 to 60 carbon atoms, and may be further substituted by other substituents. The number of carbon atoms of the alkenyl group may be 2 to 60, specifically 2 to 40, and more specifically 2 to 20. Specific examples of the alkenyl group may include a vinyl group, an 1-propenyl group, an isopropenyl group, an 1-butenyl group, a 2-butenyl group, a 3-butenyl group, an 1-pentenyl group, a 2-pentenyl group, a 3-pentenyl group, a 3-methyl-1-butenyl group, an 1,3-butadienyl group, an allyl group, an 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, etc., but the present application is not limited thereto.

In the present specification, the alkynyl group may include a linear or branched chain having 2 to 60 carbon atoms, and may be further substituted by other substituents. The number of carbon atoms of the alkynyl group may be 2 to 60, specifically 2 to 40, and more specifically 2 to 20.

In the present specification, the alkoxy group may be a linear, branched, or cyclic chain. The number of carbon atoms of the alkoxy group is not particularly limited, but is preferably 1 to 20. Specific examples of the alkoxy group may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, etc., but the present application is not limited thereto.

In the present specification, the cycloalkyl group may include a monocyclic or polycyclic ring having 3 to 60 carbon atoms, and may be further substituted by other substituents. Here, the polycyclic ring refers to a group in which a cycloalkyl group is directly connected or condensed with other ring group. Here, although the other ring group may be a cycloalkyl group, it may also be a different type of ring group, for example, a heterocycloalkyl group, an aryl group, a heteroaryl group, or the like. The number of carbon atoms of the cycloalkyl group may be 3 to 60, specifically 3 to 40, and more specifically 5 to 20. Specific examples of the cycloalkyl group may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, etc., but the present application is not limited thereto.

In the present specification, the heterocycloalkyl group may contain O, S, Se, N, or Si as a heteroatom, may include a monocyclic or polycyclic ring having 2 to 60 carbon atoms, and may be further substituted by other substituents. Here, the polycyclic ring refers to a group in which a heterocycloalkyl group is directly connected or condensed with other ring group. Here, although the other ring group may be a heterocycloalkyl group, it may also be a different type of ring group, for example, a cycloalkyl group, an aryl group, a heteroaryl group, or the like. The number of carbon atoms of the heterocycloalkyl group may be 2 to 60, specifically 2 to 40, and more specifically 3 to 20.

In the present specification, the aryl group may include a monocyclic or polycyclic ring having 6 to 60 carbon atoms, and may be further substituted by other substituents. Here, the polycyclic ring means a group in which an aryl group is directly connected or condensed with other ring group. Here, although the other ring group may be an aryl group, it may also be a different type of ring group, for example, a cycloalkyl group, a heterocycloalkyl group, a heteroaryl group, or the like. The aryl group includes a spiro group. The number of carbon atoms of the aryl group may be 6 to 60, specifically 6 to 40, and more specifically 6 to 25. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, condensed ring groups thereof, etc., but the present application is not limited thereto.

In the present specification, the terphenyl group may be selected from structures below.

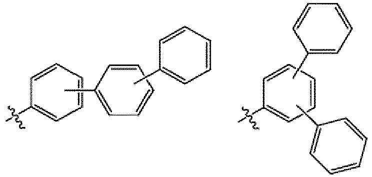

In the present specification, when the substituent is a carbazole group, it means bonding with nitrogen or carbon of carbazole.

In the present specification, when the carbazole group is substituted, an additional substituent may be substituted for nitrogen or carbon of carbazole.

In the present specification, a benzocarbazole group may be any one of structures below.

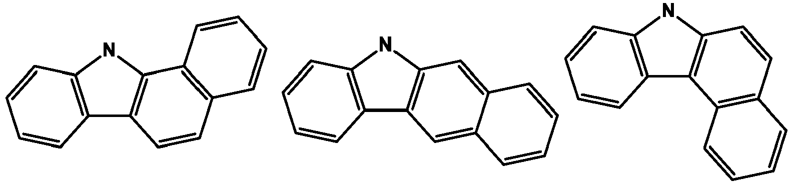

In the present specification, a dibenzocarbazole group may be any one of structures below.

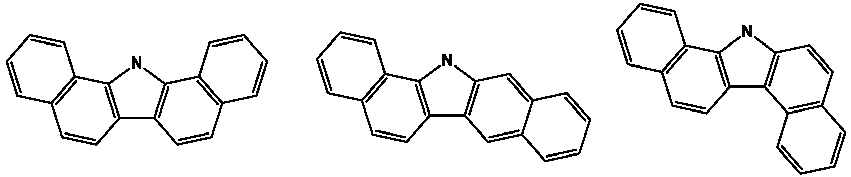

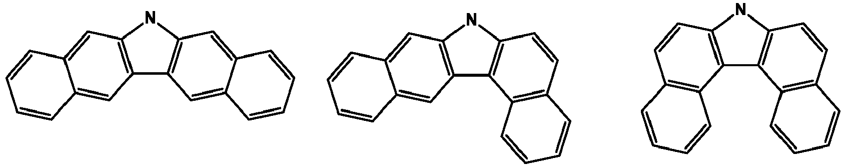

In the present specification, a naphthobenzofuran group may be any one of structures below.

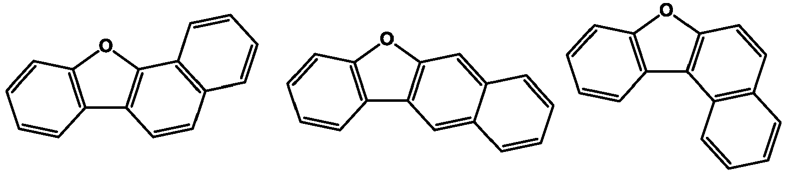

In the present specification, a naphthobenzothiophene group may be any one of structures below.

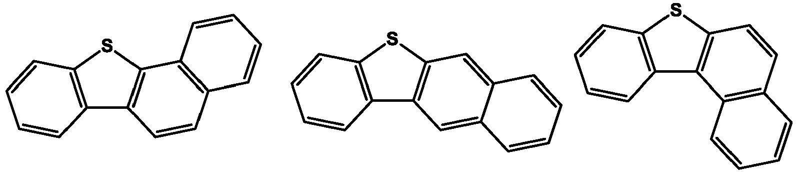

In the present specification, the phosphine oxide group may be represented by -P(=O)R101R102, wherein R101 and R102 may be the same as or different from each other, and may be each independently a substituent consisting of at least one of hydrogen; heavy hydrogen; a halogen group; an alkyl group; an alkenyl group; an alkoxy group; a cycloalkyl group; an aryl group; and a heterocyclic group. The phosphine oxide group may specifically include a diphenylphosphine oxide group, a dinaphthylphosphine oxide group, etc., but the present application is not limited thereto.

In the present specification, the silyl group may be a substituent which contains Si and to which the Si atom is directly connected as a radical, and may be represented by -SiR104R105R106, wherein R104 to R106 may be the same as or different from each other, and may be each independently a substituent consisting of at least one of hydrogen; heavy hydrogen; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, etc., the present application is not limited thereto.

Examples of the silyl group may include 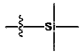 (a trimethylsilyl group), 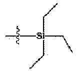 (a triethylsilyl group), 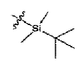 (a t-butyldimethylsilyl group), 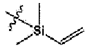 (a vinyldimethylsilyl group), 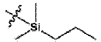 (a propyldimethylsilyl group), 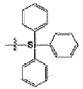 (a triphenylsilyl group), 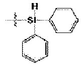 (a diphenylsilyl group), 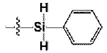 (a phenylsilyl group), etc., the present application is not limited thereto.

In the present specification, the fluorenyl group may be substituted, and adjacent substituents may be bonded to each other to form a ring.

In the present specification, the spiro group is a group including a spiro structure, and may have 15 to 60 carbon atoms. For example, the spiro group may include a structure in which a 2,3-dihydro-1H-indene group or a cyclohexane group is spiro-bonded to a fluorenyl group. Specifically, the spiro group may include any one of groups of structural formulas below.

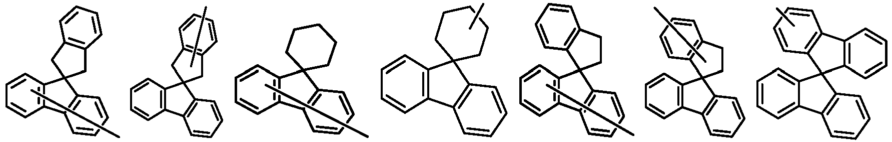

In the present specification, the heteroaryl group may contain S, O, Se, N, or Si as a heteroatom, may include a monocyclic or polycyclic ring having 2 to 60 carbon atoms, and may be further substituted by other substituents. Here, the polycyclic ring refers to a group in which a heteroaryl group is directly connected or condensed with other ring group. Here, although the other ring group may be a heteroaryl group, it may also be a different type of ring group, for example, a cycloalkyl group, a heterocycloalkyl group, an aryl group, or the like. The number of carbon atoms of the heteroaryl group may be 2 to 60, specifically 2 to 40, and more specifically 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a deoxynyl group, a triazinyl group, a tetrazinyl group, a quinolyl group, an isoquinolyl group, a quinazolinyl group, an isoquinazolinyl group, a quinozolilyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilol group, a spirobi (dibenzosilole) group, a dihydrophenazinyl group, a phenoxazinyl group, a phenantridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiazinyl group, a phthalazinyl group, a naphthylidinyl group, a phenanthrolinyl group, a benzo[c] [1,2,5]thiadiazolyl group, a 5,10-dihydrodibenzo[b,e] [1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, etc., but the present application is not limited thereto.

In the present specification, the amine group may be selected from the group consisting of: a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, etc., but the present application is not limited thereto.

In the present specification, the arylene group means one having two bonding positions in the aryl group, that is, a divalent group. The description of the aryl group described above may be applied except that each of these is a divalent group. Further, the heteroarylene group means one having two bonding positions in the heteroaryl group, that is, a divalent group. The description of the heteroaryl group described above may be applied except that each of these is a divalent group.

In the present specification, the "adjacent" group may mean a substituent substituted on an atom directly connected to the atom in which the corresponding substituent is substituted, a substituent positioned to be sterically closest to the corresponding substituent, or another substituent substituted on the atom in which the corresponding substituent is substituted. For example, two substituents substituted at an ortho position in a benzene ring and two substituents substituted at the same carbon in an aliphatic ring may be interpreted as groups "adjacent" to each other.

In the present specification, "when a substituent is not indicated in a chemical formula or compound structure" means that a hydrogen atom is bonded to a carbon atom. However, since heavy hydrogen (²H, deuterium) is an isotope of hydrogen, some hydrogen atoms may be heavy hydrogen.

In an embodiment of the present application, "when a substituent is not indicated in a chemical formula or compound structure" may mean that all positions which can come as a substituent are hydrogen or heavy hydrogen. That is, heavy hydrogen may be an isotope of hydrogen, and some hydrogen atoms may be heavy hydrogen that is an isotope, and the content of heavy hydrogen may be 0 to 100% at this time.

In an embodiment of the present application, "when a substituent is not indicated in a chemical formula or compound structure", if heavy hydrogen is not explicitly excluded, such as the content of heavy hydrogen of 0%, the content of hydrogen of 100%, all of the substituents being hydrogen, etc., hydrogen and heavy hydrogen may be mixed and used in the compound.

In an embodiment of the present application, heavy hydrogen is an element having a deuteron consisting of one proton and one neutron as one of the isotopes of hydrogen as a nucleus, it may be expressed as hydrogen-2, and an element symbol may also be written as D or 2H.

In an embodiment of the present application, although isotopes meaning atoms which have the same atomic number (Z), but have different mass numbers (A) have the same number of protons, they may also be interpreted as elements with different numbers of neutrons.

In an embodiment of the present application, when the total number of substituents that a basic compound may have is defined as T1, and the number of specific substituents among them is defined as T2, the meaning of the content T% of the specific substituents may be defined as T2/T1×100 = T%.

That is, as an example, the 20% content of heavy hydrogen in the phenyl group represented by 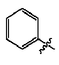 may be expressed as 20% when the total number of substituents that the phenyl group may have is 5 (T1 in Equation) and the number of heavy hydrogens among them is 1 (T2 in Equation). That is, it may be represented by the structural formula below that the content of heavy hydrogen in the phenyl group is 20%.

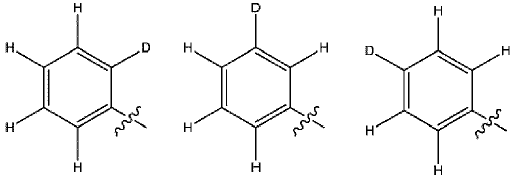

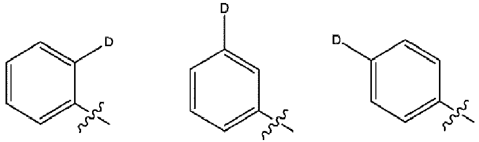

Further, in an embodiment of the present application, in the case of "a phenyl group having a heavy hydrogen content of 0%", it may mean a phenyl group that does not contain a heavy hydrogen atom, that is, has 5 hydrogen atoms.

In an embodiment of the present application, there is provided a heterocyclic compound represented by Chemical Formula 1 below.

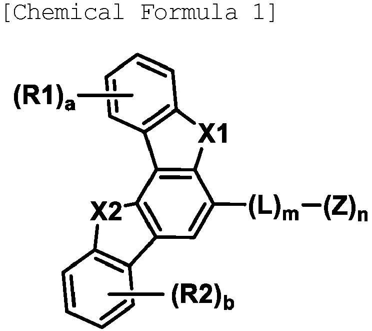

In Chemical Formula 1,
X1 and X2 are each independently O; or S, and X1 and X2 are different from each other,
L is a direct bond; a substituted or unsubstituted C₆-C₆₀ arylene group; or a substituted or unsubstituted C₂-C₆₀ heteroarylene group,
Z is a substituted or unsubstituted amine group; or a substituted or unsubstituted C₂-C₆₀ heteroaryl group,
R1 and R2 are the same as or different from each other, and are each independently hydrogen; heavy hydrogen; a cyano group; a nitro group; a substituted or unsubstituted C₁-C₆₀ alkyl group; a substituted or unsubstituted C₁-C₂₀ alkoxy group; a substituted or unsubstituted C₃-C₆₀ cycloalkyl group; a substituted or unsubstituted C₂-C₆₀ alkenyl group; a substituted or unsubstituted C₂-C₆₀ alkynyl group; a substituted or unsubstituted C₁-C₆₀ aryl group; a substituted or unsubstituted C₂-C₆₀ heterocycloalkyl group; a substituted or unsubstituted C₂-C₆₀ heteroaryl group; a substituted or unsubstituted phosphine oxide group; or a substituted or unsubstituted silyl group,
a and b are each independently an integer of 0 to 4, and when a and b are each 2 or more, the substituents in parentheses are the same as or different from each other, and
m and n are each independently an integer of 0 to 6, and when m and n are each 2 or more, the substituents in parentheses are the same as or different from each other.

Since the heterocyclic compound represented by Chemical Formula 1 above has a steric arrangement by fixing a substituent to a specific position, and spatially separates Highest Occupied Molecular Orbital (HOMO) and Lowest Unoccupied Molecular Orbital (LUMO) so that strong charge transfer is possible, high efficiency and increased lifespan of the organic light emitting device may be expected when it is used as an organic material in an organic light emitting device.

In an embodiment of the present application, L of Chemical Formula 1 above may be a direct bond; a substituted or unsubstituted arylene group; or a substituted or unsubstituted heteroarylene group.

In another embodiment, L may be a direct bond; a substituted or unsubstituted C₆-C₆₀ arylene group; or a substituted or unsubstituted C₂-C₆₀ heteroarylene group.

In another embodiment, L may be a direct bond; a substituted or unsubstituted C₆-C₄₀ arylene group; or a substituted or unsubstituted C₂-C₄₀ heteroarylene group.

In another embodiment, L may be a direct bond; a substituted or unsubstituted C₆-C₂₀ arylene group; or a substituted or unsubstituted C₂-C₂₀ heteroarylene group.

In another embodiment, L may be a direct bond; a substituted or unsubstituted phenylene group; a substituted or unsubstituted biphenylene group; a substituted or unsubstituted triphenylene group; a substituted or unsubstituted fluorenylene group; a substituted or unsubstituted naphthalenylene group; a substituted or unsubstituted anthracenylene group; a substituted or unsubstituted 9,10-dihydroanthracene group; a substituted or unsubstituted carbazole group; a substituted or unsubstituted dibenzothiophenylene group; a substituted or unsubstituted benzofuranylene group; a substituted or unsubstituted benzonaphthothiophenylene group; or a substituted or unsubstituted naphthobenzofuranylene group.

In an embodiment of the present application, m of Chemical Formula 1 above is an integer of 0 to 6, and when m is 2 or more, L in parentheses are the same as or different from each other.

In an embodiment of the present application, m is an integer of 1 to 6, and when m is 2 or more, L in parentheses are the same as or different from each other.

In an embodiment of the present application, m is 0.

In an embodiment of the present application, m is 1.

In an embodiment of the present application, m is 2.

In an embodiment of the present application, m is 3.

In an embodiment of the present application, m is 4.

In an embodiment of the present application, m is 5.

In an embodiment of the present application, m is 6.

In an embodiment of the present application, when m is 2 or more, L in parentheses are the same as or different from each other.

In an embodiment of the present application, n is an integer of 0 to 6, and when n is 2 or more, Z in parentheses are the same as or different from each other.

In an embodiment of the present application, n is an integer of 1 to 6, and when n is 2 or more, Z in parentheses are the same as or different from each other.

In an embodiment of the present application, n is 0.

In an embodiment of the present application, n is 1.

In an embodiment of the present application, n is 2.

In an embodiment of the present application, n is 3.

In an embodiment of the present application, n is 4.

In an embodiment of the present application, n is 5.

In an embodiment of the present application, n is 6.

In an embodiment of the present application, when n is 2 or more, Z in parentheses are the same as or different from each other.

In an embodiment of the present application, m and n are each independently an integer of 0 to 6, and m+n≥1.

In an embodiment of the present application, m and n may be each independently an integer of 1 to 6, and when m and n are each 2 or more, the substituents in parentheses are the same as or different from each other.

In Chemical Formula 1 above, when a heterocyclic compound represented as a case in which L is not a direct bond, or m is not 0 is used as an organic material in an organic light emitting device, the efficiency and lifespan of the organic light emitting device are more excellent than a case in which L is a direct bond, or m is 0. This is considered to be since stronger charge transfer is possible by spatially further separating HOMO and LUMO since L is not a direct bond.

In an embodiment of the present application, X1 and X2 of Chemical Formula 1 above are each independently O; or S, and X1 and X2 may be different from each other.

In an embodiment of the present application, X1 is O, and X2 is S.

In an embodiment of the present application, X1 is S, and X2 is O.

In an embodiment of the present application, Z of Chemical Formula 1 above may be a substituted or unsubstituted C₂-C₆₀ heteroaryl group; or an amine group substituted or unsubstituted with one or more selected from the group consisting of a substituted or unsubstituted C₆-C₄₀ aryl group and a substituted or unsubstituted C₂-C₄₀ heteroaryl group.

In an embodiment of the present application, Z may be a substituted or unsubstituted C₂-C₄₀ heteroaryl group; or an amine group substituted or unsubstituted with one or more selected from the group consisting of a substituted or unsubstituted C₆-C₄₀ aryl group and a substituted or unsubstituted C₂-C₄₀ heteroaryl group.

In an embodiment of the present application, Z may be a substituted or unsubstituted C₂-C₂₀ heteroaryl group; or an amine group substituted or unsubstituted with one or more selected from the group consisting of a substituted or unsubstituted C₆-C₂₀ aryl group and a substituted or unsubstituted C₂-C₂₀ heteroaryl group.

In an embodiment of the present application, Z of Chemical Formula 1 above may be a group represented by any one of Chemical Formulas 2 to 4 below.

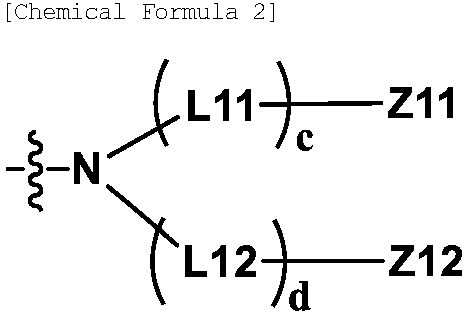

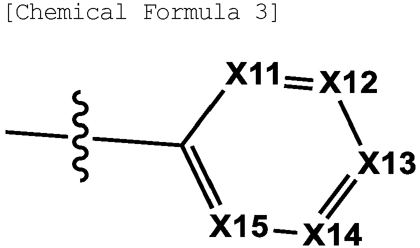

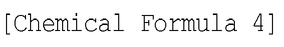

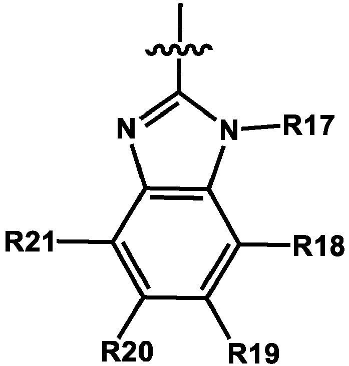

In Chemical Formulas 2 to 4,
L11 and L12 are the same as or different from each other, and are each independently a direct bond; and a substituted or unsubstituted C₆-C₄₀ arylene group or a substituted or unsubstituted C₂-C₄₀ heteroarylene group, c and d are each 0 or 1,
Z11 and Z12 may be the same as or different from each other, and may be each independently a substituted or unsubstituted C₆-C₄₀ aryl group or a substituted or unsubstituted C₂-C₄₀ heteroaryl group, and Z11 and Z12 may be bonded to each other to form a substituted or unsubstituted C₆-C₆₀ aromatic hydrocarbon ring or a substituted or unsubstituted C₂-C₆₀ heterocycle,
X11 is CR11 or N, X12 is CR12 or N, X13 is CR13 or N, X14 is CR14 or N, X15 is CR15 or N, and at least one of X11 to X15 is N, and
R11 to R15 and R17 to R21 may be the same as or different from each other, and may be each independently selected from the group consisting of: hydrogen; heavy hydrogen; halogen; a cyano group; a substituted or unsubstituted C₁-C₆₀ alkyl group; a substituted or unsubstituted C₂-C₆₀ alkenyl group; a substituted or unsubstituted C₂-C₆₀ alkynyl group; a substituted or unsubstituted C₁-C₂₀ alkoxy group; a substituted or unsubstituted C₃-C₆₀ cycloalkyl group; a substituted or unsubstituted C₂-C₆₀ heterocycloalkyl group; a substituted or unsubstituted C₆-C₆₀ aryl group; a substituted or unsubstituted C₂-C₆₀ heteroaryl group; a substituted or unsubstituted phosphine oxide group; and a substituted or unsubstituted amine group, or two or more groups adjacent to each other may be bonded to each other to form a substituted or unsubstituted C₆-C₆₀ aromatic hydrocarbon ring or a substituted or unsubstituted C₂-C₆₀ heterocycle, and 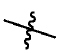 denotes a position bonded to Chemical Formula 1 above.

In an embodiment of the present application, L11 and L12 of Chemical Formula 2 above may be the same as or different from each other, and may be each independently a direct bond; a substituted or unsubstituted C₆-C₄₀ arylene group or a substituted or unsubstituted C₂-C₄₀ heteroarylene group.

In an embodiment of the present application, L11 and L12 may be the same as or different from each other, and may be each independently a direct bond; a substituted or unsubstituted C₆-C₂₀ arylene group; or a substituted or unsubstituted C₂-C₂₀ heteroarylene group.

In an embodiment of the present application, Z11 and Z12 of Chemical Formula 1 above may be the same as or different from each other, and may be each independently a substituted or unsubstituted C₆-C₄₀ aryl group or a substituted or unsubstituted C₂-C₄₀ heteroaryl group.

In an embodiment of the present application, Z11 and Z12 may be the same as or different from each other, and may be each independently a substituted or unsubstituted C₆-C₂₀ aryl group or a substituted or unsubstituted C₂-C₂₀ heteroaryl group.

In an embodiment of the present application, Z11 and Z12 may be bonded to each other to form a substituted or unsubstituted C₆-C₆₀ aromatic hydrocarbon ring or a substituted or unsubstituted C₂-C₆₀ heterocycle.

In an embodiment of the present application, Z11 and Z12 may be different.

In an embodiment of the present application, Z11 may be a substituted or unsubstituted C₆-C₄₀ aryl group, and Z12 may be a substituted or unsubstituted C₂-C₄₀ heteroaryl group.

In an embodiment of the present application, Z12 may be a substituted or unsubstituted C₆-C₄₀ aryl group, and Z11 may be a substituted or unsubstituted C₂-C₄₀ heteroaryl group.

In Chemical Formula 2 above, when a heterocyclic compound represented as a case in which one of Z11 and Z12 is an aryl group and the other is a heteroaryl group is used as an organic material in an organic light emitting device, the efficiency and lifespan of the organic light emitting device are more excellent than a case in which both Z11 and Z12 are aryl groups. This is considered to be since stronger charge transfer is possible by spatially further separating HOMO and LUMO since one of Z11 and Z12 is an aryl group and the other is a heteroaryl group.

In an embodiment of the present application, Chemical Formula 2 above may be represented by any one of Chemical Formulas 2-1 to 2-4 below.

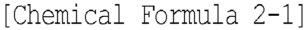

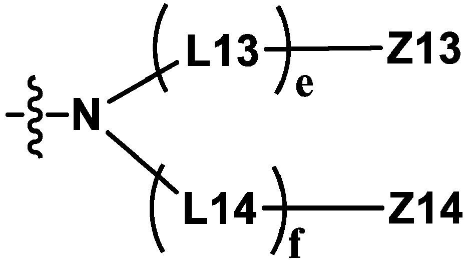

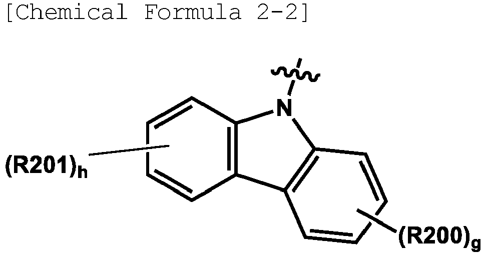

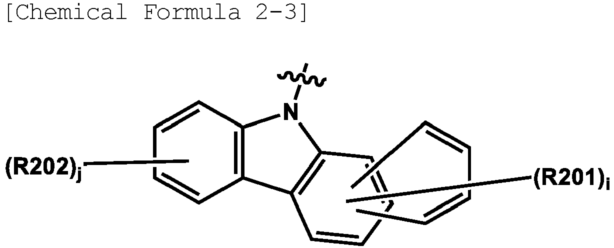

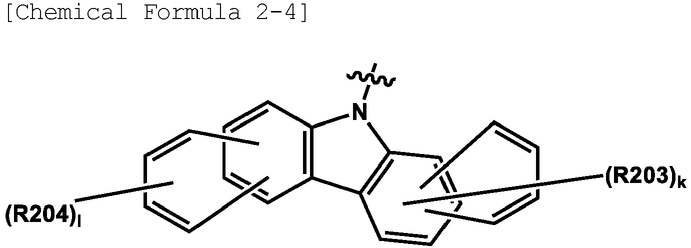

In Chemical Formulas 2-1 to 2-4,
L13 and L14 are the same as or different from each other, and are each independently a direct bond; a substituted or unsubstituted C₆-C₄₀ arylene group; or a substituted or unsubstituted C₂-C₄₀ heteroarylene group,
Z13 and Z14 are the same as or different from each other, and are each independently a substituted or unsubstituted C₆-C₄₀ aryl group; or a substituted or unsubstituted C₂-C₄₀ heteroaryl group,
R200 to R204 are each independently hydrogen; heavy hydrogen; a halogen group; a cyano group; a substituted or unsubstituted C₆-C₂₀ aryl group; or a substituted or unsubstituted C₂-C₂₀ heteroaryl group,
e and f are each 0 or 1,
g, h, j, and l are each an integer of 0 to 4, i and k are integers of 0 to 6, and when g to l are each 2 or more, the substituents in parentheses are the same as or different from each other, and 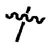
denotes a position bonded to L1 of Chemical Formula 1 above.

In an embodiment of the present application, L13 and L14 of Chemical Formula 2-1 above may be the same as or different from each other, and may be each independently a direct bond; and a substituted or unsubstituted C₆-C₄₀ arylene group or a substituted or unsubstituted C₂-C₄₀ heteroarylene group.

In an embodiment of the present application, L13 and L14 may be the same as or different from each other, and may be each independently a direct bond; a substituted or unsubstituted C₆-C₂₀ arylene group; or a substituted or unsubstituted C₂-C₂₀ heteroarylene group.

In an embodiment of the present application, Z13 and Z14 of Chemical Formula 2-1 above may be the same as or different from each other, and may be each independently a substituted or unsubstituted C₆-C₄₀ aryl group or a substituted or unsubstituted C₂-C₄₀ heteroaryl group.

In an embodiment of the present application, Z13 and Z14 may be the same as or different from each other, and may be each independently a substituted or unsubstituted C₆-C₂₀ aryl group or a substituted or unsubstituted C₂-C₂₀ heteroaryl group.

In an embodiment of the present application, Chemical Formula 3 above may be represented by one of Chemical Formulas 3-1 to 3-4 below. Here, 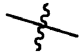 denotes a position bonded to L1 of Chemical Formula 1 above.

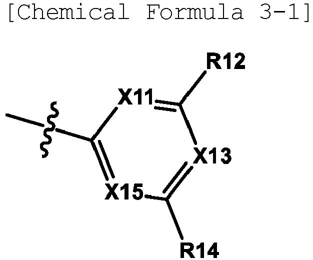

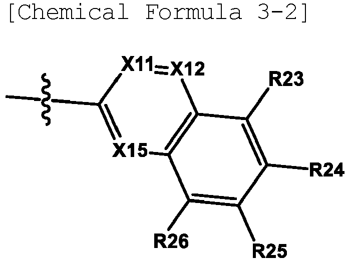

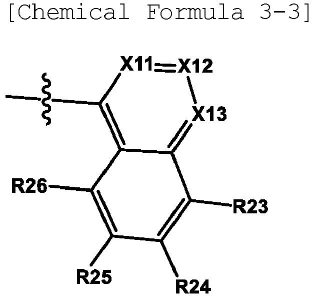

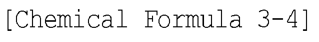

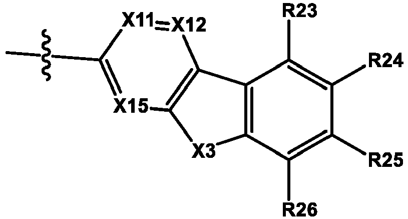

In Chemical Formula 3-1, one or more of X11, X13, and X15 are N, and the rest are as defined in Chemical Formula 3,
In Chemical Formula 3-2, one or more of X11, X12, and X15 are N, and the rest are as defined in Chemical Formula 3,
In Chemical Formula 3-3, one or more of X11 to X13 are N, and the rest are as defined in Chemical Formula 3,
In Chemical Formula 3-4, one or more of X11, X12, and X15 are N, and the rest are as defined in Chemical Formula 3,
X3 is O; or S, and
R12, R14, and R23 to R26 are the same as or different from each other, and are each independently selected from the group consisting of: hydrogen; heavy hydrogen; halogen; a cyano group; a substituted or unsubstituted C₁-C₆₀ alkyl group; a substituted or unsubstituted C₂-C₆₀ alkenyl group; a substituted or unsubstituted C₂-C₆₀ alkynyl group; a substituted or unsubstituted C₁-C₂₀ alkoxy group; a substituted or unsubstituted C₃-C₆₀ cycloalkyl group; a substituted or unsubstituted C₂-C₆₀ heterocycloalkyl group; a substituted or unsubstituted C₆-C₆₀ aryl group; a substituted or unsubstituted C₆-C₆₀ heteroaryl group; a substituted or unsubstituted phosphine oxide group; and a substituted or unsubstituted amine group, or two or more groups adjacent to each other are bonded to each other to form a substituted or unsubstituted aliphatic or aromatic hydrocarbon ring or heterocycle.

In an embodiment of the present application, Chemical Formula 3 above may be selected from structural formulas of Group A below.

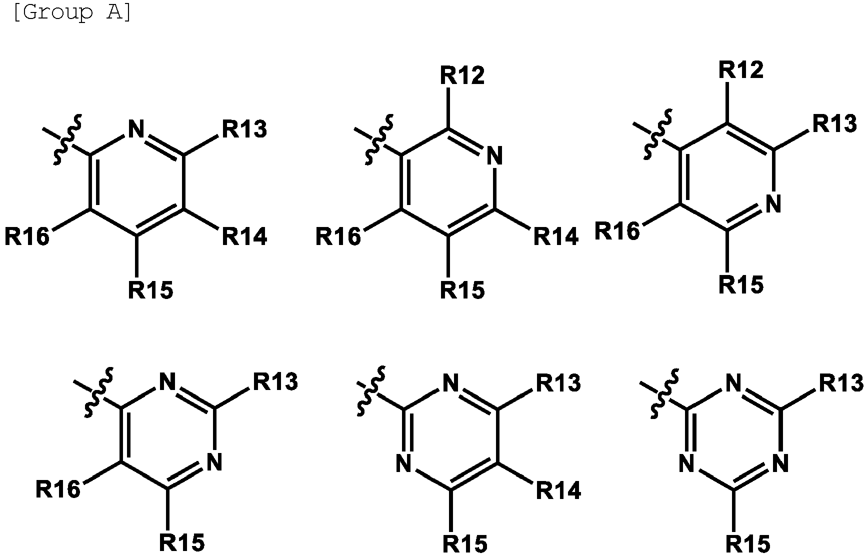

Substituent of the structural formulas of Group A above are defined as in Chemical Formula 3 above.

In an embodiment of the present application, Chemical Formula 3-2 above may be represented by Chemical Formula 3-2-1 below.

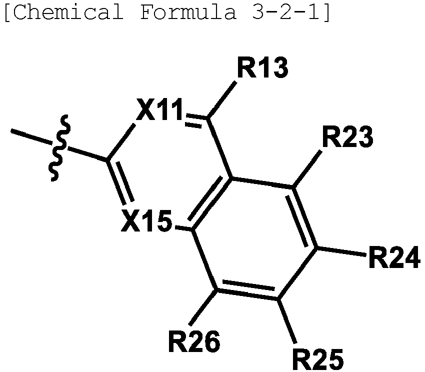

The definitions of the substituents of Chemical Formula 3-2-1 above are as defined in Chemical Formula 3-2.

In an embodiment of the present application, Chemical Formula 3-3 above may be represented by Chemical Formula 3-3-1 below.

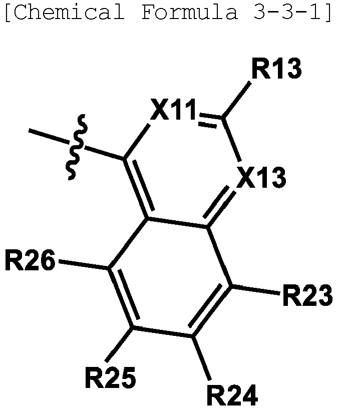

The definitions of the substituents of Chemical Formula 3-3-1 above are as defined in Chemical Formula 3-3.

In an embodiment of the present application, Chemical Formula 3-2 above may be represented by Chemical Formula 3-2-2 or 3-2-3 below.

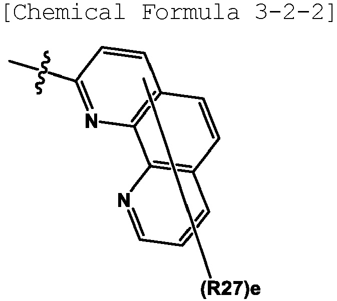

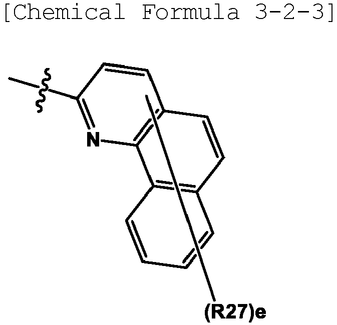

In Chemical Formulas 3-2-2 and 3-2-3,
R27 are the same as or different from each other and are selected from the group consisting of: hydrogen; heavy hydrogen; halogen; a cyano group; a substituted or unsubstituted C₁-C₆₀ alkyl group; a substituted or unsubstituted C₂-C₆₀ alkenyl group; a substituted or unsubstituted C₂-C₆₀ alkynyl group; a substituted or unsubstituted C₁-C₂₀ alkoxy group; a substituted or unsubstituted C₃-C₆₀ cycloalkyl group; a substituted or unsubstituted C₂-C₆₀ heterocycloalkyl group; a substituted or unsubstituted C₆-C₆₀ aryl group; a substituted or unsubstituted C₂-C₆₀ heteroaryl group; a substituted or unsubstituted phosphine oxide group; and a substituted or unsubstituted amine group, or two or more groups adjacent to each other are bonded to each other to form a substituted or unsubstituted aliphatic or aromatic hydrocarbon ring or heterocycle, e is an integer of 0 to 7, and when e is 2 or more, R₂₇ are the same as or different from each other.

In an embodiment of the present application, Chemical Formula 3-4 above may be represented by Chemical Formula 3-4-1 below.

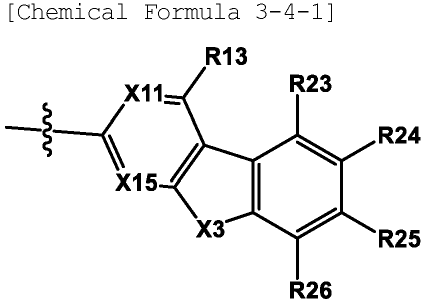

The definitions of the substituents of Chemical Formula 3-4-1 above are as defined in Chemical Formula 3-4.

In an embodiment of the present application, R1 and R2 of Chemical Formula 1 above may be the same as or different from each other, and may be each independently hydrogen; heavy hydrogen; a halogen group; a cyano group; a substituted or unsubstituted C₁-C₃₀ alkyl group; or a substituted or unsubstituted C₃-C₃₀ cycloalkyl group.

In an embodiment of the present application, a and b are each independently an integer of 0 to 4, and when a and b are each 2 or more, the substituents in parentheses are the same as or different from each other.

In an embodiment of the present application, a is an integer of 0 to 4, and when a is 2 or more, R1 in parentheses are the same as or different from each other.

In an embodiment of the present application, b is an integer of 0 to 4, and when b is 2 or more, R2 in parentheses are the same as or different from each other.

In an embodiment of the present application, R1 and R2 of Chemical Formula 1 above are hydrogen.

In an embodiment of the present application, Chemical Formula 1 above may be represented by Chemical Formula 1-1 or 1-2 below.

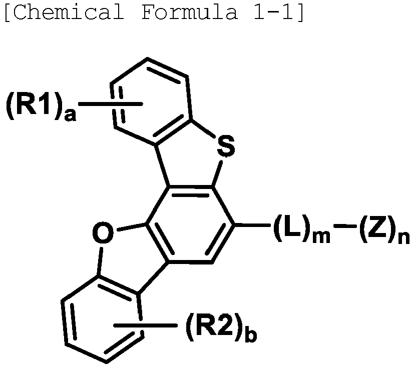

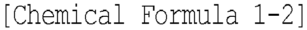

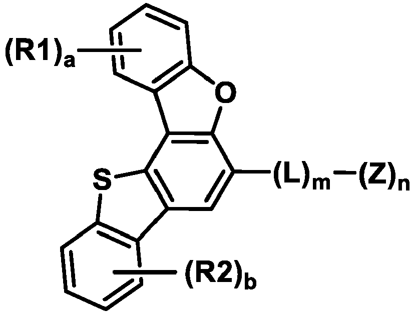

In Chemical Formulas 1-1 and 1-2, the definition of each substituent is the same as in Chemical Formula 1.

In an embodiment of the present application, Chemical Formula 1 above provides a heterocyclic compound represented by any one of compounds below.

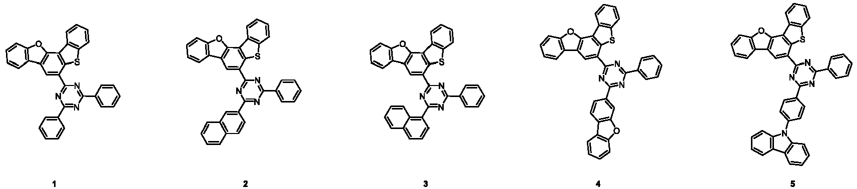

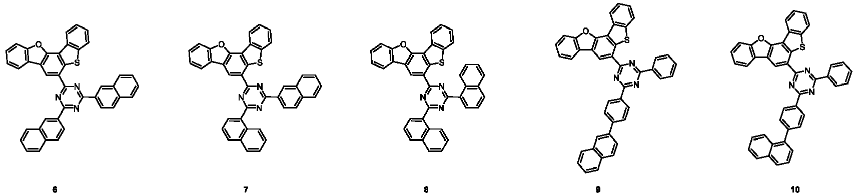

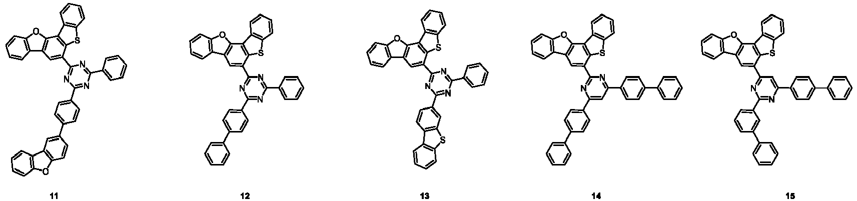

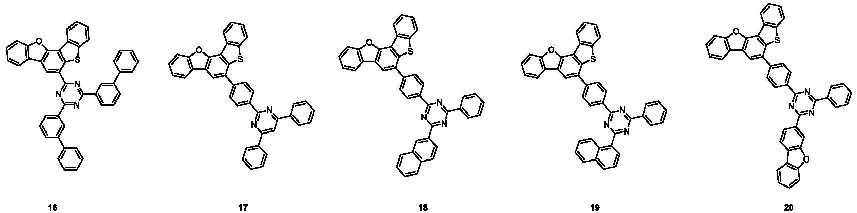

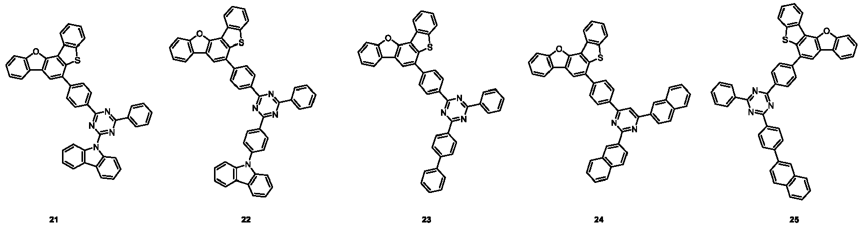

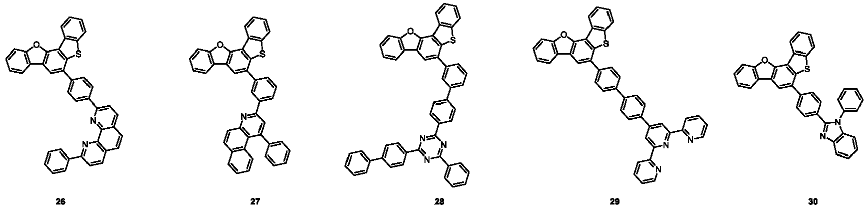

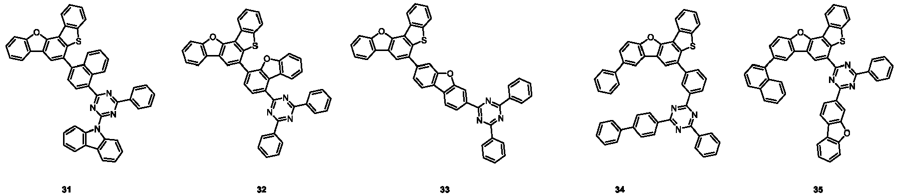

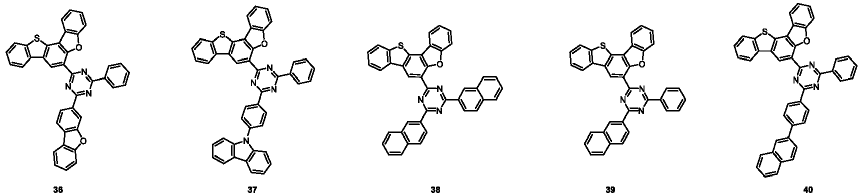

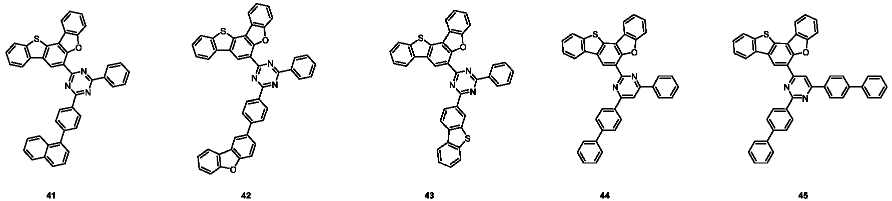

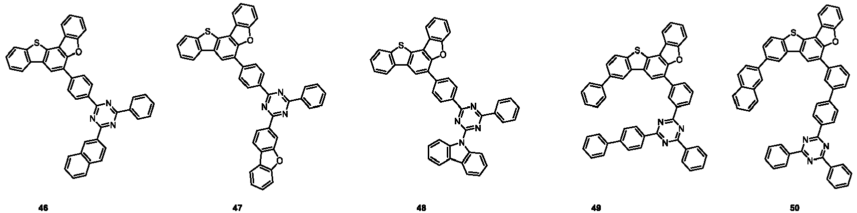

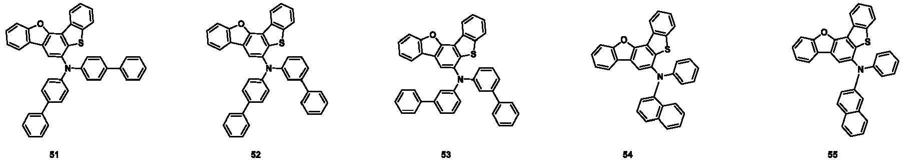

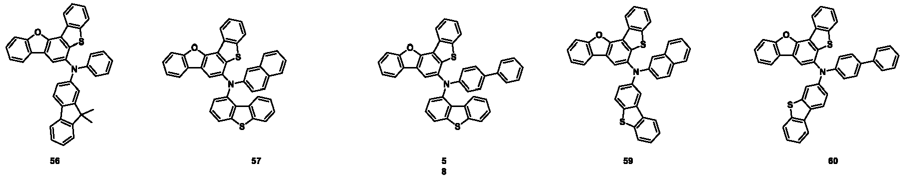

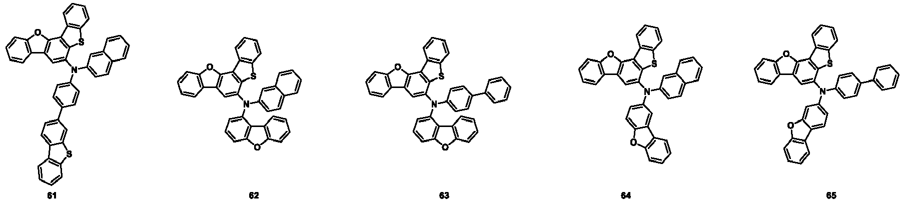

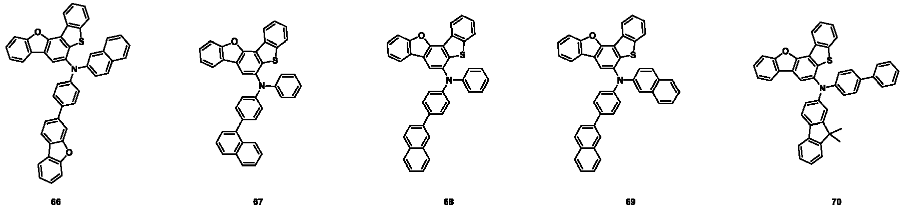

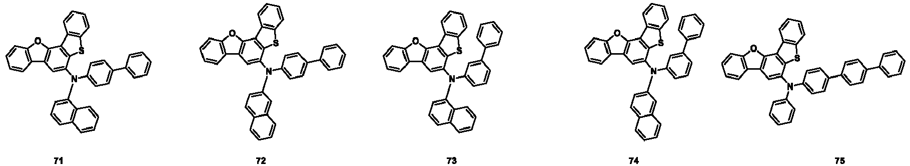

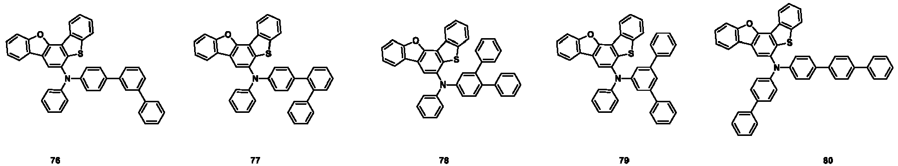

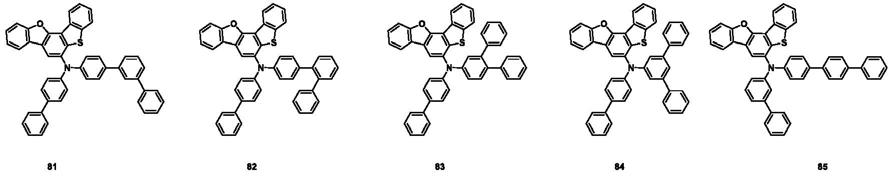

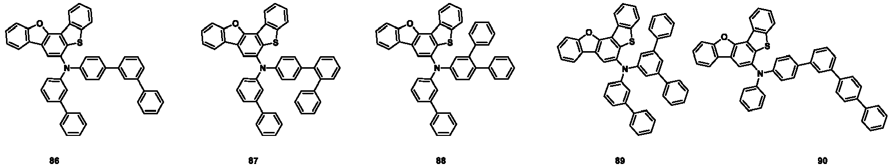

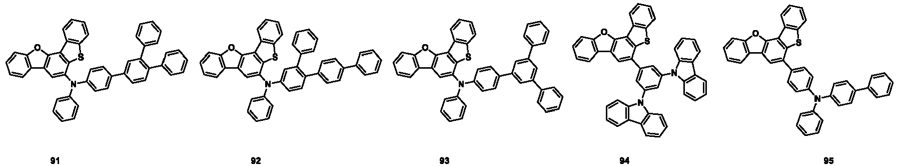

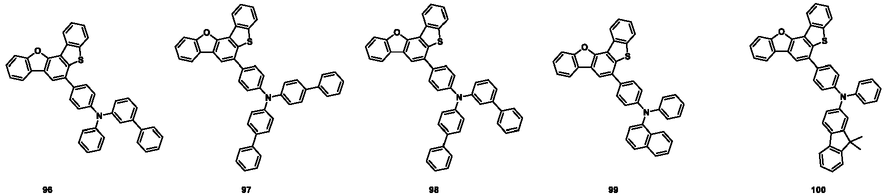

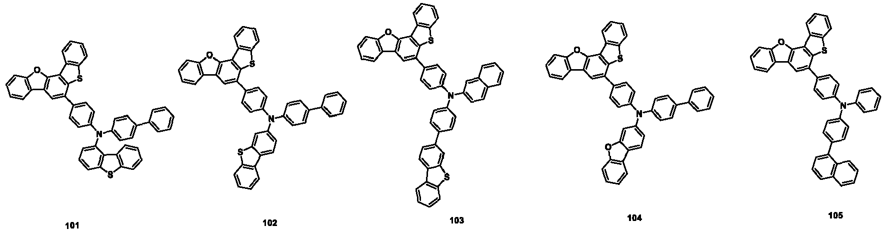

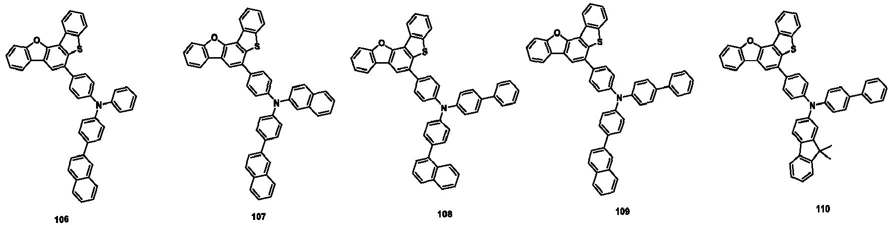

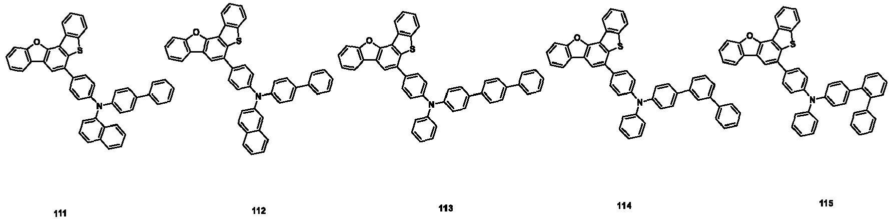

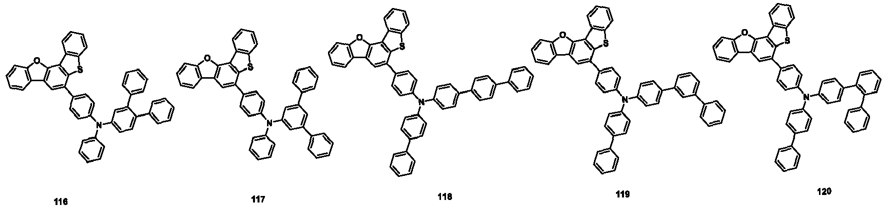

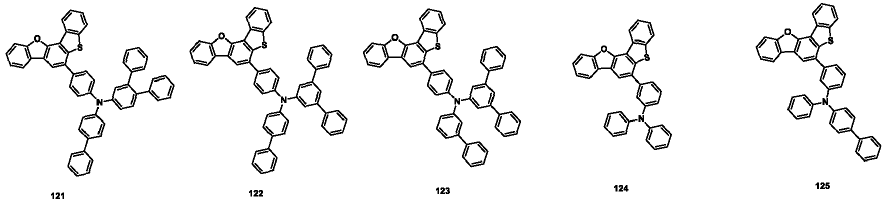

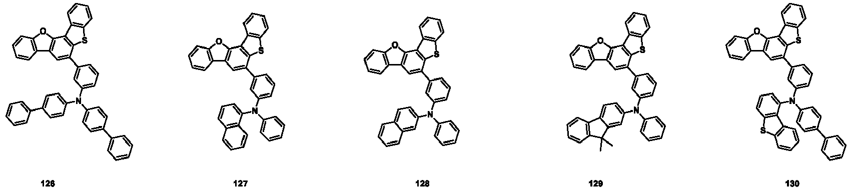

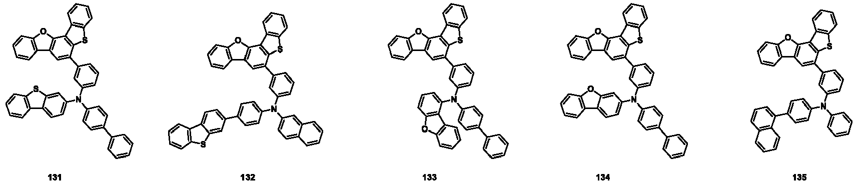

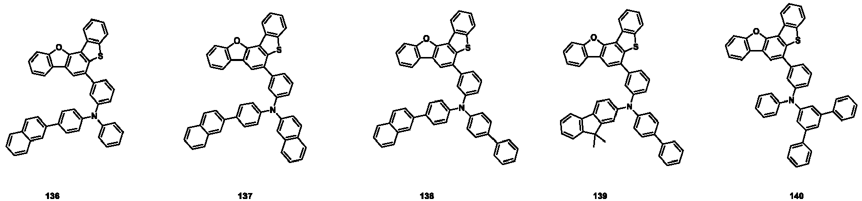

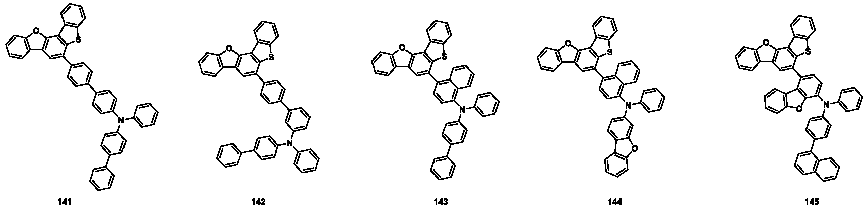

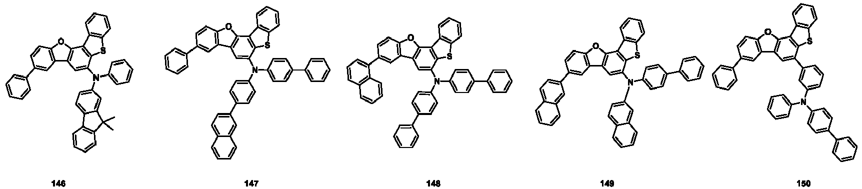

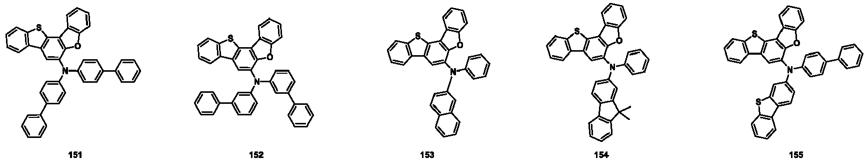

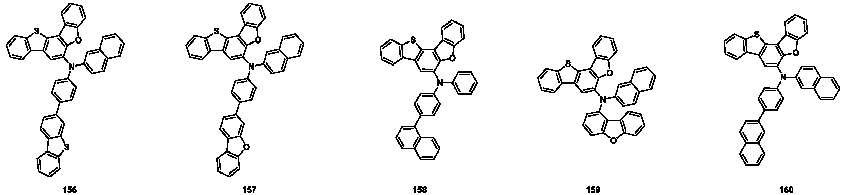

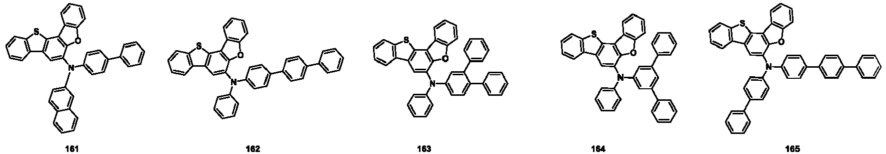

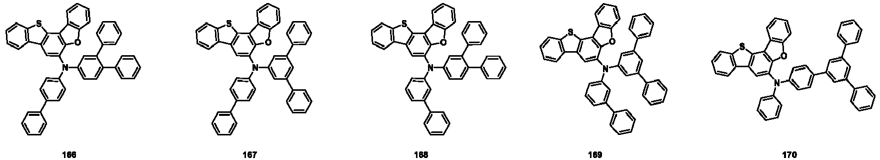

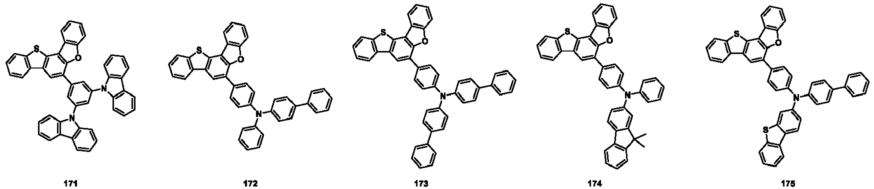

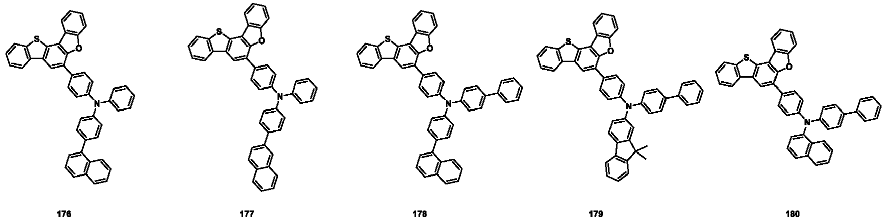

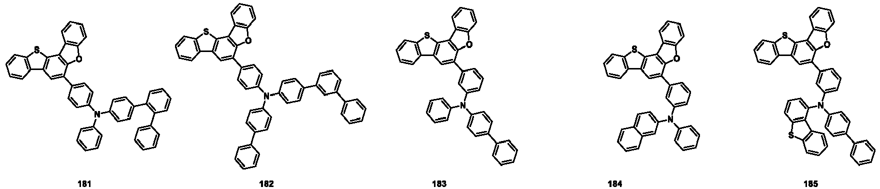

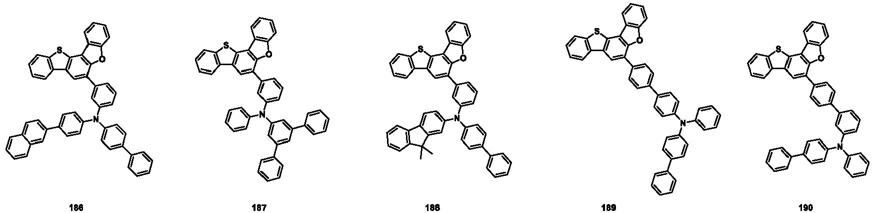

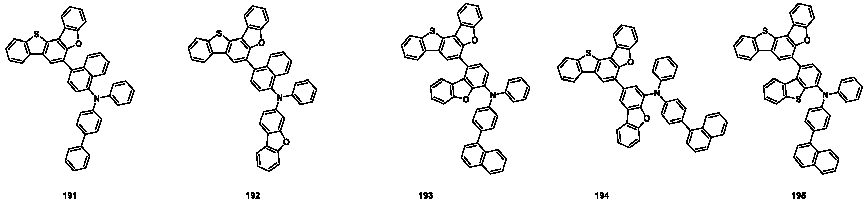

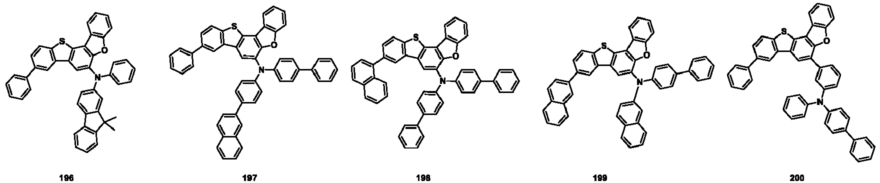

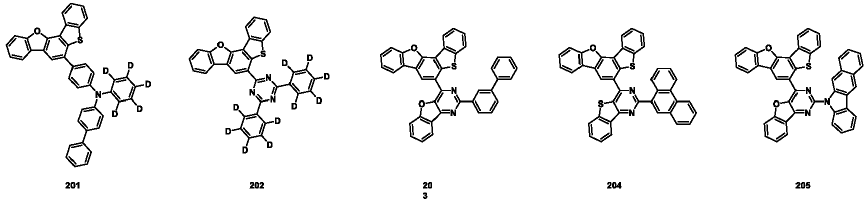

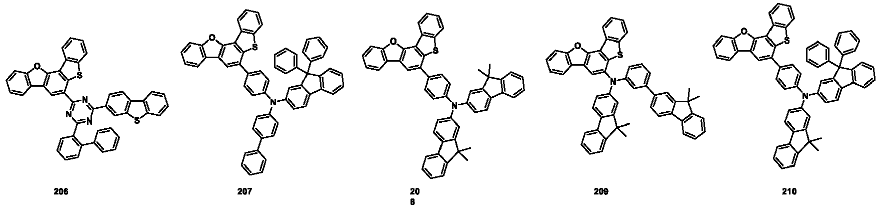

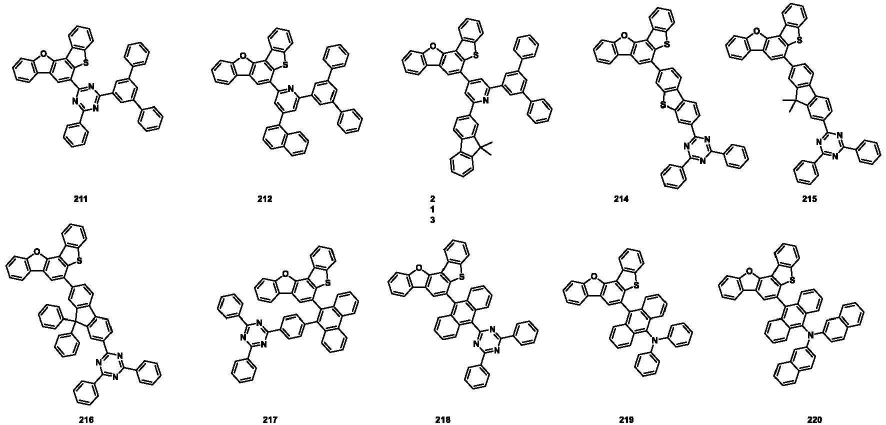

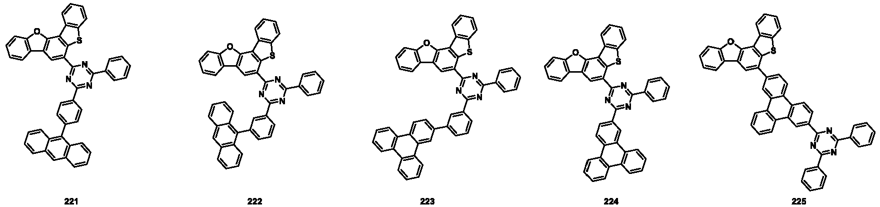

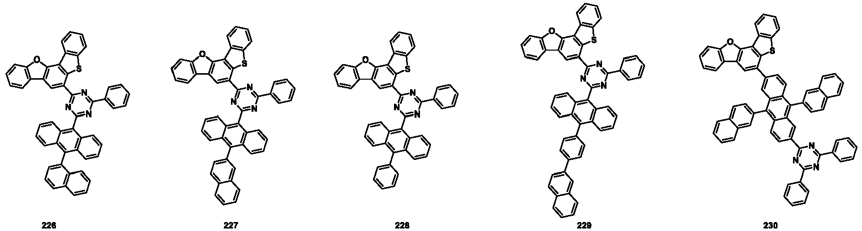

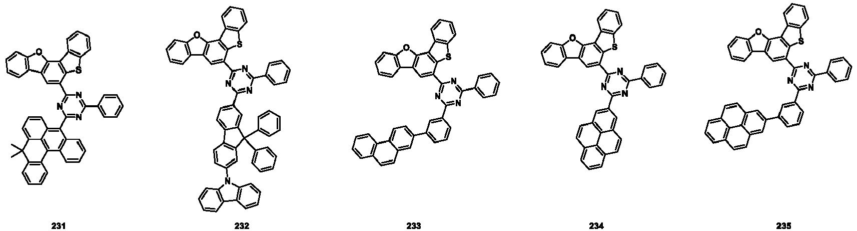

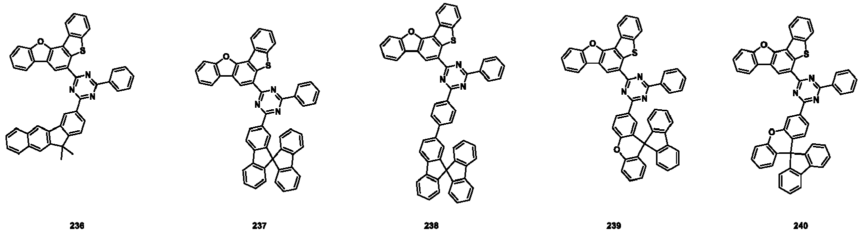

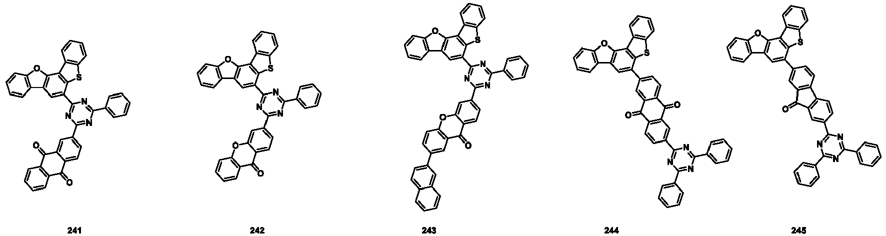

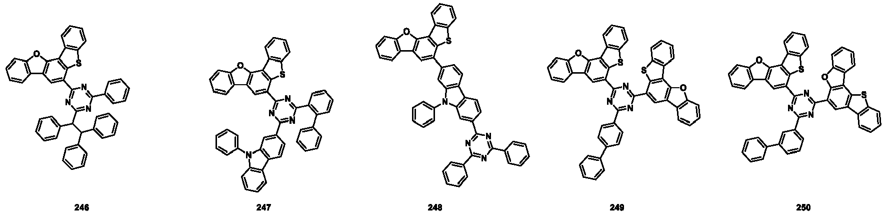

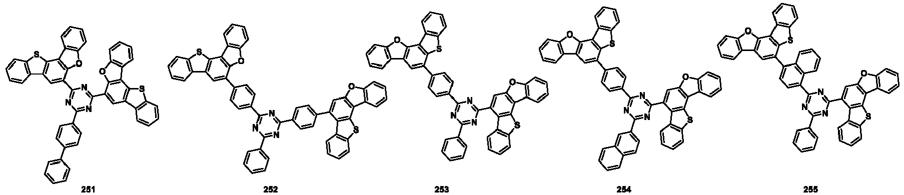

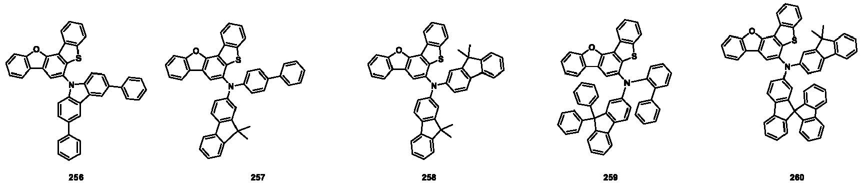

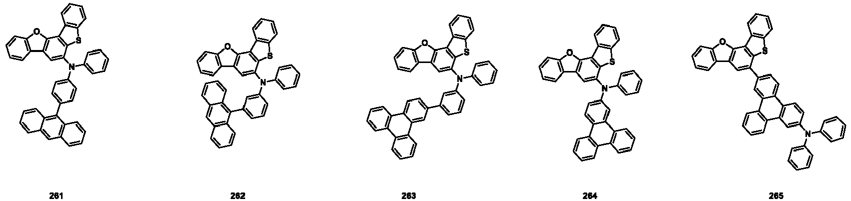

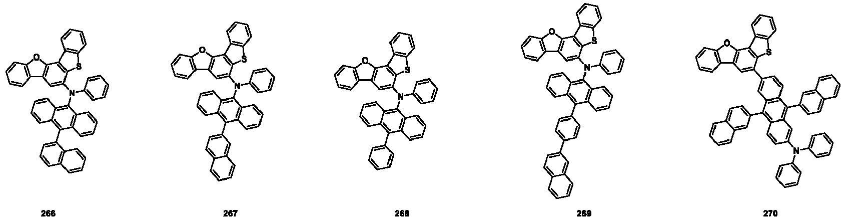

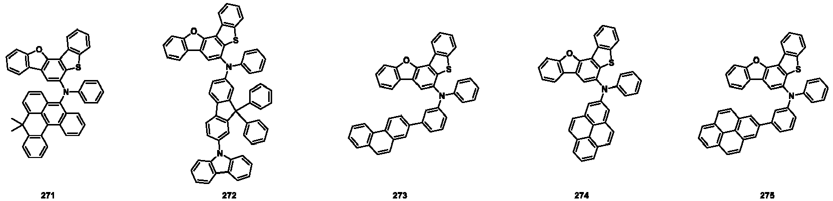

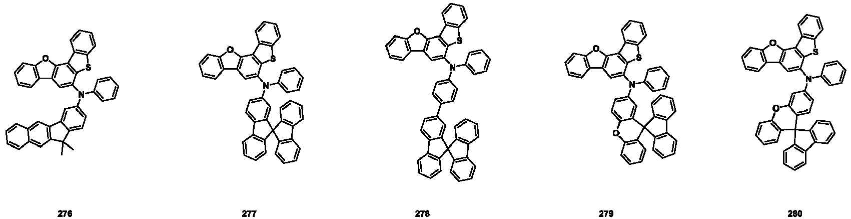

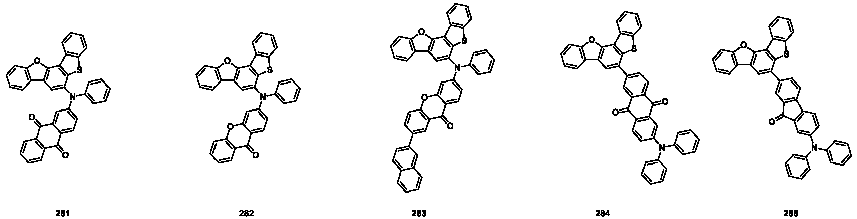

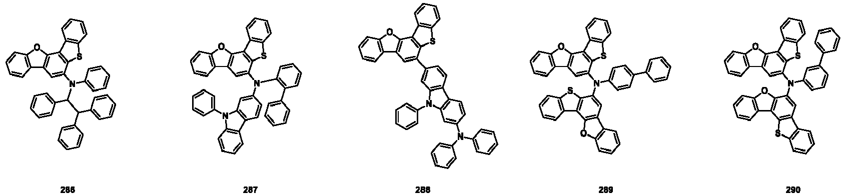

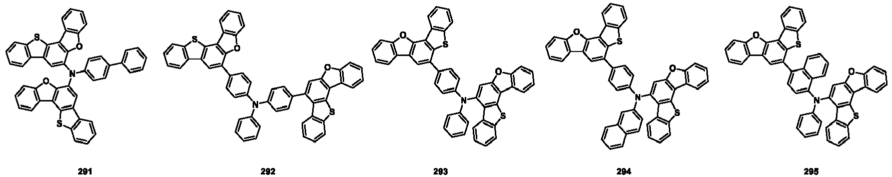

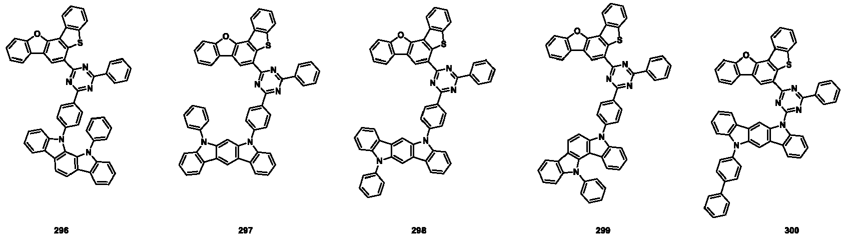

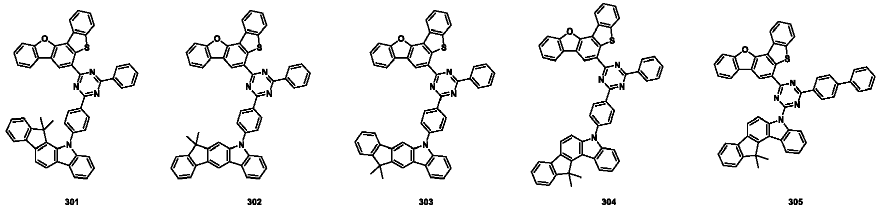

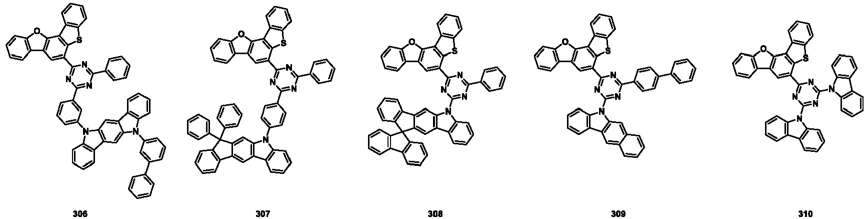

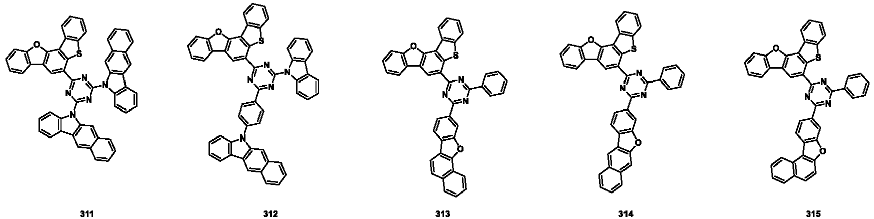

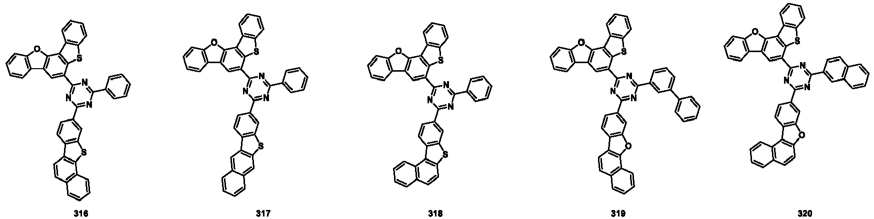

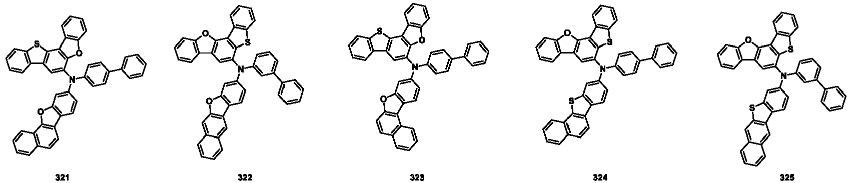

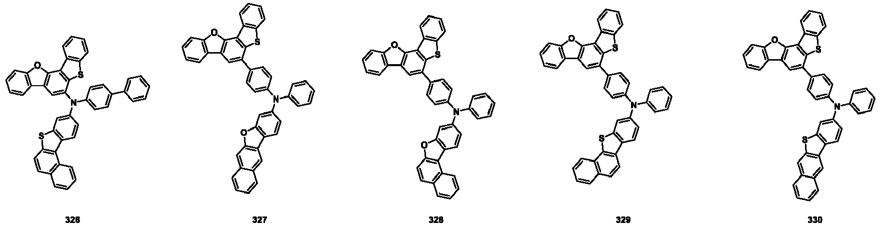

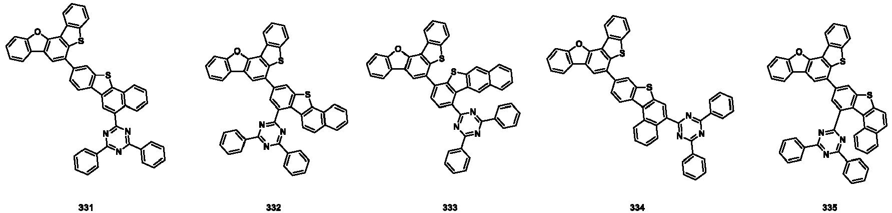

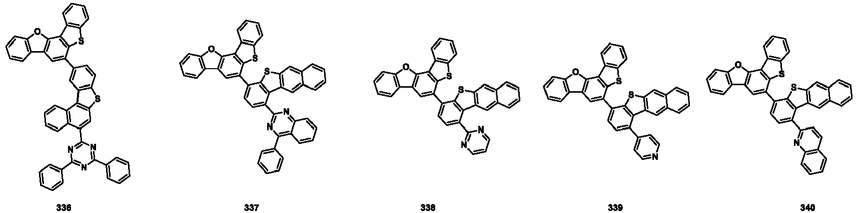

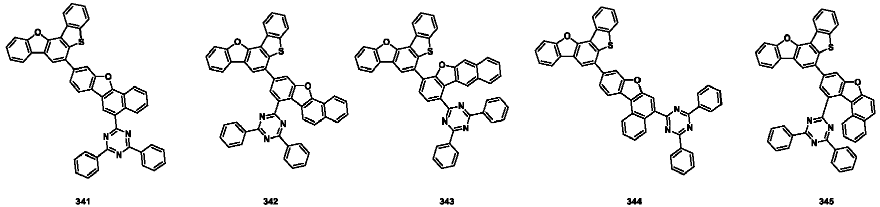

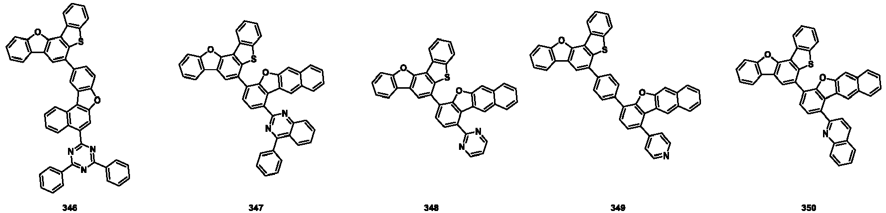

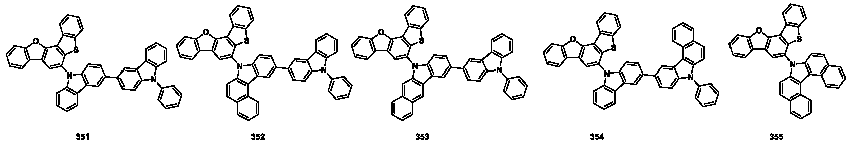

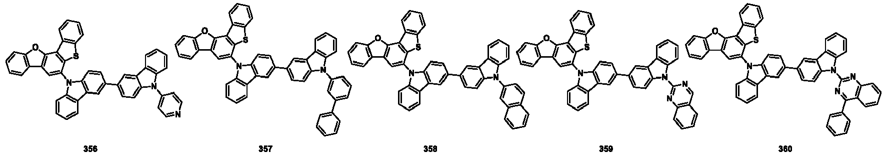

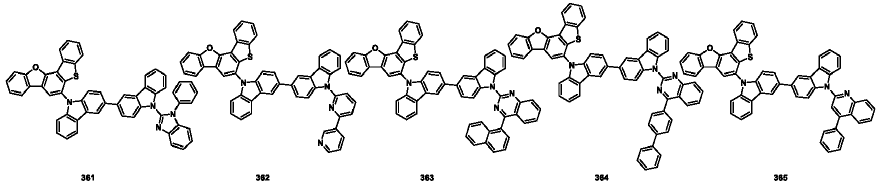

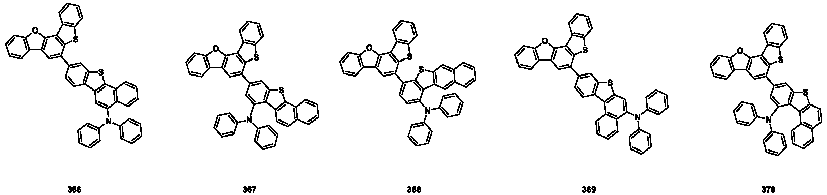

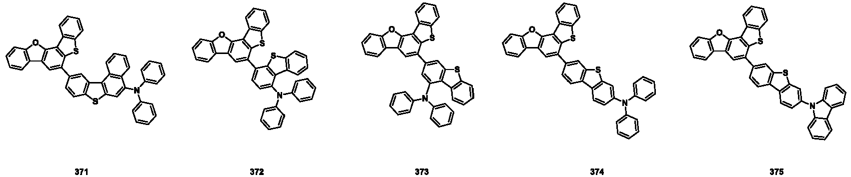

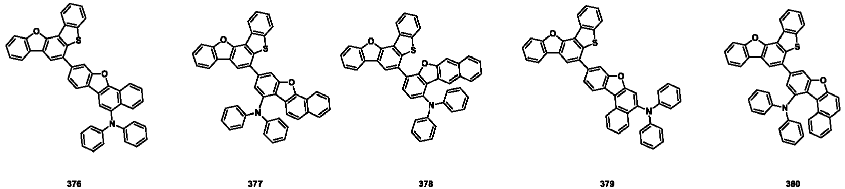

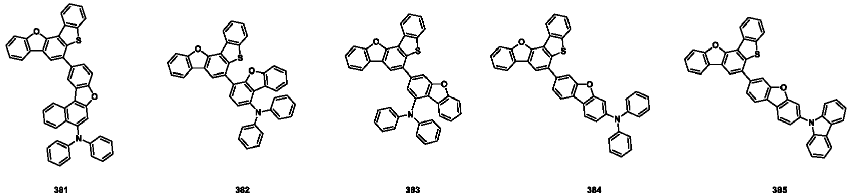

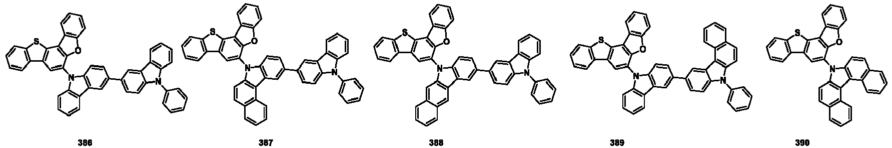

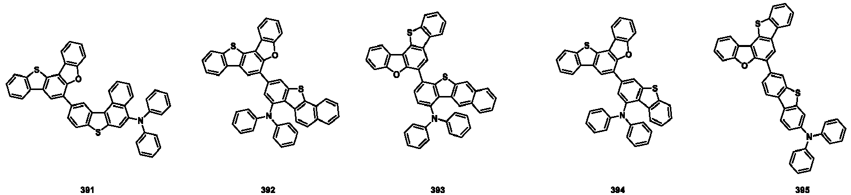

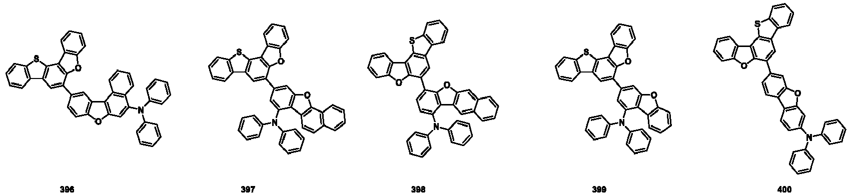

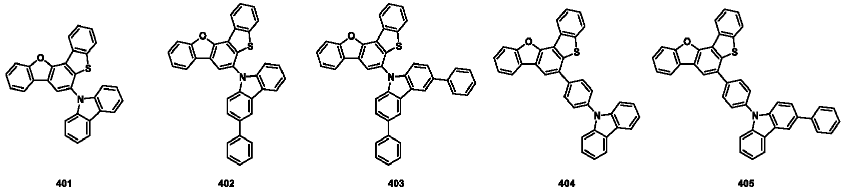

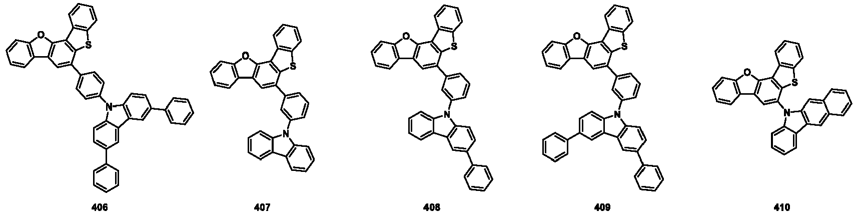

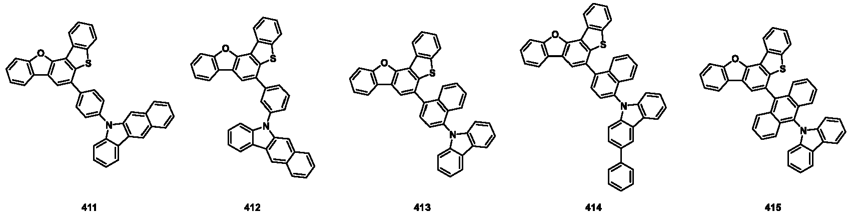

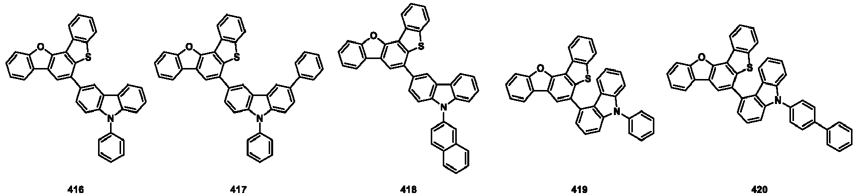

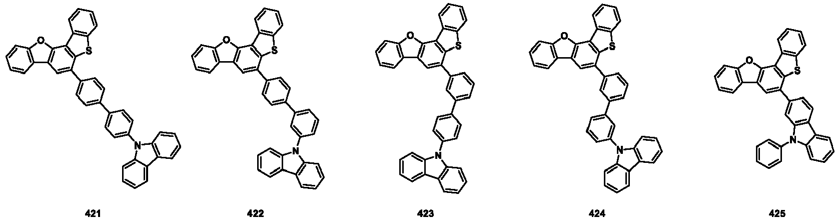

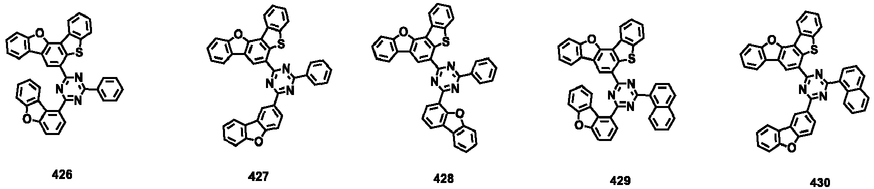

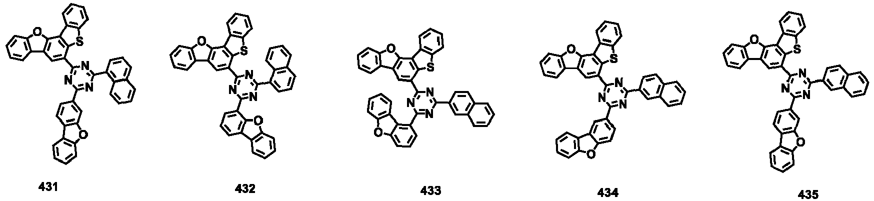

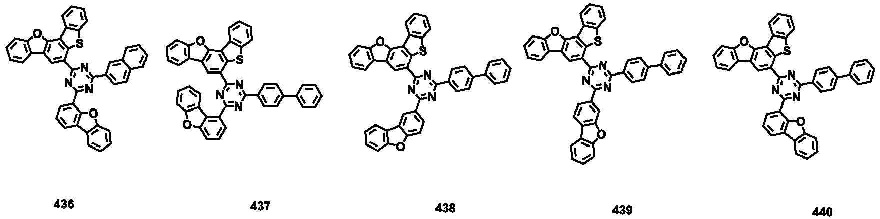

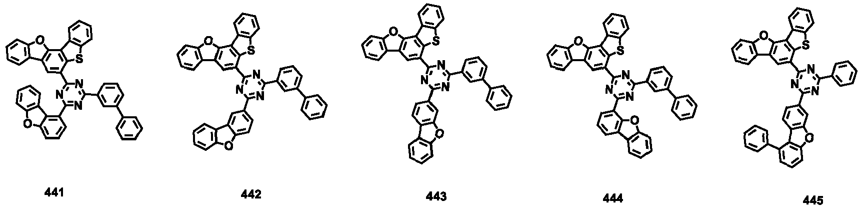

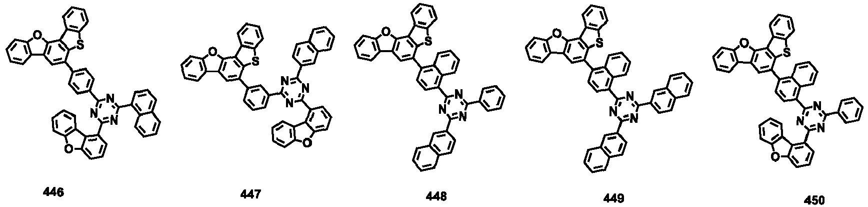

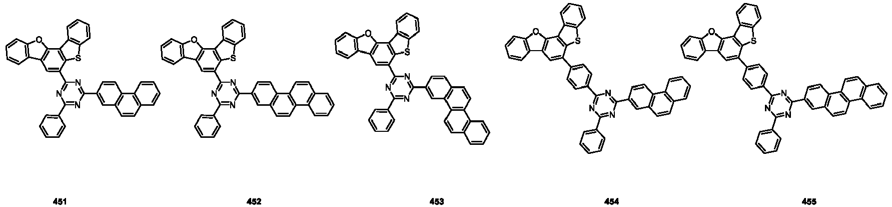

Further, compounds having intrinsic properties of the introduced substituents may be synthesized by introducing various substituents into the structure of Chemical Formula 1 above. For example, substances satisfying the conditions required for each organic material layer may be synthesized by introducing substituents mainly used for a hole injection layer material, a hole transport layer material, a light emitting layer material, an electron transport layer material, and a charge generation layer material used when manufacturing an organic light emitting device into the core structure.

Further, various substituents are introduced into the structure of Chemical Formula 1 above so that the energy band gap may be enabled to be finely controlled, whereas the properties at the interface between organic materials may be improved, and the use of the substances may be enabled to be diversified.

Meanwhile, the heterocyclic compound is excellent in thermal stability by having a high glass transition temperature (Tg). Such an increase in thermal stability becomes an important factor providing driving stability to the device.

The heterocyclic compound according to an embodiment of the present application may be prepared by a multi-step chemical reaction. Some intermediate compounds may be prepared first, and the compound of Chemical Formula 1 may be prepared from the intermediate compounds. More specifically, the heterocyclic compound according to an embodiment of the present application may be prepared based on Preparation Examples to be described later.

Another embodiment of the present application provides an organic light emitting device comprising the heterocyclic compound represented by Chemical Formula 1 above. The "organic light emitting device" may be expressed by terms such as "an organic light emitting diode", "an organic light emitting diode (OLED)", "an OLED device", "an organic electroluminescent device", etc.

In an embodiment of the present application, there is provided an organic light emitting device comprising: a first electrode; a second electrode; and an organic material layer with one or more layers provided between the first electrode and the second electrode, wherein one or more layers of the organic material layer comprise the heterocyclic compound represented by Chemical Formula 1 above.

In an embodiment of the present application, the first electrode may be an anode, and the second electrode may be a cathode.

In another embodiment of the present application, the first electrode may be a cathode, and the second electrode may be an anode.

In an embodiment of the present application, the organic light emitting device may be a blue organic light emitting device, and the heterocyclic compound according to Chemical Formula 1 above may be used as a material of the blue organic light emitting device.

In another embodiment of the present application, the organic light emitting device may be a green organic light emitting device, and the heterocyclic compound according to Chemical Formula 1 above may be used as a material of the green organic light emitting device.

In another embodiment of the present application, the organic light emitting device may be a red organic light emitting device, and the heterocyclic compound according to Chemical Formula 1 above may be used as a material of the red organic light emitting device.

Specific details of the heterocyclic compound represented by Chemical Formula 1 above are the same as described above.

The organic light emitting device of the present application may be manufactured by a conventional method and material for manufacturing an organic light emitting device except that an organic material layer with one or more layers is formed using the above-described heterocyclic compound.

The heterocyclic compound may be formed into an organic material layer by a solution application method as well as a vacuum deposition method when manufacturing an organic light emitting device. Here, the solution application method refers to spin coating, dip coating, inkjet printing, screen printing, spraying, roll coating, or the like, but the present application is not limited thereto.

The organic material layer of the organic light emitting device of the present application may be formed in a single layer structure, but may be formed in a multilayer structure in which an organic material layer with two or more layers is laminated. For example, the organic light emitting device of the present disclosure may have a structure including a hole injection layer, a hole transport layer, a hole auxiliary layer, a light emitting layer, an electron transport layer, an electron injection layer, etc. as an organic material layer. However, the structure of the organic light emitting device is not limited thereto and may include an organic material layer with a smaller number of layers.

In the organic light emitting device of the present application, the organic material layer may include a light emitting layer, and the light emitting layer may comprise the heterocyclic compound. Since, when the heterocyclic compound is used in the light emitting layer, strong charge transfer is possible by spatially separating Highest Occupied Molecular Orbital (HOMO) and Lowest Unoccupied Molecular Orbital (LUMO), the driving voltage, efficiency, and lifespan of the organic light emitting device may become excellent.

The organic light emitting device of the present disclosure may further comprise one or more layers selected from the group consisting of a light emitting layer, a hole injection layer, a hole transport layer, an electron injection layer, an electron transport layer, an electron blocking layer, a hole auxiliary layer, and a hole blocking layer.

FIGS. 1 to 3 exemplify the lamination order of the electrodes and the organic material layer of the organic light emitting device according to an embodiment of the present application. However, it is not intended that the scope of the present application be limited by these drawings, and the structure of an organic light emitting device known in the art may also be applied to the present application.

According to FIG. 1, an organic light emitting device in which an anode 200, an organic material layer 300, and a cathode 400 are sequentially stacked on a substrate 100 is illustrated. However, the present application is not limited to such a structure, and as shown in FIG. 2, an organic light emitting device in which a cathode, an organic material layer, and an anode are sequentially stacked on a substrate may also be implemented.

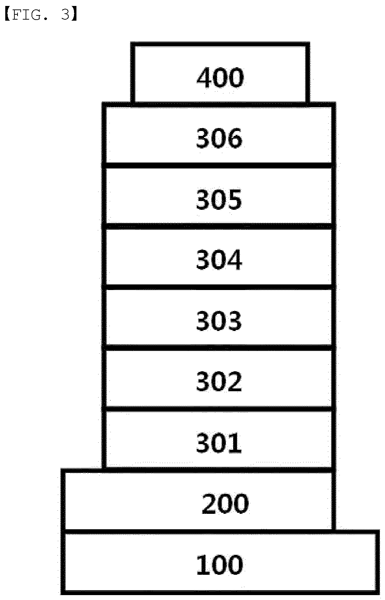

FIG. 3 exemplifies a case in which the organic material layer is multiple layers. The organic light emitting device according to FIG. 3 comprises a hole injection layer 301, a hole transport layer 302, a light emitting layer 303, a hole blocking layer 304, an electron transport layer 305, and an electron injection layer 306. However, the scope of the present application is not limited by such a lamination structure, and the remaining layers except for the light emitting layer may be omitted as needed, and other necessary functional layers may be further added.

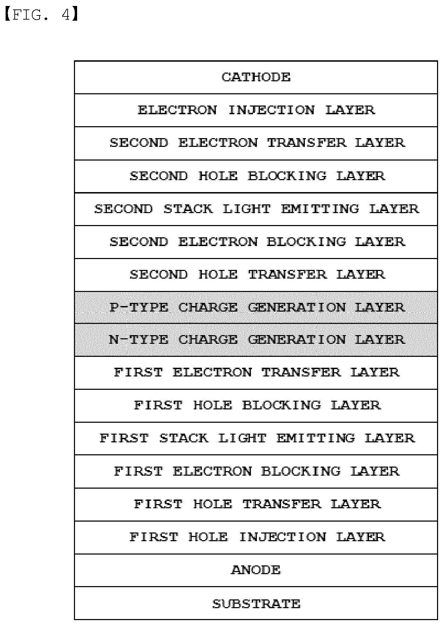

As an organic light emitting device according to an embodiment of the present application, an organic light emitting device having a two-stack tandem structure is schematically shown in FIG. 4 below.

At this time, the first electron blocking layer, the first hole blocking layer, the second hole blocking layer, etc. described in FIG. 4 may be omitted in some cases.

An organic material layer comprising the heterocyclic compound represented by Chemical Formula 1 above may further comprise other materials as needed.

In the organic light emitting device according to an embodiment of the present application, materials other than the heterocyclic compound of Chemical Formula 1 above are exemplified below, but these are for illustration only and not for limiting the scope of the present application, and may be substituted with materials known in the art.

As an anode material, materials having a relatively high work function may be used, and transparent conductive oxides, metals, conductive polymers, or the like may be used. Specific examples of the anode material may include: metals such as vanadium, chromium, copper, zinc, and gold, or alloys thereof; metal oxides such as zinc oxide, indium oxide, indium tin oxide (ITO), and indium zinc oxide (IZO) ; combinations of metals such as ZnO:Al or SnO₂:Sb and oxides; conductive polymers such as poly(3-methylthiophene), poly[3,4-(ethylene-1,2-dioxy)thiophene] (PEDOT), polypyrrole, and polyaniline; etc., but the present application is not limited thereto.

As a cathode material, materials having a relatively low work function may be used, and metals, metal oxides, conductive polymers, or the like may be used. Specific examples of the cathode material may include: metals such as magnesium, calcium, sodium, potassium, titanium, indium, yttrium, lithium, gadolinium, aluminum, silver, tin, and lead, or alloys thereof; a multilayer structure material such as LiF/Al or LiO₂/Al; etc., but the present application is not limited thereto.

As a hole injection material, known hole injection materials may also be used, and for example, phthalocyanine compounds such as copper phthalocyanine, etc. disclosed in US Pat. No. 4,356,429, or starburst-type amine derivatives disclosed in the literature [Advanced Material, 6, p.677 (1994)] such as Tris(4-carbazoyl-9-ylphenyl) amine (TCTA), 4, 4', 4"-Tris [phenyl (m-tolyl)amino]triphenylamine (m-MTDATA), 1,3,5-tris[4-(3-methylphenylphenylamino)phenyl]benzene (m-MTDAPB), and a soluble conductive polymer of polyaniline/dodecylbenzenesulfonic acid or poly(3,4-ethylenedioxythiophene)/poly(4-styrenesulfonate), polyaniline/camphor sulfonic acid or polyaniline/poly(4-styrene sulfonate), etc. may be used.

As a hole transport material, pyrazoline derivatives, arylamine-based derivatives, stilbene derivatives, triphenyldiamine derivatives, etc. may be used, and low molecular weight or high molecular weight materials may also be used.

As an electron transport material, metal complexes or the like of oxadiazole derivatives, anthraquinodimethane and its derivatives, benzoquinone and its derivatives, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, and 8-hydroxyquinoline and its derivatives may be used, and high molecular weight materials as well as low molecular weight materials may also be used.

As an electron injection material, for example, LiF is typically used in the art, but the present application is not limited thereto.

As a light emitting material, a red, green, or blue light emitting material may be used, and two or more light emitting materials may be mixed and used as needed. At this time, two or more light emitting materials may be deposited and used as individual sources, or may be premixed to be deposited and used as a single source. Further, as a light emitting material, a fluorescent material may be used, but a phosphorescent material may also be used. As the light emitting material, a material that emits light by combining holes and electrons respectively injected from the anode and the cathode may be used alone, but materials in which the host material and the dopant material involve together in light emission may also be used.

When mixing and using the host of the light emitting material, hosts of the same series may be mixed and used, or hosts of different series may also be mixed and used. For example, any two or more types of materials of n-type host materials or p-type host materials may be selected and used as a host material of the light emitting layer.

In the organic light emitting device of the present application, the organic material layer may include a light emitting layer, and the light emitting layer may comprise the heterocyclic compound as a host material of the light emitting material.

In the organic light emitting device of the present application, the light emitting layer may comprise two or more host materials, and at least one of the host materials may comprise the heterocyclic compound as a host material of the light emitting material.

In the organic light emitting device of the present application, two or more host materials may be pre-mixed and used as the light emitting layer, and at least one of the two or more host materials may include the heterocyclic compound as a host material of a light emitting material.

The pre-mixing means placing and mixing two or more host materials of the light emitting layer in one source of supply before depositing on the organic material layer.

In the organic light emitting device of the present application, the light emitting layer may comprise two or more host materials, the two or more host materials may each include one or more p-type host materials and n-type host materials, and at least one of the host materials may include the heterocyclic compound as a host material of the light emitting material. In this case, the driving voltage, efficiency and lifespan of the organic light emitting device may become excellent.

The organic light emitting device according to an embodiment of the present application may be a top emission type, a back emission type, or a double side emission type depending on materials used.

A heterocyclic compound according to an embodiment of the present application may act on a principle similar to that applied to an organic light emitting device even in an organic electronic device including an organic solar cell, an organic photoreceptor, an organic transistor, etc.

Hereinafter, the present specification will be described in more detail through Examples, but these are only for exemplifying the present application and not for limiting the scope of the present application.

### <Preparation Example>

### <Preparation Example 1> Preparation of Intermediate A

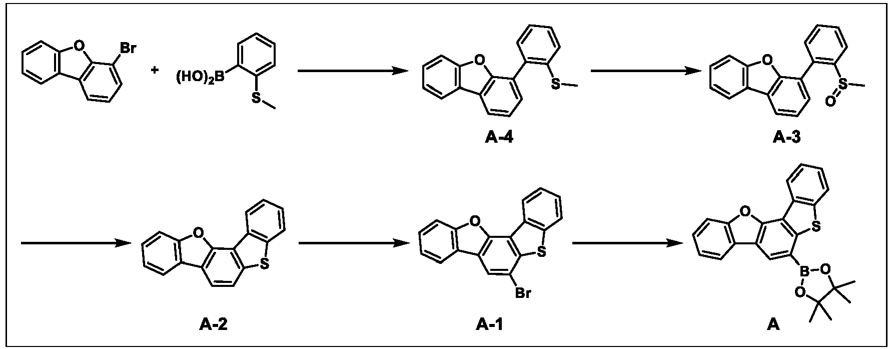

### 1) Preparation of Intermediate A-4

44.2 g (179 mmol) of 4-bromodibenzofuran, 30.0 g (179 mmol) of 2-(methylthio)phenylboronic acid, 10.3 g (8.92 mmol) of Pd(PPh₃)₄, 56.8 g (536 mmol) of Na₂CO₃, 300 mL of toluene, 50 mL of ethanol, and 50 mL of H₂O were put into a 1 L round-bottom flask and stirred at 125°C for 16 hours (h). After completing the reaction, the temperature was lowered to room temperature, and the reaction product was extracted with distilled water, brine, and ethyl acetate (hereinafter referred to as EtOAc). The solution was dried over anhydrous MgSO₄ and concentrated. The concentrated reaction product was purified by a hexane column to obtain 39.4 g (136 mmol)(76%) of Intermediate A-4.

### 2) Preparation of Intermediate A-3

39.4 g (136 mmol) of Intermediate A-4 was put into a 2 L round-bottom flask and dissolved in tetrahydrofuran (hereinafter referred to as THF) and acetic acid, and then 35 wt% (18 mL) of hydrogen peroxide was slowly added dropwise. Stirring was performed at room temperature for 8 hours. After completing the reaction, the solvent was removed from the reaction product using a rotary evaporator, and the solvent-removed reaction product was extracted with DCM, brine, and distilled water. The solution was dried over anhydrous MgSO₄ and concentrated. After performing concentration, 41.6 g (136 mmol)(100%) of Intermediate A-3 was obtained, and the next reaction was carried out immediately. DCM refers to dichloromethane (hereinafter referred to as DCM).

### 3) Preparation of Intermediate A-2

41.6 g (136 mmol) of Intermediate A-3 was put into a 1 L round-bottom flask, dissolved in 180 mL of Triflic acid, and then stirred at room temperature for 2 days. Thereafter, 420 mL of pyridine and 10 mL of an aqueous K₂CO₃ saturated solution were slowly added dropwise to the solution. The mixture was refluxed for 4 hours by gradually raising the temperature. After completing the reaction, the reaction product was cooled to room temperature, and washed with distilled water and methanol. After drying the solid, it was separated by a column under the condition of DCM:Hexane=1:1 to obtain 30.53 g (111 mmol)(82%) of Intermediate A-2.

### 4) Preparation of Intermediate A-1

30.5 g (111 mmol) of Intermediate A-2 was put into a 1 L round-bottom flask and dissolved in 300 mL of chloroform, and then 6.4 mL (117 mmol) of bromine was slowly added dropwise. Thereafter, after stirring the mixture at room temperature, when the reaction was completed, the reaction product was washed with methanol, and the solid was dried. After dissolving the dried solid in hot DCB, it was separated by a column under the condition of DCB to obtain 31.4 g (80%) of Intermediate A-1. DCB refers to 1,2-dichlorobenzene (hereinafter referred to as DCB).

### 5) Preparation of Intermediate A

10.0 g (28.3 mmol) of Intermediate A-1, 10.8 g (42.5 mmol) of bis(pinacolato)diboron, 1.04 g (1.42 mmol) of Pd(dppf)Cl₂, 5.55 g (56.6 mmol) of potassium acetate (hereinafter referred to as KOAc), and 100 mL of 1,4-dioxane were put into a 250 mL round-bottom flask, and refluxed at 130°C for 3 hours. After sieving inorganic materials from a reaction product while it was hot, the reaction product was washed with DCM. The solution was dried with a rotary evaporator, and then separated by a column under the condition of DCM;Hexane=1:2 to obtain 10.1 g (25.2 mmol)(89%) of Intermediate A.

### <Preparation Example 2> Preparation of Intermediate B

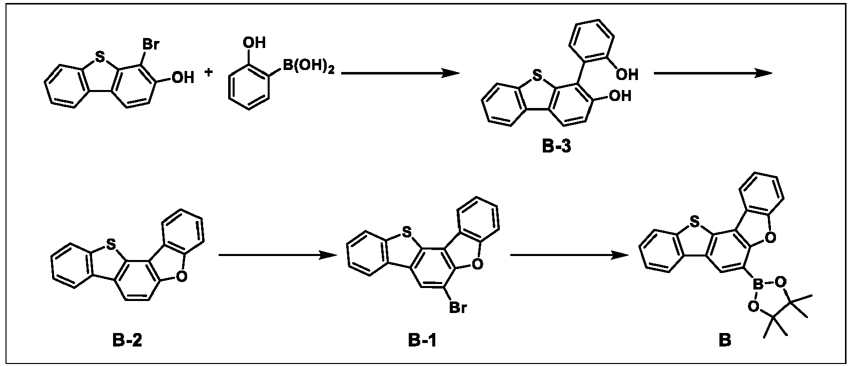

### 1) Preparation of Intermediate B-3

60.7 g (218 mmol) of 4-bromodibenzo[b,d]thiophen-3-ol, 30.0 g (218 mmol) of (2-hydroxyphenyl)boronic acid, 12.6 g (10.9 mmol of Pd(PPh₃)₄, 46.11 g (435 mmol) of Na₂CO₃, 300 mL of toluene, 50 mL of ethanol, and 50 mL of H₂O were put into a 1 L round-bottom flask, and stirred at 130°C for 14 hours. After completing the reaction, the temperature was lowered to room temperature, and the reaction product was extracted with distilled water, brine, and EtOAc. Thereafter, the solution was dried over anhydrous MgSO₄ and concentrated. The concentrated reaction product was purified by a column under the condition of MC to obtain 50.2 g (172 mmol)(79%) of Intermediate B-3.

### 2) Preparation of Intermediate B-2

50.2 g (172 mmol) of Intermediate B-3 was put into a 1 L round-bottom flask, dissolved in 28.6 mL (206 mmol) of p-toluenesulfonic acid and 500 mL of toluene under nitrogen condition, and then stirred at 100°C for 14 hours. After completing the reaction, the reaction product was cooled to room temperature, and extracted with an aqueous NaHCO₃ saturated solution, distilled water, and EtOAc. Thereafter, after drying the solution over anhydrous MgSO₄, the reaction product was concentrated by a rotary evaporator. The concentrated reaction product was purified by a column under the condition of MC:Hex=1:1 to obtain 26.4 g (96.2 mmol) (56%) of Intermediate B-2.

### 3) Preparation of Intermediate B-1

26.4 g (96.2 mmol) of Intermediate B-2 was put into a 1 L round-bottom flask and dissolved in 260 mL of chloroform, and then 5.6 mL (101 mmol) of bromine was slowly added dropwise. After stirring the mixture at room temperature, when the reaction was completed, the reaction product was washed with methanol, and the solid was dried. After dissolving the dried solid in hot DCB, it was separated by a column under the condition of DCB to obtain 29.2 g (82.8 mmol)(86%) of Intermediate B-1.

### 4) Preparation of Intermediate B

10.0 g (28.3 mmol) of Intermediate B-1, 10.8 g (42.5 mmol) of bis(pinacolato)diboron, 1.04 g (1.42 mmol) of Pd(dppf)Cl₂, 5.56 g (56.6 mmol) of KOAc, and 100 mL of 1,4-dioxane were put into a 250 mL round-bottom flask, and refluxed at 130°C for 2 hours. After sieving inorganic materials from a reaction product while it was hot, the reaction product was washed with DCM. The solution was dried with a rotary evaporator, and then separated by a column under the condition of DCM;Hexane=1:2 to obtain 10.1 g (25.2 mmol) (89%) of Intermediate B.

### <Preparation Example 3> Preparation of Intermediate C

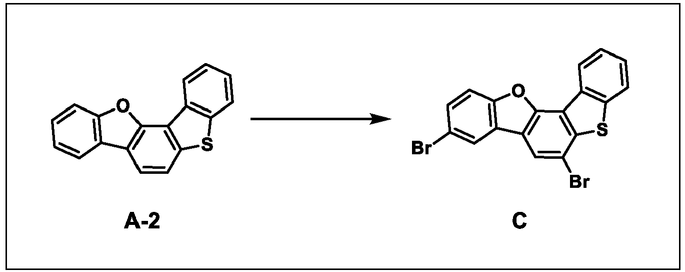

10.0 g (36.5 mmol) of Intermediate A-2 was put into a 250 mL round-bottom flask and dissolved in 100 mL of chloroform, and then 4.2 mL (76.5 mmol) of bromine was slowly added dropwise. After stirring the mixture at room temperature, when the reaction was completed, the reaction product was washed with methanol, and the solid was dried. After dissolving the dried solid in hot DCB, it was separated by a column under the condition of DCB to obtain 9.61 g (61%) of Intermediate C.

### <Preparation Example 4> Preparation of Compounds 1, 2, 4, 5, 6, 9, 10, 12, 14, 36, 38, 41, 202, 236, 310, 426, 433, 437, and 441

### 1) Preparation of Compound 1

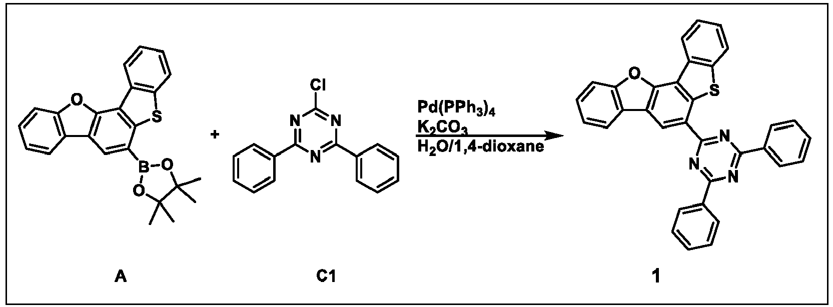

10.0 g (25.0 mmol) of Intermediate A, 6.55 g (24.5 mmol) of 2-chloro-4,6-diphenyl-1,3,5-triazine (Compound C1), 1.44 g (1.2 mmol) of Pd(PPh₃)₄, 6.91 g (50.0 mmol) of K₂CO₃, 30 mL of distilled water, and 100 mL of 1,4-dioxane were put into a 500 mL round-bottom flask, and refluxed at 130°C for 3 hours. After completing the reaction, the temperature was lowered to room temperature, and the reaction product was washed with distilled water and methanol. After drying the solid and dissolving the dried solid in hot DCB, it separated by a column under the condition of DCB to obtain 10.1 g (20.0 mmol) (80%) of Compound 1.

### 2) Preparation of Compound 2

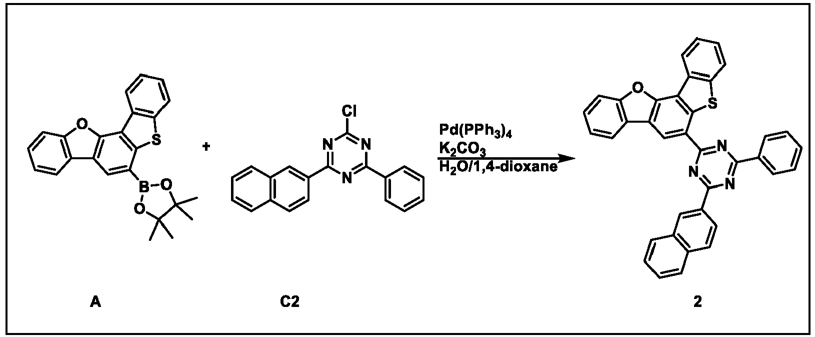

10.0 g (25.0 mmol) of Intermediate A, 7.78 g (24.5 mmol) of 2-chloro-4-(naphthalen-2-yl)-6-phenyl-1,3,5-triazine (Compound C2), 1.44 g (1.2 mmol) of Pd(PPh₃)₄, 6.91 g (50.0 mmol) of K₂CO₃, 30 mL of distilled water, and 100 mL of 1,4-dioxane were put into a 500 mL round-bottom flask, and refluxed at 130°C for 2 hours. After completing the reaction, the temperature was lowered to room temperature, and the reaction product was washed with distilled water and methanol. After drying the solid and dissolving the dried solid in hot DCB, it was separated by a column under the condition of DCB to obtain 11.8 g (21.2 mmol)(85%) of Compound 2.

### 3) Preparation of Compound 4

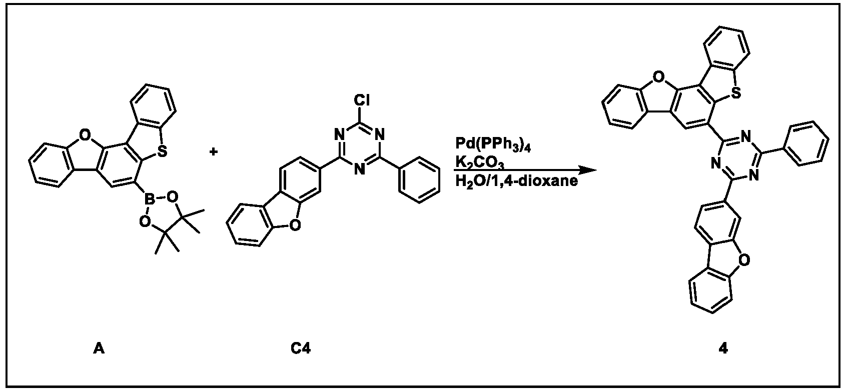

10.0 g (25.0 mmol) of Intermediate A, 8.76 g (24.5 mmol) of 2-chloro-4-(dibenzo[b,d]furan-3-yl)-6-phenyl-1,3,5-triazine (Compound C2), 1.44 g (1.2 mmol) of Pd(PPh₃)₄, 6.91 g (50.0 mmol) of K₂CO₃, 30 mL of distilled water, and 100 mL of 1,4-dioxane were put into a 500 mL round-bottom flask, and refluxed at 130°C for 3 hours. After completing the reaction, the temperature was lowered to room temperature, and the reaction product was washed with distilled water and methanol. After drying the solid and dissolving the dried solid in hot DCB, it was separated by a column under the condition of DCB to obtain 12.1 g (20.3 mmol)(81%) of Compound 4.

### 4) Preparation of Compounds 5, 6, 9, 10, 12, 14, 202, 236, 310, 426, 433, 437, and 441

Compounds 5, 6, 9, 10, 12, 14, 202, 236, 310, 426, 433, 437, and 441 were prepared and synthesized in the same manner as in the preparation of Compound 1 above except that C of Table 1 below was used instead of Compound C1 in the preparation of Compound 1 above.

### 5) Preparation of Compounds 36, 38, and 41

Compounds 36, 38, and 41 were prepared and synthesized in the same manner as in the preparation of Compound 1 above except that Intermediate B of Table 1 below was used instead of Intermediate A in the preparation of Compound 1 above, and C of Table 1 below was used instead of Compound C1.

**[Table 1]**

| A/B | C | P | P yield |
|---|---|---|---|
| 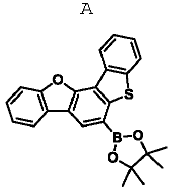 | 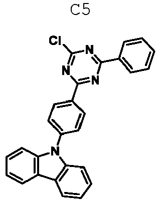 | 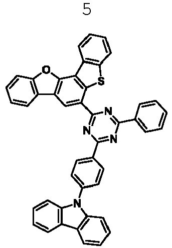 | 85% |
| 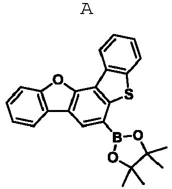 | 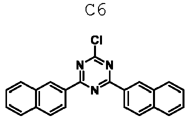 | 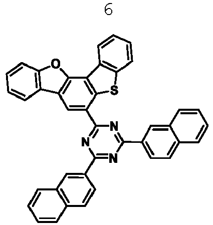 | 84% |
| 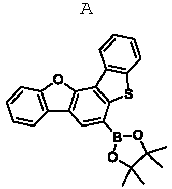 | 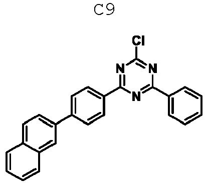 | 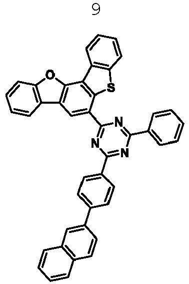 | 79% |
| 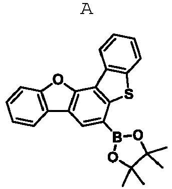 | 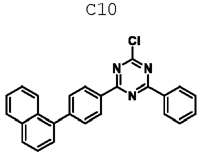 | 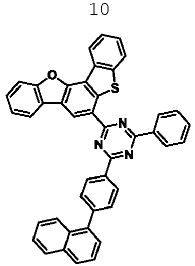 | 80% |
| 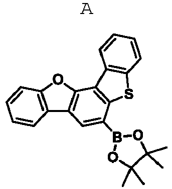 | 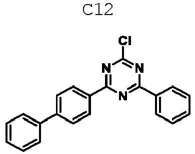 | 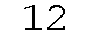 | 81% |
| | | 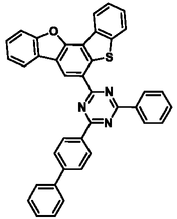 | |
| 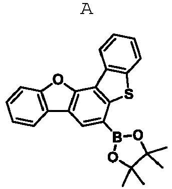 | 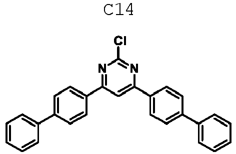 | 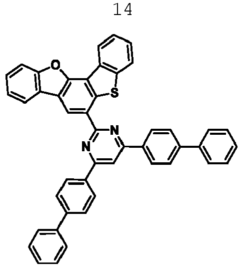 | 82% |
| 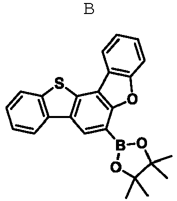 | 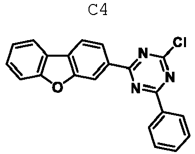 | 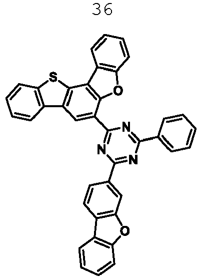 | 88% |
| 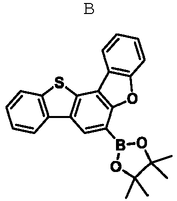 | 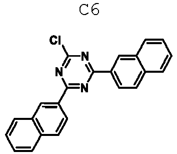 | 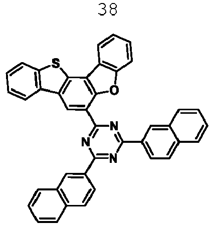 | 78% |
| 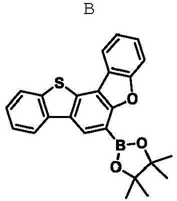 | 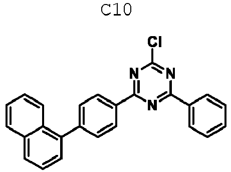 | 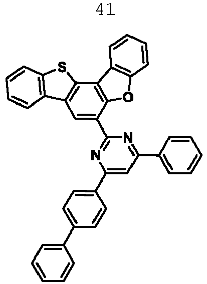 | 85% |
| 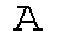 | 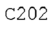 | 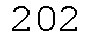 | 79% |
| 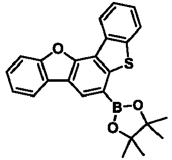 | 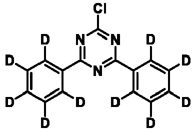 | 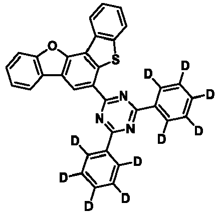 | |
| 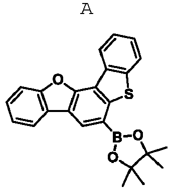 | 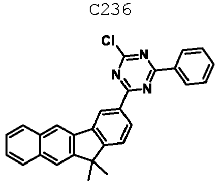 | 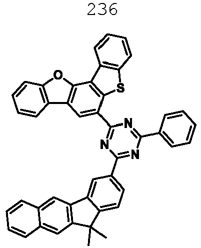 | 81% |
| 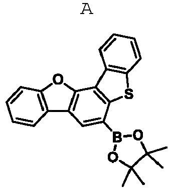 | 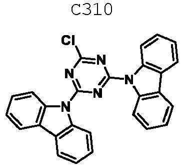 | 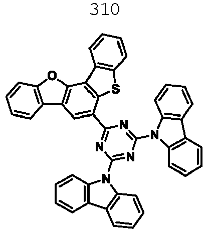 | 76% |
| 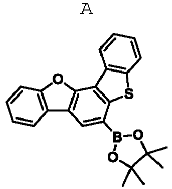 | 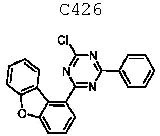 | 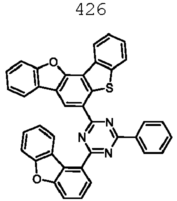 | 90% |
| 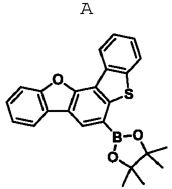 | 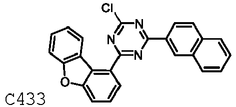 | 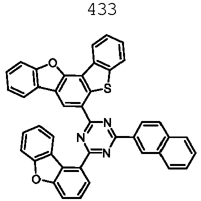 | 92% |
| 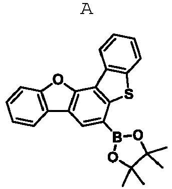 | 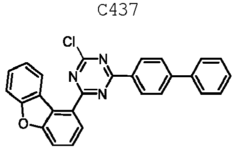 | 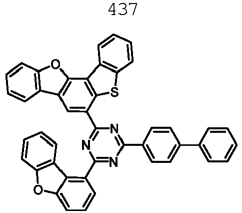 | 89% |
| 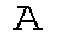 | 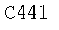 | 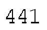 | 86% |
| 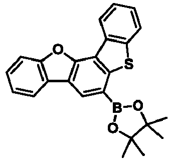 | 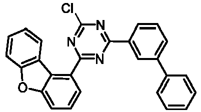 | 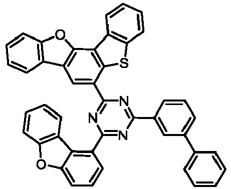 | |

### 6) Preparation of Compound 249

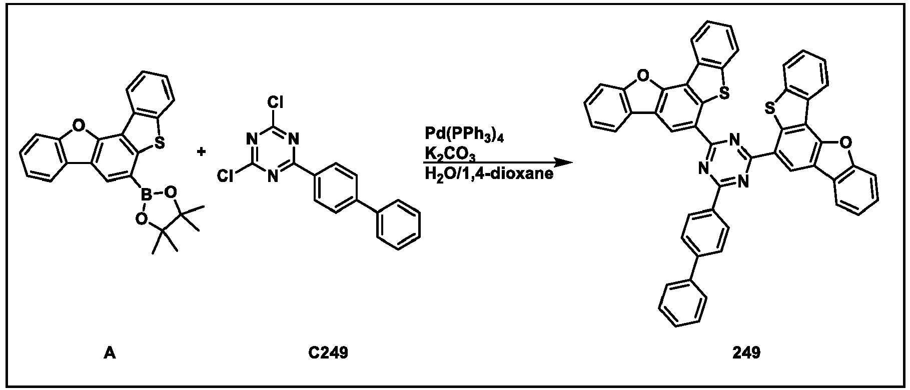

20.0 g (50.0 mmol) of Intermediate A, 7.40 g (24.5 mmol) of 2-([1,1'-biphenyl]-4-yl)-4,6-dichloro-1,3,5-triazine (Compound C249), 1.44 g (1.2 mmol) of Pd(PPh₃)₄, 6.91 g (50.0 mmol) of K₂CO₃, 30 mL of distilled water, and 100 mL of 1,4-dioxane were put into a 500 mL round-bottom flask, and refluxed at 130°C for 5 hours. After completing the reaction, the temperature was lowered to room temperature, and the reaction product was washed with distilled water and methanol. After drying the solid and dissolving the dried solid in hot DCB, it was separated by a column under the condition of DCB to obtain 16.4 g (21.1 mmol)(86%) of Compound 249.

### <Preparation Example 5> Preparation of Compounds 17, 23, 26, 446, and 450

### 1) Preparation of Compound 17

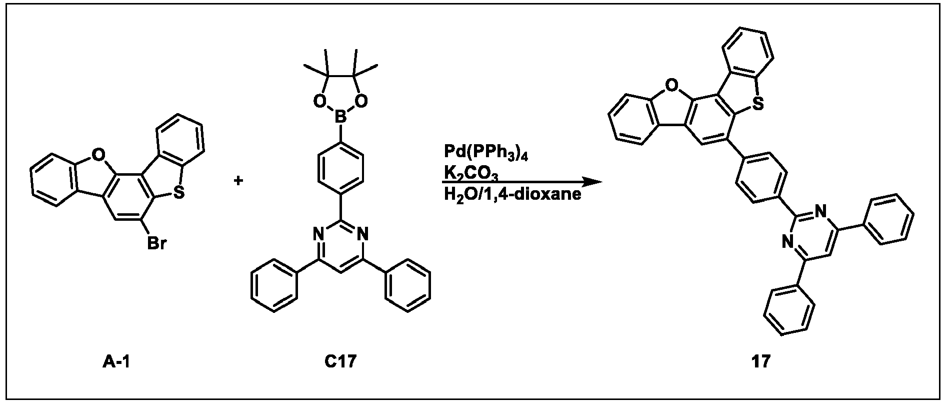

10.0 g (28.3 mmol) of Intermediate A-1, 12.9 g (29.7 mmol) of 4,6-diphenyl-2-(4-(4,4,5,5-tetramethyl-1,3,2-dioxaborolan-2-yl)phenyl)pyrimidine (Compound C17), 1.64 g (1.4 mmol) of Pd(PPh₃)₄, 7.82 g (56.6 mmol) of K₂CO₃, 30 mL of distilled water, and 100 mL of 1,4-dioxane were put into a 500 mL round-bottom flask, and refluxed at 130°C for 4 hours. After completing the reaction, the temperature was lowered to room temperature, and the reaction product was washed with distilled water and methanol. After drying the solid and dissolving the dried solid in hot DCB, it was separated by a column under the condition of DCB to obtain 14.3 g (24.6 mmol) (87%) of Compound 17.

### 2) Preparation of Compounds 23, 26, 446, and 450

Compounds 23, 26, 446, and 450 were prepared and synthesized in the same manner as in the preparation of Compound 17 above except that C of Table 2 below was used instead of Compound C17 in the preparation of Compound 17 above.

**[Table 2]**

| A-1 | C | P | P yield |
|---|---|---|---|
| 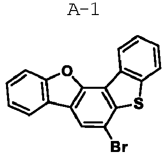 | 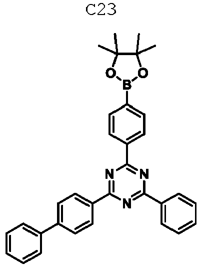 | 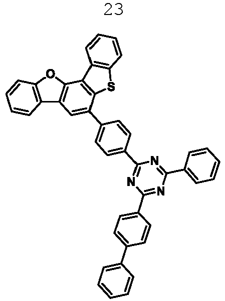 | 90% |
| 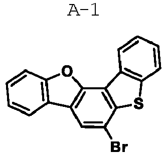 | 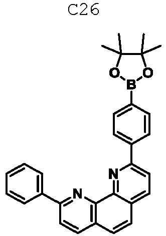 | 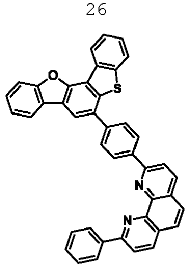 | 88% |
| 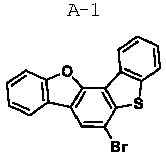 | 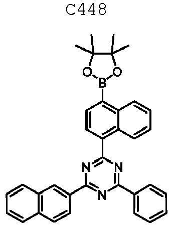 | 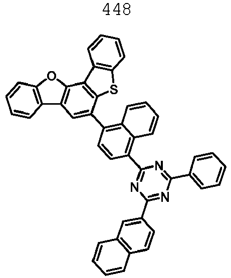 | 93% |
| 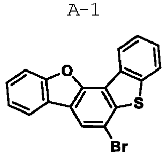 | 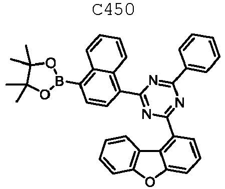 | 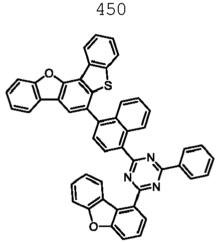 | 91% |

### <Preparation Example 6> Preparation of Compounds 51, 67, 68, 70, 72, 80, 84, 151, 152, 165, 256, 258, 276, 351, and 386

### 1) Preparation of Compound 51

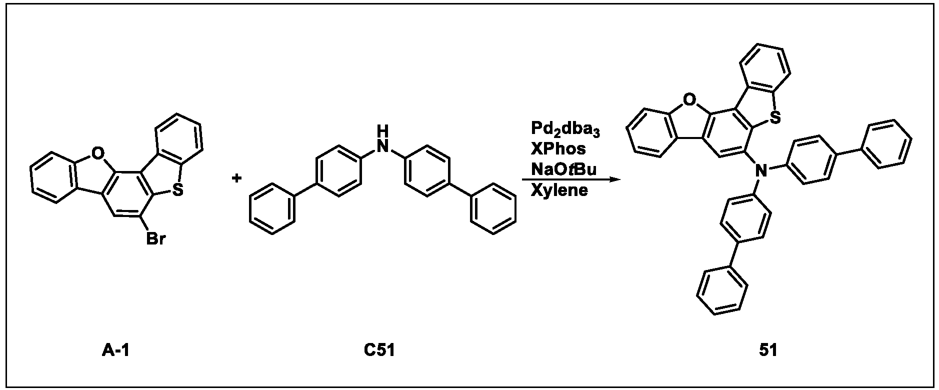

10.0 g (28.3 mmol) of Intermediate A-1, 10.01 g (31.1 mmol) of di([1,1'-biphenyl]-4-yl)amine (Compound C51), 1.30 g (1.4 mmol) of Pd₂dba₃, 1.35 g (2.8 mmol) of XPhos, 5.44 g (56.6 mmol) of sodium tert-butoxide (hereinafter referred to as NaOtBu), and 100 mL of xylene were put into a 500 mL round-bottom flask, and refluxed at 130°C for 2 hours. After completing the reaction, the temperature was lowered to room temperature, and filtration was performed with a Celite filter. After concentrating the reaction product, the concentrated reaction product was separated by a column under the condition of MC:Hexane=1:4 to obtain 15.6 g (26.3 mmol)(93%) of Compound 51.

### 1) Preparation of Compounds 67, 68, 70, 72, 80, 84, 256, 258, 276, and 351

Compounds 67, 68, 70, 72, 80, 84, 256, 258, 276, and 351 were prepared and synthesized in the same manner as in the preparation of Compound 51 above except that C of Table 3 below was used instead of Compound C51 in the preparation of Compound 51 above.

### 2) Preparation of Compounds 151, 152, 153, and 386

Compounds 151, 152, 153, and 386 were prepared and synthesized in the same manner as in the preparation of Compound 51 above except that Intermediate B-1 was used instead of Intermediate A-1 in the preparation of Compound 51 above, and C of Table 3 below was used instead of Compound C51.

**[Table 3]**

| A-1 | C | P | P yield |
|---|---|---|---|
| 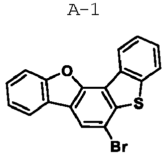 | 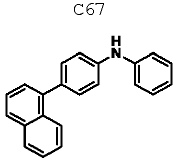 | 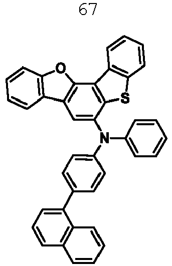 | 910 |
| 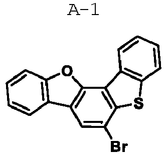 | 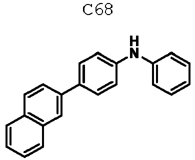 | 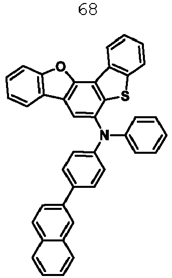 | 89% |
| 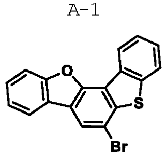 | 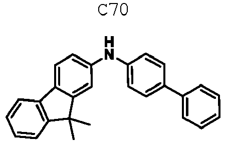 | 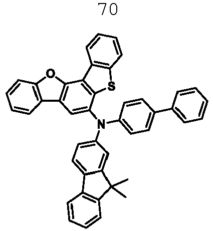 | 88% |
| 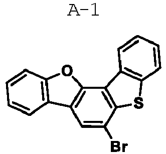 | 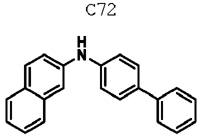 | 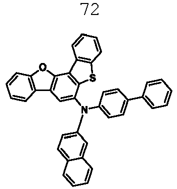 | 83% |
| 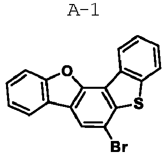 | 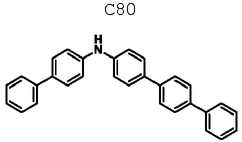 | 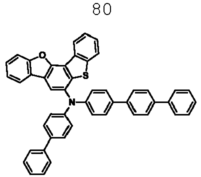 | 95% |
| 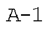 | 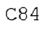 | 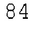 | 94% |
| 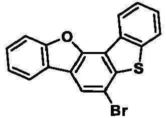 | 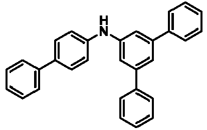 | 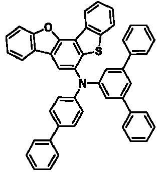 | |
| 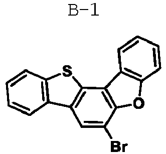 | 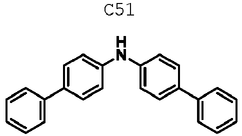 | 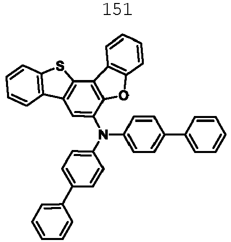 | 88% |
| 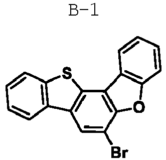 | 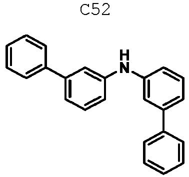 | 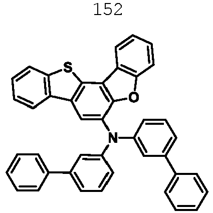 | 91% |
| 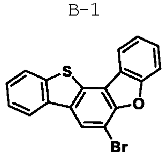 | 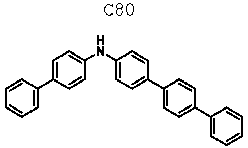 | 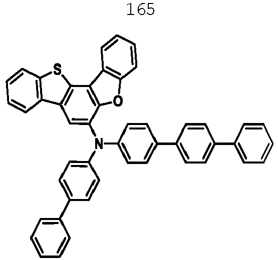 | 85% |
| 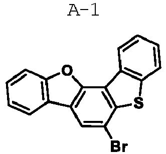 | 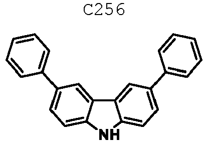 | 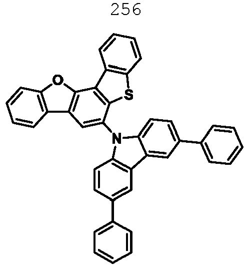 | 89% |
| 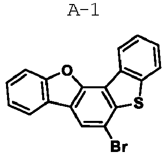 | 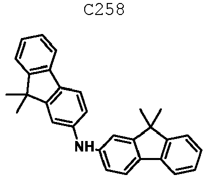 | 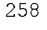 | 88% |
| | | 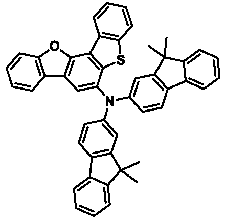 | |
| 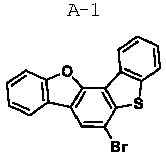 | 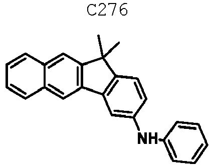 | 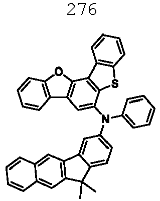 | 87% |
| 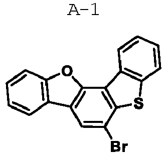 | 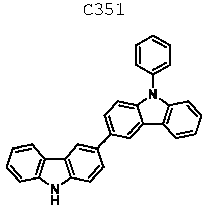 | 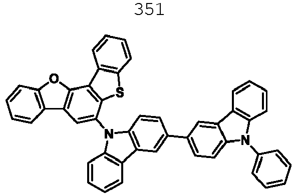 | 86% |
| 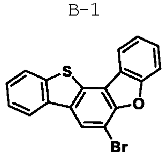 | 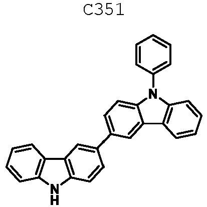 | 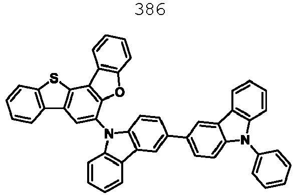 | 89% |

### <Preparation Example 7> Preparation of Compounds 95, 97, 106, 109, 118, 122, 126, 140, 141, 143, 172, 173, 201, 208, and 384

### 1) Preparation of Compound 95

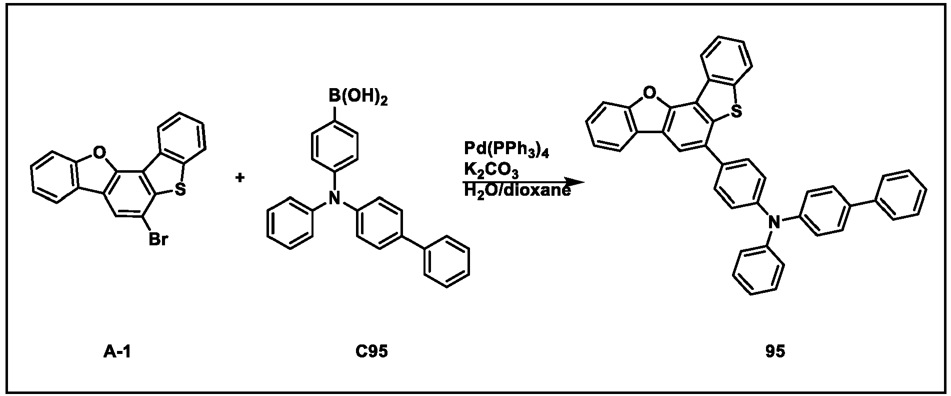

10.0 g (28.3 mmol) of Intermediate A-1, 11.36 g (31.1 mmol) of (4-([1,1'-biphenyl]-4-yl(phenyl)amino)phenyl)boronic acid (Compound C95), 1.64 g (1.4 mmol) of Pd(PPh₃)₄, 7.82 g (56.6 mmol) of K₂CO₃, 30 mL of distilled water, and 100 mL of 1,4-dioxane were put into a 500 mL round-bottom flask, and refluxed at 130°C for 4 hours. After completing the reaction, the temperature was lowered to room temperature, and filtration was performed with a Celite filter. After concentrating the reaction product, the concentrated reaction product was separated by a column under the condition of MC:Hexane=1:4 to obtain 14.5 g (24.3 mmol)(86%) of Compound 95.

### 2) Preparation of Compounds 97, 106, 109, 118, 122, 126, 140, 141, 143, 201, 208, and 384

Compounds 97, 106, 109, 118, 122, 126, 140, 141, 143, 201, 208, and 384 were prepared and synthesized in the same manner as in the preparation of Compound 95 above except that C of Table 4 below was used instead of Compound C95 in the preparation of Compound 95 above.

### 3) Preparation of Compounds 172 and 173

Compounds 172 and 173 were prepared and synthesized in the same manner as in the preparation of Compound 95 above except that Intermediate B-1 was used instead of Intermediate A-1 in the preparation of Compound 95 above, and C of Table 4 below was used instead of Compound C95.

**[Table 4]**

| A-1 | C | P | P yield |
|---|---|---|---|
| 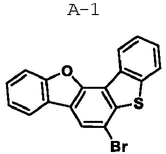 | 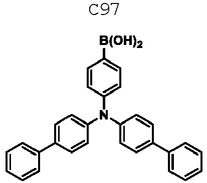 | 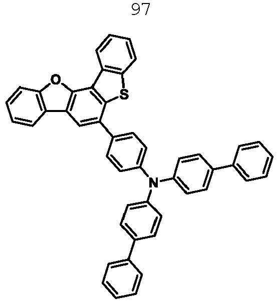 | 84% |
| 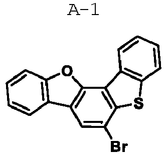 | 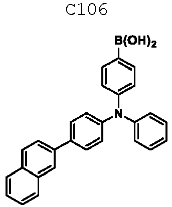 | 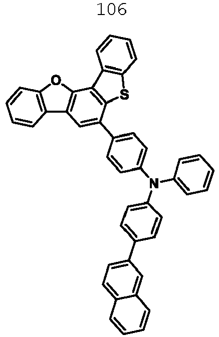 | 88% |
| 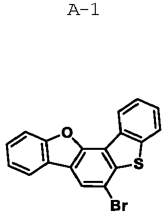 | 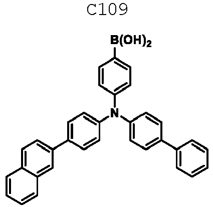 | 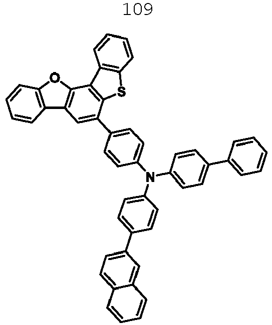 | 83% |
| 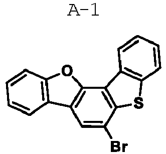 | 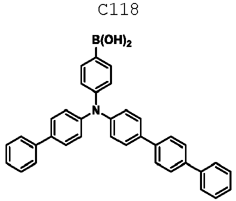 | 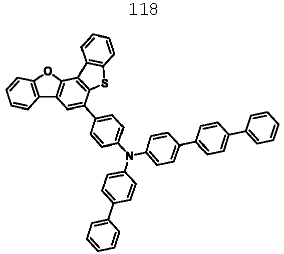 | 89% |
| 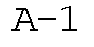 | 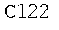 | 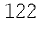 | 88% |
| 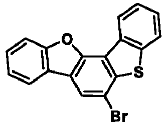 | 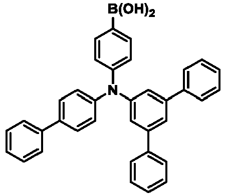 | 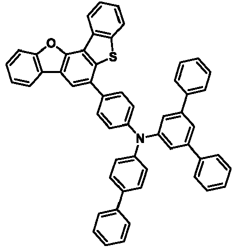 | |
| 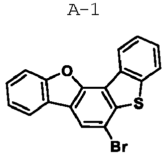 | 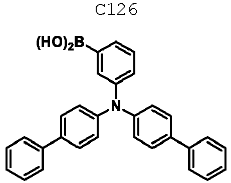 | 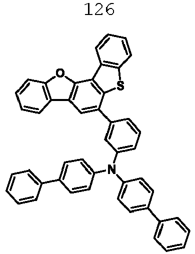 | 84% |
| 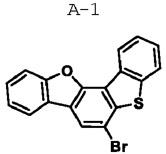 | 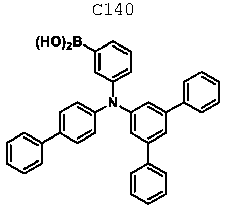 | 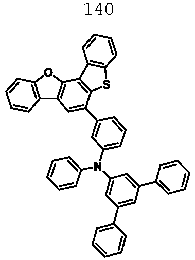 | 82% |
| 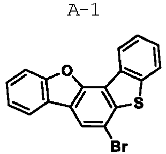 | 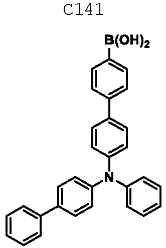 | 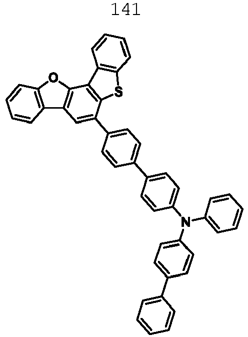 | 79% |
| 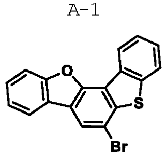 | 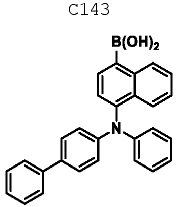 | 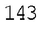 | 85% |
| | | 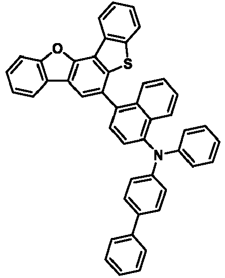 | |
| 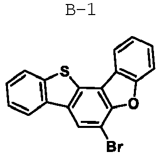 | 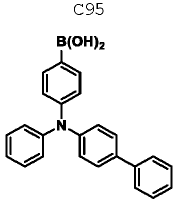 | 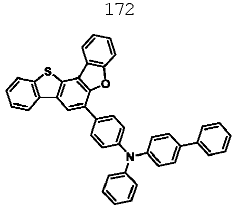 | 83% |
| 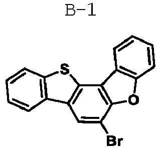 | 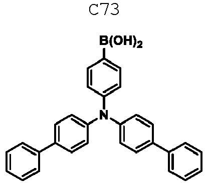 | 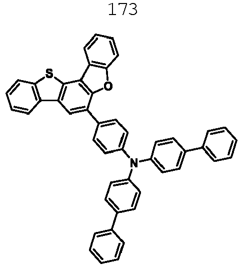 | 84% |
| 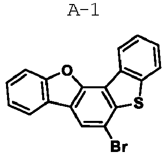 | 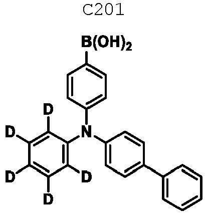 | 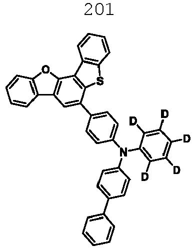 | 79% |
| 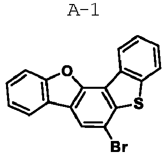 | 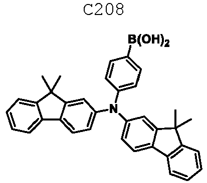 | 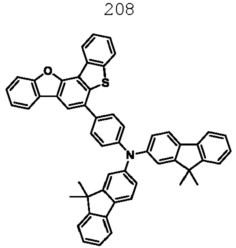 | 81% |
| 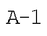 | 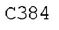 | 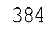 | 85% |
| 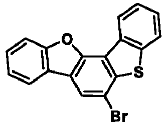 | 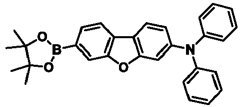 | 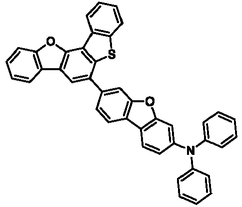 | |

### <Preparation Example 8> Preparation of Compounds 147, 148, and 149

### 1) Preparation of Compound 147

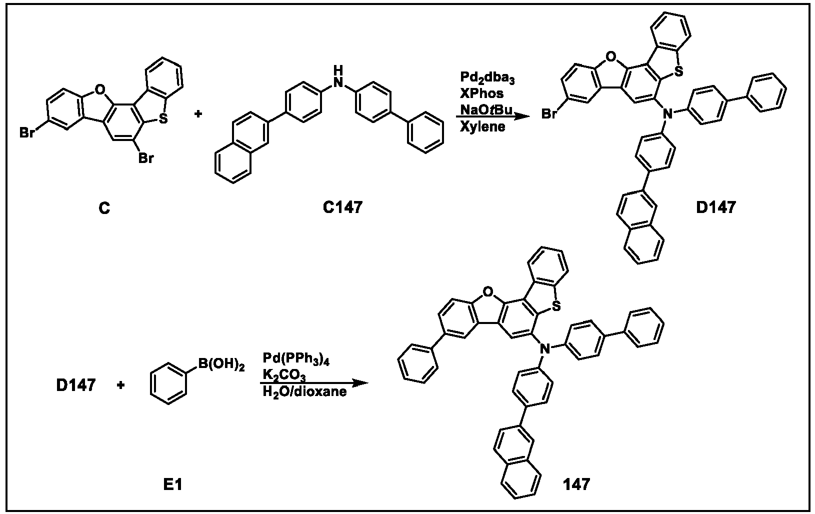

10.0 g (23.6 mmol) of 6,9-dibromobenzo[b]benzo[4,5]thieno[2,3-g]benzofuran (Intermediate C), 8.78 g (23.6 mmol) of N-(4-(naphthalen-2-yl)phenyl)-[1,1'-biphenyl]-4-amine (Compound C147), 1.06 g (1.16 mmol) of Pd₂dba₃, 1.13 g (2.36 mmol) of XPhos, 4.54 g (47.2 mmol) of NaOtBu, and 100 mL of xylene were put into a 500 mL round-bottom flask, and refluxed at 130°C for 4 hours. After completing the reaction, the temperature was lowered to room temperature, and filtration was performed with a Celite filter. After concentrating the reaction product, the concentrated reaction product was separated by a column under the condition of MC:Hexane=1:4 to obtain 10.2 g (14.1 mmol)(61%) of N-([1,1'-biphenyl]-4-yl)-9-bromo-N-(4-(naphthalen-2-yl)phenyl)benzo[b]benzo[4,5]thieno[2,3-g]benzofuran-6-amine (Compound D147). 10.0 g (13.8 mmol) of D147, 2.53 g (20.8 mmol) of phenylboronic acid (Compound E1), 0.80 g (0.7 mmol) of Pd(PPh₃)₄, 3.81 g (27.6 mmol) of K₂CO₃, 30 mL of distilled water, and 100 mL of 1,4-dioxane were put into a 500 mL round-bottom flask, and refluxed at 130°C for 4 hours. After completing the reaction, the temperature was lowered to room temperature, and filtration was performed with a Celite filter. After concentrating the reaction product, the concentrated reaction product was separated by a column under the condition of MC:Hexane=1:4 to obtain 9.36 g (13.0 mmol) (94%) of Compound 147.

### 2) Preparation of Compounds 148 and 149

Compounds 148 and 149 were prepared and synthesized in the same manner as in the preparation of Compound 147 above except that C and E of Table 5 below were used instead of Compound C147 and Compound E1 in the preparation of Compound 147 above.

**[Table 5]**

| Intermediate C | C | E | P | P yield |
|---|---|---|---|---|
| 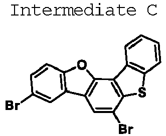 | 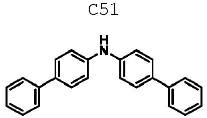 | 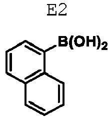 | 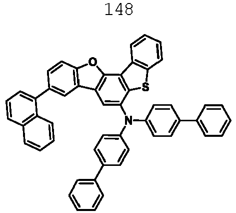 | 54% |
| 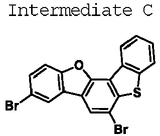 | 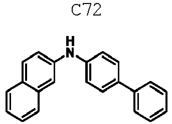 | 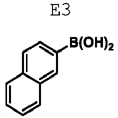 | 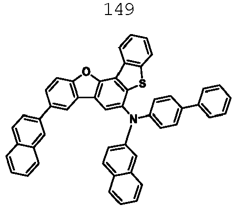 | 57% |

The compounds described in the present specification were prepared in the same manner as in Preparation Examples above, and the synthesis confirmation results of the prepared compounds are shown in Tables 6 and 7 below. Table 6 below is the measurement values of ¹H NMR(CDCl₃, 400 Mz), and Table 7 below is the measurement values of the FD-mass spectrometer (FD-MS: Field desorption mass spectrometry).

**[Table 6]**

| Compound | ¹H NMR(CDCl₃, 400Mz) |
|---|---|
| 1 | δ = 8.98-8.96 (d, 2H), 8.92-8.90 (m, 3H), 8.23~8.21 (d, 1H), 7.98~7.96 (d, 2H), 7.71 (s, 1H), 7.60~7.43 (m, 8H), 7.41~7.39 (t, 1H), 7.38~7.36 (t, 1H) |
| 2 | δ = 9.21 (s, 1H), 8.99~8.97 (d, 2H), 8.92~8.89 (m, 3H), 8.21 (s, 1H), 8.00~7.98 (d, 2H), 7.73 (s, 1H), 7.61~7.44 (m, 9H), 7.40~7.37 (m, 2H) |
| 4 | δ = 8.96~8.94 (d, 1H), 8.92~8.90 (d, 2H), 8.25~8.09 (m, 4H), 8.01~7.99 (d, 2H), 7.82 (s, 1H), 7.72 (s, 1H), 7.60~7.39 (m, 10H) |
| 5 | δ = 8.99~8.97 (d, 2H), 8.92~8.89 (m, 3H), 8.21~8.19 (d, 1H), 7.97~7.95 (d, 2H), 7.70 (s, 1H), 7.62~7.42 (m, 10H), 7.40~7.27 (m, 7H) |
| 6 | δ = 9.24 (s, 1H), 9.20 (s, 1H), 8.97~8.95 (d, 2H), 8.91~8.88 (m, 2H), 8.21 (s, 1H), 7.99~7.97 (d, 2H), 7.72 (s, 1H), 7.60~7.44 (m, 9H), 7.41~7.34 (m, 4H) |
| 9 | δ = 8.98~8.96 (d, 2H), 8.92~8.90 (m, 3H), 8.26~8.24 (d, 1H), 7.99~7.97 (d, 2H), 7.71 (s, 1H), 7.64 (s, 1H), 7.61~7.44 (m, 9H), 7.39~7.28 (m, 6H) |
| 10 | δ = 8.99~8.97 (d, 2H), 8.92~8.90 (m, 3H), 8.25~8.23 (d, 1H), 8.00~7.98 (d, 2H), 7.72 (s, 1H), 7.62~7.44 (m, 10H), 7.40~7.29 (m, 6H) |
| 12 | δ = 8.98~8.96 (d, 2H), 8.92~8.90 (m, 3H), 8.23~8.21 (d, 1H), 7.98~7.96 (d, 2H), 7.71 (s, 1H), 7.60~7.43 (m, 10H), 7.41~7.39 (m, 2H), 7.38~7.36 (m, 2H) |
| 14 | δ = 8.97~8.95 (d, 2H), 8.93~8.89 (m, 3H), 8.44 (s, 1H), 8.20~8.18 (d, 1H), 7.98~7.96 (d, 2H), 7.68 (s, 1H), 7.64~7.43 (m, 12H), 7.42~7.37 (m, 3H), 7.36~7.33 (m, 3H) |
| 17 | δ = 8.96~8.94 (d, 2H), 8.40 (s, 1H), 8.41~8.34 (m, 8H), 7.87 (s, 1H), 7.70~7.49 (m, 11H), 7.43~7.41 (t, 1H) |
| 23 | δ = 9.00~8.98 (d, 2H), 8.93~8.92(m, 3H), 8.87~8.85 (d, 2H), 8.15 (s, 1H), 8.11~8.09 (d, 1H), 8.09~8.04 (d, 2H), 7.95~7.93 (d, 1H), 7.86~7.84 (d, 2H), 7.82~7.80 (d, 1H), 7.75~7.73 (d, 2H), 7.67~7.58 (m, 4H), 7.58~7.40 (m, 6H) |
| 26 | δ = 8.91~8.87 (m, 4H), 8.8~-8.83 (d, 1H), 8.71~8.69 (d, 1H), 8.67~8.65 (d, 1H), 8.31~8.26 (m, 3H), 7.85 (s, 1H), 7.81~7.70 (m, 6H), 7.68~7.62 (m, 2H), 7.54~7.42 (m, 5H) |
| 36 | δ = 8.98-8.96 (d, 1H), 8.92-8.89 (d, 2H), 8.23 (s, 1H), 8.23~8.21 (d, 2H), 7.99-7.98 (d, 2H), 7.71 (s, 1H), 7.62-7.43 (m, 9H), 7.41-7.36 (m, 3H) |
| 38 | δ = 9.19 (s, 1H), 8.90-8.98 (d, 2H), 8.92-8.88 (m, 3H), 8.18 (s, 2H), 8.01-7.99 (d, 2H), 7.70 (s, 1H), 7.61-7.43 (m, 9H), 7.40-7.35 (m, 3H) |
| 41 | δ = 8.99-8.97 (d, 2H), 8.91-8.89 (m, 3H), 8.24-8.22 (d, 1H), 8.00-7.98 (d, 2H), 7.71 (s, 1H), 7.62-7.43 (m, 10H), 7.40-7.28 (m, 6H) |
| 51 | δ = 8.90-8.88 (d, 1H), 8.03~8.01 (d, 1H), 7.92-7.90 (d, 1H), 7.79-7.77 (d, 1H), 7.73-7.70 (d, 2H), 7.66-7.57 (m, 4H), 7.56-7.53 (m, 4H), 7.47-7.42 (m, 3H), 7.37-7.27 (m, 10H) |
| 67 | δ = 8.99 (s, 1H), 8.86-8.84 (d, 1H), 8.32-8.30 (d, 1H), 7.98-7.96 (d, 1H), 7.79-7.77 (d, 1H), 7.73-7.70 (d, 2H), 7.65-7.57 (m, 4H), 7.56-7.52 (m, 4H), 7.49-7.44 (m, 3H), 7.35-7.27 (m, 7H) |
| 68 | δ = 8.87-8.85 (d, 1H), 8.30-8.28 (d, 1H), 7.98-7.96 (d, 1H), 7.80-7.78 (d, 1H), 7.74-7.70 (d, 2H), 7.67-7.57 (m, 5H), 7.56-7.53 (m, 4H), 7.50-7.45 (m, 3H), 7.33-7.25 (m, 7H) |
| 70 | δ = 8.89-8.87 (d, 1H), 8.02-8.00 (d, 1H), 7.91-7.89 (d, 1H), 7.78-7.76 (d, 1H), 7.73-7.70 (d, 2H), 7.66-7.57 (m, 4H), 7.56~7.51 (m, 4H), 7.47-7.44 (m, 2H), 7.38-7.27 (m, 9H), 1.86 (s, 6H) |
| 72 | δ = 8.88-8.86 (d, 1H), 8.30-8.28 (d, 1H), 7.99-7.98 (d, 1H), 7.80-7.78 (d, 1H), 7.73~7.71 (d, 2H), 7.69-7.60 (m, 4H), 7.58-7.50 (m, 4H), 7.48-7.43 (m, 3H), 7.38-7.25 (m, 7H), 7. 21 (s, 1H) |
| 80 | δ = 8.90-8.88 (d, 1H), 8.02-8.00 (d, 1H), 7.91-7.89 (d, 1H), 7.75-7.73 (d, 1H), 7.71-7.69 (d, 2H), 7.68-7.59 (m, 5H), 7.54-7.45 (m, 5H), 7.47-7.42 (m, 3H), 7.38-7.25 (m, 12H) |
| 84 | δ = 8.92-8.90 (d, 1H), 8.01-7.99 (d, 1H), 7.90-7.80 (d, 1H), 7.78-7.76 (d, 1H), 7.75-7.73 (d, 2H), 7.69 (s, 1H), 7.69-7.59 (m, 5H), 7.53-7.46 (m, 4H), 7.44~7.41 (m, 2H), 7.38-7.24 (m, 13H) |
| 95 | δ = 8.90-8.88 (d, 1H), 8.05-8.04 (d, 1H), 8.04 (s, 1H), 7.93~7.91 (d, 1H), 7.79-7.77 (d, 1H), 7.72-7.70 (d, 2H), 7.65-7.56 (m, 3H), 7.56-7.52 (m, 4H), 7.46-7.42 (m, 3H), 7.37-7.28 (m, 9H), 7.13-7.09 (t, 1H) |
| 97 | δ = 8.89-8.87 (d, 1H), 8.04-8.03 (d, 1H), 8.02 (s, 1H), 7.92-7.90 (d, 1H), 7.79-7.76 (d, 1H), 7.71-7.68 (d, 2H), 7.67-7.58 (m, 3H), 7.57~7.51 (m, 5H), 7.47~7.41 (m, 4H), 7.38-7.26 (m, 10H), 7.14-7.09 (m, 2H) |
| 106 | δ = 8.87-8.85 (d, 1H), 8.29-8.27 (d, 1H), 7.99-7.96 (d, 1H), 7.81-7.79 (d, 1H), 7.74-7.70 (d, 2H), 7.65-7.57 (m, 6H), 7.56-7.48 (m, 5H), 7.46-7.38 (m, 5H), 7.35-7.28 (m, 7H) |
| 109 | δ = 8.88-8.86 (d, 1H), 8.27-8.25 (d, 1H), 8.00-7.98 (d, 1H), 7.80-7.78 (d, 1H), 7.71-7.69 (d, 2H), 7.63-7.52 (m, 7H), 7.50-7.43 (m, 6H), 7.41-7.34 (m, 6H), 7.31-7.23 (m, 8H) |
| 118 | δ = 8.91-8.89 (d, 1H), 8.05-8.03 (d, 1H), 8.01 (s, 1H), 7.90-7.88 (d, 1H), 7.80-7.78 (d, 1H), 7.70-7.68 (d, 2H), 7.67-7.62 (m, 3H), 7.58-7.49 (m, 6H), 7.45-7.39 (m, 5H), 7.37-7.25 (m, 11H), 7.15-7.10 (m, 3H) |
| 122 | δ = 8.93~8.91 (d, 1H), 8.04-8.02 (d, 1H), 8.00 (s, 1H), 7.91-7.89 (d, 1H), 7.81-7.79 (d, 1H), 7.71-7.68 (d, 2H), 7.68 (s, 1H), 7.68-7.62 (m, 3H), 7.58-7.48 (m, 5H), 7.46-7.38 (m, 5H), 7.36-7.25 (m, 10H), 7.16-7.10 (m, 4H) |
| 126 | δ = 8.89-8.87 (d, 1H), 8.05-8.03 (d, 1H), 8.02 (s, 1H), 7.93~7.91 (d, 1H), 7.80-7.78 (d, 1H), 7.72-7.70 (d, 2H), 7.65-7.59 (m, 3H), 7.55-7.48 (m, 4H), 7.46~7.41 (m, 4H), 7.39-7.26 (m, 11H), 7.14-7.10 (m, 2H) |
| 140 | δ = 8.92-8.90 (d, 1H), 8.03~8.01 (d, 1H), 8.03(s, 1H), 7.90-7.88 (d, 1H), 7.79-7.77 (d, 1H), 7.71-7.69 (d, 2H), 7.67 (s, 1H), 7.65-7.60 (m, 3H), 7.58-7.50 (m, 4H), 7.47-7.39 (m, 4H), 7.36-7.28 (m, 9H), 7.15-7.09 (m, 3H) |
| 141 | δ = 8.91-8.89 (d, 1H), 8.04-8.02 (d, 1H), 7.98 (s, 1H), 7.89-7.87 (d, 1H), 7.79-7.77 (d, 1H), 7.71-7.69 (d, 2H), 7.65-7.60 (m, 3H), 7.57-7.49 (m, 6H), 7.45-7.36 (m, 5H), 7.39-7.25 (m, 9H), 7.14-7.12 (t, 1H) |
| 143 | δ = 8.94-8.92 (d, 1H), 8.06-8.04 (d, 1H), 8.01 (s, 1H), 7.94-7.92 (d, 1H), 7.78-7.76 (d, 1H), 7.74-7.72 (d, 2H), 7.66-7.58 (m, 4H), 7.58-7.49 (m, 5H), 7.45-7.40 (m, 3H), 7.38-7.29 (m, 9H), 7.12-7.10 (t, 1H) |
| 147 | δ = 8.88-8.86 (d, 1H), 8.30-8.28 (d, 1H), 7.97-7.95 (d, 2H), 7.81-7.79 (d, 1H), 7.74-7.72 (d, 2H), 7.68-7.58 (m, 6H), 7.56~7.51 (m, 5H), 7.49-7.40 (m, 5H), 7.35-7.26 (m, 10H) |
| 148 | δ = 9.04 (s, 1H), 8.93~8.91 (d, 1H), 8.63~8.61 (d, 1H), 8.35-8.33 (d, 1H), 8.31-8.29 (d, 1H), 8.19-8.17 (d, 1H), 7.92-7.90 (d, 1H), 7.80-7.77 (d, 2H), 7.74-7.72 (d, 2H), 7.69~7.61 (m, 4H), 7.56-7.49 (m, 4H), 7.47-7.39 (m, 4H), 7.34-7.26 (m, 10H) |
| 149 | δ = 8.89-8.87 (d, 1H), 8.30-8.28 (d, 1H), 8.16-8.14 (d, 1H), 8.12-8.10 (d, 1H), 8.00-7.98 (d, 2H), 7.81-7.79 (d, 1H), 7.73~7.71 (d, 2H), 7.69~7.61 (m, 4H), 7.57-7.50 (m, 6H), 7.48~7.41 (m, 4H), 7.38-7.26 (m, 7H), 7. 19 (s, 1H) |
| 151 | δ = 8.91-8.89 (d, 1H), 8.03~8.01 (d, 1H), 7.90-7.88 (d, 1H), 7.80-7.78 (d, 1H), 7.71-7.69 (d, 2H), 7.65-7.58 (m, 4H), 7.57-7.53 (m, 4H), 7.48-7.43 (m, 3H), 7.36-7.27 (m, 10H) |
| 152 | δ = 8.90-8.88 (d, 1H), 8.05-8.03 (d, 1H), 7.91-7.89 (d, 1H), 7.79-7.77 (d, 1H), 7.72-7.70 (d, 2H), 7.68-7.52 (m, 3H), 7.56-7.52 (m, 4H), 7.49-7.43 (m, 3H), 7.38-7.26 (m, 11H) |
| 165 | δ = 8.88-8.86 (d, 1H), 8.02-8.00 (d, 1H), 7.88-7.86 (d, 1H), 7.75-7.73 (d, 1H), 7.70-7.67 (d, 2H), 7.67-7.58 (m, 5H), 7.53-7.44 (m, 5H), 7.48-7.42 (m, 3H), 7.40-7.27 (m, 12H) |
| 172 | δ = 8.90-8.88 (d, 1H), 8.05-8.04 (d, 1H), 8.04 (s, 1H), 7.93~7.91 (d, 1H), 7.79-7.77 (d, 1H), 7.72-7.70 (d, 2H), 7.65-7.56 (m, 3H), 7.56-7.52 (m, 4H), 7.46-7.42 (m, 3H), 7.37-7.28 (m, 9H), 7.13-7.09 (t, 1H) |
| 173 | δ = 8.89-8.87 (d, 1H), 8.05-8.03 (d, 1H), 8.02 (s, 1H), 7.91-7.89 (d, 1H), 7.80-7.78 (d, 1H), 7.71-7.69 (d, 2H), 7.66-7.58 (m, 3H), 7.57-7.52 (m, 5H), 7.48-7.42 (m, 4H), 7.37-7.26 (m, 10H), 7.14-7.10 (m, 2H) |
| 201 | δ = 8.91-8.89 (d, 1H), 8.05-8.03 (d, 1H), 7.99 (s, 1H), 7.94-7.92 (d, 1H), 7.79-7.77 (d, 1H), 7.72-7.70 (d, 2H), 7.64-7.60 (m, 2H), 7.58-7.52 (m, 3H), 7.46~7.41 (m, 3H), 7.38-7.27 (m, 7H) |
| 202 | δ = 8.95-8.93 (d, 1H), 8.90-8.87 (m, 2H), 7.78 (s, 1H), 7.59-7.54 (m, 3H), 7.45-7.47 (t, 1H), 7.39-7.37 (t, 1H) |
| 208 | δ = 8.88-8.86 (d, 1H), 8.05-8.03 (d, 1H), 8.00 (s, 1H), 7.93~7.91 (d, 1H), 7.78-7.76 (d, 1H), 7.73~7.71 (d, 2H), 7.65-7.59 (m, 3H), 7.59-7.52 (m, 4H), 7.47~7.41 (m, 3H), 7.39-7.26 (m, 8H), 7.13-7.09 (m, 2H), 1.72 (s, 12H) |
| 236 | δ = 8.99-8.97 (d, 2H), 8.93-8.89 (m, 3H), 8.85-8.83 (d, 2H), 8.17 (s, 1H), 8.13~8.11 (d, 1H), 8.08-8.05 (d, 2H), 7.97-7.95 (d, 1H), 7.85-7.83 (d, 2H), 7.80-7.78 (d, 1H), 7.74-7.72 (d, 2H), 7.62-7.53 (m, 5H), 7.50-7.38 (m, 7H), 1.77 (s, 6H) |
| 249 | δ = 8.96-8.94 (d, 2H), 8.91-8.87 (m, 6H), 8.43 (s, 2H), 8.20-8.18 (d, 2H), 7.96-7.94 (d, 2H), 7.62-7.48 (m, 8H), 7.42-7.38 (m, 3H), 7.34~7.31 (m, 2H) |
| 256 | δ = 8.91-8.89 (d, 1H), 8.10-8.08 (d, 1H), 7.90-7.88 (d, 1H), 7.78-7.76 (d, 1H), 7.71-7.69 (d, 2H), 7.65-7.57 (m, 4H), 7.56-7.53 (m, 3H), 7.46-7.42 (m, 3H), 7.39-7.27 (m, 9H) |
| 258 | δ = 8.90-8.88 (d, 1H), 8.04-8.02 (d, 1H), 7.91-7.89 (d, 1H), 7.79-7.77 (d, 1H), 7.73~7.71 (d, 2H), 7.65-7.56 (m, 4H), 7.57-7.52 (m, 4H), 7.46~7.41 (m, 3H), 7.37-7.27 (m, 10H), 1.78 (s, 12H) |
| 276 | δ = 8.88-8.86 (d, 1H), 8.03~8.01 (d, 1H), 7.88-7.86 (d, 1H), 7.77-7.75 (d, 1H), 7.72-7.70 (d, 2H), 7.66-7.64 (d, 2H), 7.59-7.52 (m, 4H), 7.45-7.40 (m, 3H), 7.35-7.28 (m, 8H), 1.76 (s, 6H) |
| 310 | δ = 8.97-8.95 (d, 2H), 8.91-8.87 (m, 2H), 8.20-8.18 (d, 1H), 7.90-7.88 (d, 2H), 7.70 (s, 1H), 7.65-7.56 (m, 10H), 7.45-7.28 (m, 7H) |
| 351 | δ = 9.10-9.08 (d, 1H), 8.95-8.93 (d, 1H), 8.80-8.78 (d, 1H), 8.65-8.63 (d, 1H), 8.51-8.49 (d, 1H), 8.22-8.15 (m, 6H), 7.90-7.88 (d, 1H), 7.75-7.70 (m, 3H), 7.64-7.56 (m, 4H), 7.57-7.53 (m, 3H), 7.46~7.41 (m, 3H), 7.37-7.29 (m, 7H) |
| 384 | δ = 8.89-8.87 (d, 1H), 8.55-8.47 (m, 4H), 8.33-8.29 (m, 3H), 7.71 (s, 1H), 7.56-7.53 (m, 3H), 7.48-7.42 (m, 6H), 7.36-7.28 (m, 7H) |
| 386 | δ = 9.09-9.07 (d, 1H), 8.96-8.94 (d, 1H), 8.79-8.77 (d, 1H), 8.63~8.61 (d, 1H), 8.50-8.48 (d, 1H), 8.23-8.15 (m, 6H), 7.91-7.89 (d, 1H), 7.74-7.69 (m, 3H), 7.63-7.56 (m, 4H), 7.58-7.53 (m, 3H), 7.46-7.42 (m, 3H), 7.37-7.28 (m, 7H) |
| 426 | δ = 8.86-8.84 (d, 1H), 8.75-8.70 (d, 2H), 8.55-8.50 (d, 1H), 7.87-7.83 (m, 3H), 7.69-7.56 (m, 8H), 7.52-7.48 (t, 1H), 7.41-7.39 (d, 1H), 7.31-7.26 (m, 3H), 7.21-7.19 (d, 1H) |
| 433 | δ = 9.43 (s, 1H), 8.93-8.89 (d, 2H), 8.87-8.85 (d, 1H), 8.73~8.71 (d, 1H), 8.51-8.49 (d, 1H), 8.16-8.05 (m, 3H), 7.95-7.93 (d, 1H), 7.87-7.85 (d, 2H), 7.79~7.51 (m, 6H), 7.41-7.39 (m, 3H), 7.22-7.18 (d, 2H) |
| 437 | δ = 8.85-8.83 (d, 1H), 8.77-8.75 (d, 2H), 8.52-8.50 (d, 1H), 7.89-7.82 (m, 4H), 7.68-7.54 (m, 9H), 7.52-7.49 (t, 1H), 7.43~7.41 (d, 2H), 7.35-7.27 (m, 4H), 7.25-7.23 (d, 1H) |
| 441 | δ = 8.82-8.80 (d, 1H), 8.79 (s, 1H), 8.76-8.74 (d, 1H), 8.51-8.49 (d, 1H), 7.91-7.85 (m, 4H), 7.65-7.59 (m, 9H), 7.53-7.49 (t, 1H), 7.44-7.42 (d, 2H), 7.36-7.29 (m, 3H), 7.20-7.18 (d, 1H) |
| 448 | δ = 9.41 (s, 1H), 9.23~9.21 (d, 1H), 8.98-8.96 (d, 1H), 8.94-8.92 (d, 3H), 8.64-8.62 (d, 1H), 8.17-8.15 (d, 1H), 8.15 (s, 1H), 8.07-8.05 (d, 1H), 7.98-7.94 (d, 2H), 7.91-7.89 (d, 3H), 7.75-7.42 (m, 12H) |
| 450 | δ = 9.36-9.34 (d, 1H), 9.21-9.19 (d, 1H), 8.95-8.93 (d, 1H), 8.90-8.89 (d, 3H), 8.61-8.59 (d, 1H), 8.24-8.22 (d, 1H), 8.16-8.14 (d, 1H), 8.08-8.06 (d, 1H), 7.95~7.91 (d, 2H), 7.88-7.82 (d, 3H), 7.72~7.41 (m, 12H) |

**[Table 7]**

| Compound | FD-Mass | Compound | FD-Mass |
|---|---|---|---|
| 1 | m/z= 505.1305 (C33H19N3OS, 505.1249) | 2 | m/z= 555.1426 (C37H21N3OS, 555.1405) |
| 3 | m/z= 555.1438 (C37H21N3OS, 555.1405) | 4 | m/z= 595.1342 (C39H21N3O2S, 595.1354) |
| 5 | m/z= 670.1798C45H26N4OS, 670.1827) | 6 | m/z= 605.1549(C41H23N3OS, 605.1562) |
| 7 | m/z= 605.1542 (C41H23N3OS, 605.1562) | 8 | m/z= 605.1573 (C41H23N3OS, 605.1562) |
| 9 | m/z= 631.1726(C43H25N3OS, 631.1718) | 10 | m/z= 631.1724 (C43H25N3OS, 631.1718) |
| 11 | m/z= 671.1682 (C45H25N3O2S, 671.1667) | 12 | m/z= 581.1537 (C39H23N3OS, 581.1562) |
| 13 | m/z= 611.1184 (C39H21N3OS2, 611.1126) | 14 | m/z= 656.1934 (C46H28N2OS, 656.1922) |
| 15 | m/z= 656.1951 (C46H28N2OS, 656.1922) | 16 | m/z= 657.1888 (C45H27N3OS, 657.1875) |
| 17 | m/z= 580.1608 (C40H24N2OS, 580.1609) | 18 | m/z= 631.1722 (C43H25N3OS, 631.1718) |
| 19 | m/z= 631.1751 (C43H25N3OS, 631.1718) | 20 | m/z= 671.1692 (C45H25N3O2S, 671.1667) |
| 21 | m/z= 670.1834 (C45H26N4OS, 670.1827) | 22 | m/z= 746.2173 (C51H30N4OS, 746.2140) |
| 23 | m/z= 657.1882 (C45H27N3OS, 657.1875) | 24 | m/z= 680.1934 (C48H28N2OS, 680.1922) |
| 25 | m/z= 707.2034 (C49H29N3OS, 707.2031) | 26 | m/z= 604.1691 (C42H24N2OS, 604.1609) |
| 27 | m/z= 603.1627 (C43H25NOS, 603.1657) | 28 | m/z= 733.2192 (C51H31N3OS, 733.2188) |
| 29 | m/z= 657.1883 (C45H27N3OS, 657.1875) | 30 | m/z= 542.1491 (C37H22N2OS, 542.1453) |
| 31 | m/z= 720.1999(C49H28N4OS, 720.1984) | 32 | m/z= 671.1683 (C45H25N3O2S, 671.1667) |
| 33 | m/z= 671.1681 (C45H25N3O2S, 671.1667) | 34 | m/z= 733.2202 (C51H31N3OS, 733.2188) |
| 35 | m/z= 721.1831 (C49H27N3O2S, 721.1824) | 36 | m/z= 595.1372 (C39H21N3O2S, 595.1354) |
| 37 | m/z= 670.1839(C45H26N4OS, 670.1827) | 38 | m/z= 605.1585 (C41H23N3OS, 605.1562) |
| 39 | m/z= 555.1436(C37H21N3OS, 555.1405) | 40 | m/z= 631.1731 (C43H25N3OS, 631.1718) |
| 41 | m/z= 631.1732 (C43H25N3OS, 631.1718) | 42 | m/z= 671.1676(C45H25N3O2S, 671.1667) |
| 43 | m/z= 611.1137 (C39H21N3OS2, 611.1126) | 44 | m/z= 580.1621 (C40H24N2OS, 580.1609) |
| 45 | m/z= 656.1938 (C46H28N2OS, 656.1922) | 46 | m/z= 631.1729(C43H25N3OS, 631.1718) |
| 47 | m/z= 671.1681 (C45H25N3O2S, 671.1667) | 48 | m/z= 670.1833 (C45H26N4OS, 670.1827) |
| 49 | m/z= 733.2197 (C51H31N3OS, 733.2188) | 50 | m/z= 783.23418 (C55H33N3OS, 783.2344) |
| 51 | m/z= 593.1863 (C42H27NOS, 593.1813) | 52 | m/z= 593.1862 (C42H27NOS, 593.1813) |
| 53 | m/z= 593.1827 (C42H27NOS, 593.1813) | 54 | m/z= 491.1342 (C34H21NOS, 491.1344) |
| 55 | m/z= 491.1341 (C34H21NOS, 491.1344) | 56 | m/z= 557.1819(C39H27NOS, 557.1813) |
| 57 | m/z= 597.1261 (C40H23NOS2, 597.1221) | 58 | m/z= 597.1264 (C40H23NOS2, 597.1221) |
| 59 | m/z= 623.1391 (C42H25NOS2, 623.1378) | 60 | m/z= 623.1393 (C42H25NOS2, 623.1378) |
| 61 | m/z= 673.1544 (C46H27NOS2, 673.1534) | 62 | m/z= 581.1454 (C40H23NO2S, 581.1449) |
| 63 | m/z= 607.1612 (C42H25NO2S, 607.1606) | 64 | m/z= 581.1453 (C40H23NO2S, 581.1449) |
| 65 | m/z= 607.1631 (C42H25NO2S, 607.1606) | 66 | m/z= 657.1792 (C46H27NO2S, 657.1762) |
| 67 | m/z= 567.1639 (C40H25NOS, 567.1657) | 68 | m/z= 567.1649 (C40H25NOS, 567.1657) |
| 69 | m/z= 617.1831 (C44H27NOS, 617.1813) | 70 | m/z= 633.2132 (C45H31NOS, 633.2126) |
| 71 | m/z= 567.1691 (C40H25NOS, 567.1657) | 72 | m/z= 567.1648 (C40H25NOS, 567.1657) |
| 73 | m/z= 567.1634 (C40H25NOS, 567.1657) | 74 | m/z= 567.1681 (C40H25NOS, 567.1657) |
| 75 | m/z= 593.1824 (C42H27NOS, 593.1813) | 76 | m/z= 593.1834 (C42H27NOS, 593.1813) |
| 77 | m/z= 593.1811 (C42H27NOS, 593.1813) | 78 | m/z= 593.1838 (C42H27NOS, 593.1813) |
| 79 | m/z= 593.1823 (C42H27NOS, 593.1813) | 80 | m/z= 669.2131 (C48H31NOS, 669.2126) |
| 81 | m/z= 669.2139(C48H31NOS, 669.2126) | 82 | m/z= 669.2138 (C48H31NOS, 669.2126) |
| 83 | m/z= 669.2147 (C48H31NOS, 669.2126) | 84 | m/z= 669.2149(C48H31NOS, 669.2126) |
| 85 | m/z= 669.2119(C48H31NOS, 669.2126) | 86 | m/z= 669.2125 (C48H31NOS, 669.2126) |
| 87 | m/z= 669.2122 (C48H31NOS, 669.2126) | 88 | m/z= 669.2151 (C48H31NOS, 669.2126) |
| 89 | m/z= 669.2162 (C48H31NOS, 669.2126) | 90 | m/z= 669.2126(C48H31NOS, 669.2126) |
| 91 | m/z= 669.2136(C48H31NOS, 669.2126) | 92 | m/z= 669.2191 (C48H31NOS, 669.2126) |
| 93 | m/z= 669.2165 (C48H31NOS, 669.2126) | 94 | m/z= 680.1898 (C48H28N2OS, 680.1922) |
| 95 | m/z= 593.1831 (C42H27NOS, 593.1813) | 96 | m/z= 593.1797 (C42H27NOS, 593.1813) |
| 97 | m/z= 669.2135 (C48H31NOS, 669.2126) | 98 | m/z= 669.2134 (C48H31NOS, 669.2126) |
| 99 | m/z= 567.1669(C40H25NOS, 567.1657) | 100 | m/z= 633.2137 (C45H31NOS, 633.2126) |
| 101 | m/z= 699.1681 (C48H29NOS2, 699.1691) | 102 | m/z= 699.1692 (C48H29NOS2, 699.1691) |
| 103 | m/z= 749.1849(C52H31NOS2, 749.1847) | 104 | m/z= 683.1928 (C48H29NO2S, 683.1919) |
| 105 | m/z= 643.1983 (C46H29NOS, 643.1970) | 106 | m/z= 643.1969(C46H29NOS, 643.1970) |
| 107 | m/z= 693.2137 (C50H31NOS, 693.2126) | 108 | m/z= 719.2294 (C52H33NOS, 719.2283) |
| 109 | m/z= 719.2297 (C52H33NOS, 719.2283) | 110 | m/z= 709.2451 (C51H35NOS, 709.2439) |
| 111 | m/z= 643.1983 (C46H29NOS, 643.1970) | 112 | m/z= 643.1998 (C46H29NOS, 643.1970) |
| 113 | m/z= 699.2134 (C48H31NOS, 669.2126) | 114 | m/z= 669.2145 (C48H31NOS, 669.2126) |
| 115 | m/z= 669.2138 (C48H31NOS, 669.2126) | 116 | m/z= 669.2141 (C48H31NOS, 669.2126) |
| 117 | m/z= 669.2137 (C48H31NOS, 669.2126) | 118 | m/z= 745.2470 (C54H35NOS, 745.2439) |
| 119 | m/z= 745.2440 (C54H35NOS, 745.2439) | 120 | m/z= 745.2452 (C54H35NOS, 745.2439) |
| 121 | m/z= 745.2433 (C54H35NOS, 745.2439) | 122 | m/z= 745.2477 (C54H35NOS, 745.2439) |
| 123 | m/z= 745.2431 (C54H35NOS, 745.2439) | 124 | m/z= 517.1486(C36H23NOS, 517.1500) |
| 125 | m/z= 593.1837 (C42H27NOS, 593.1813) | 126 | m/z= 669.2124 (C48H31NOS, 669.2126) |
| 127 | m/z= 567.1662 (C40H25NOS, 567.1657) | 128 | m/z= 567.1664 (C40H25NOS, 567.1657) |
| 129 | m/z= 633.2131 (C45H31NOS, 633.2126) | 130 | m/z= 699.1693 (C48H29NOS2, 699.1691) |
| 131 | m/z=699.1697(C48H29NOS2, 699.1691) | 132 | m/z=749.1849(C52H31NOS2, 749.1847) |
| 133 | m/z=683.1922 (C48H29NO2S, 683.1919) | 134 | m/z=683.1927(C48H29NO2S, 683.1919) |
| 135 | m/z=643.1939(C46H29NOS, 643.1970) | 136 | m/z=643.1983(C46H29NOS, 643.1970) |
| 137 | m/z=693.2127(C50H31NOS, 693.2126) | 138 | m/z=719.2293(C52H33NOS, 719.2283) |
| 139 | m/z=709.2468 (C51H35NOS, 709.2439) | 140 | m/z=669.2129(C48H31NOS, 669.2126) |
| 141 | m/z=669.2138 (C48H31NOS, 669.2126) | 142 | m/z=669.2142 (C48H31NOS, 669.2126) |
| 143 | m/z=643.1993(C46H29NOS, 643.1970) | 144 | m/z=657.1774 (C46H27NO2S, 657.1762) |
| 145 | m/z=733.2074 (C52H31NO2S, 733.2075) | 146 | m/z=633.2117(C45H31NOS, 633.2126) |
| 147 | m/z=719.2276(C52H33NOS, 719.2283) | 148 | m/z=719.2251(C52H33NOS, 719.2283) |
| 149 | m/z=693.2135 (C50H31NOS, 693.2126) | 150 | m/z=669.2150(C48H31NOS, 669.2126) |
| 151 | m/z=669.2120(C48H31NOS, 669.2126) | 152 | m/z=669.2131(C48H31NOS, 669.2126) |
| 153 | m/z=491.1346(C34H21NOS, 491.1344) | 154 | m/z=557.1861(C39H27NOS, 557.1813) |
| 155 | m/z=623.1381(C42H25NOS2, 623.1378) | 156 | m/z=673.1539(C46H27NOS2, 673.1534) |
| 157 | m/z=657.1728 (C46H27NO2S, 657.1762) | 158 | m/z=567.1662 (C40H25NOS, 567.1657) |
| 159 | m/z=581.1458 (C40H23NO2S, 581.1449) | 160 | m/z=617.1829(C44H27NOS, 617.1813) |
| 161 | m/z=567.1669(C40H25NOS, 567.1657) | 162 | m/z=593.1810(C42H27NOS, 593.1813) |
| 163 | m/z=593.1819(C42H27NOS, 593.1813) | 164 | m/z=593.1834 (C42H27NOS, 593.1813) |
| 165 | m/z=669.2161(C48H31NOS, 669.2126) | 166 | m/z=669.2130(C48H31NOS, 669.2126) |
| 167 | m/z=669.2123(C48H31NOS, 669.2126) | 168 | m/z=669.2120(C48H31NOS, 669.2126) |
| 169 | m/z=669.2143(C48H31NOS, 669.2126) | 170 | m/z=669.2141(C48H31NOS, 669.2126) |
| 171 | m/z=680.1938 (C48H28N2OS, 680.1922) | 172 | m/z=593.1811(C42H27NOS, 593.1813) |
| 173 | m/z=669.2137(C48H31NOS, 669.2126) | 174 | m/z=633.2134 (C45H31NOS, 633.2126) |
| 175 | m/z=699.1677(C48H29NOS2, 699.1691) | 176 | m/z=643.1989(C46H29NOS, 643.1970) |
| 177 | m/z=719.2291(C52H33NOS, 719.2283) | 178 | m/z=719.2297(C52H33NOS, 719.2283) |
| 179 | m/z=709.2464 (C51H35NOS, 709.2439) | 180 | m/z=643.1955 (C46H29NOS, 643.1970) |
| 181 | m/z=669.2136(C48H31NOS, 669.2126) | 182 | m/z=745.2444 (C54H35NOS, 745.2439) |
| 183 | m/z=593.1822 (C42H27NOS, 593.1813) | 184 | m/z=567.1660(C40H25NOS, 567.1657) |
| 185 | m/z=699.1687(C48H29NOS2, 699.1691) | 186 | m/z=719.2293(C52H33NOS, 719.2283) |
| 187 | m/z=669.2131(C48H31NOS, 669.2126) | 188 | m/z=709.2447(C51H35NOS, 709.2439) |
| 189 | m/z=669.2167(C48H31NOS, 669.2126) | 190 | m/z=669.2141(C48H31NOS, 669.2126) |
| 191 | m/z=643.1983(C46H29NOS, 643.1970) | 192 | m/z=657.1760(C46H27NO2S, 657.1762) |
| 193 | m/z=733.2079(C52H31NO2S, 733.2075) | 194 | m/z=733.2070(C52H31NO2S, 733.2075) |
| 195 | m/z=749.1851(C52H31NOS2, 749.1847) | 196 | m/z= 633.2137 (C45H31NOS, 633.2126) |
| 197 | m/z=719.2292 (C52H33NOS, 719.2283) | 198 | m/z=719.2280(C52H33NOS, 719.2283) |
| 199 | m/z=693.2109(C50H31NOS, 693.2126) | 200 | m/z=669.2134 (C48H31NOS, 669.2126) |
| 201 | m/z=598.2098 (C42H22D5NOS, 598.2127 | 202 | m/z=515.1892 (C33H9D10N3OS, 515.1877) |
| 203 | m/z=594.1443(C40H22N2O2S, 594.1402) | 204 | m/z=634.1131(C42H22N2OS2, 634.1174) |
| 205 | m/z=657.1547(C44H23N3O2S, 657.1511) | 206 | m/z=687.1493(C45H25N3OS2, 687.1439) |
| 207 | m/z=833.2762 (C61H39NOS, 833.2752) | 208 | m/z=749.2802(C54H39NOS, 749.2752) |
| 209 | m/z=749.2749(C54H39NOS, 749.2752) | 210 | m/z= 873.3082 (C64H43NOS, 873.3065) |
| 211 | m/z=657.1892 (C45H27N3OS, 657.1875) | 212 | m/z=705.2462 (C51H31NOS, 705.2126) |
| 213 | m/z=771.2621(C56H37NOS, 771.2596) | 214 | m/z=687.1444 (C45H25N3OS2, 687.1439) |
| 215 | m/z=697.2192 (C48H31N3OS, 697.2188) | 216 | m/z=821.2487(C58H35N3OS, 821.2501) |
| 217 | m/z=757.2193(C53H31N3OS, 757.2188) | 218 | m/z=681.1892 (C47H27N3OS, 681.1875) |
| 219 | m/z=617.1822 (C44H27NOS, 617.1813) | 220 | m/z=717.2132 (C52H31NOS, 717.2126) |
| 221 | m/z=681.1882 (C47H27N3OS, 681.1875) | 222 | m/z=681.1889(C47H27N3OS, 681.1875) |
| 223 | m/z=731.2064 (C51H29N3OS, 731.2031) | 224 | m/z=655.1724 (C45H25N3OS, 655.1718) |
| 225 | m/z=731.2035 (C51H29N3OS, 731.2031) | 226 | m/z=731.2036(C51H29N3OS, 731.2031) |
| 227 | m/z=731.2055 (C51H29N3OS, 731.2031) | 228 | m/z=681.1892C47H27N3OS, 681.1875 |
| 229 | m/z=807.2366(C57H33N3OS, 807.2344) | 230 | m/z=933.2852 (C67H39N3OS, 933.2814) |
| 231 | m/z=721.2193(C50H31N3OS, 721.2188) | 232 | m/z=910.2772 (C64H38N4OS, 910.2766) |
| 233 | m/z=681.1883(C47H27N3OS, 681.1875) | 234 | m/z=629.1567(C43H23N3OS, 629.1562) |
| 235 | m/z=705.1886(C49H27N3OS, 705.1875) | 236 | m/z=671.2021(C46H29N3OS, 671.2031) |
| 237 | m/z=746.2028 (C52H29N3OS, 743.2031) | 238 | m/z=819.2346(C58H33N3OS, 819.2344) |
| 239 | m/z=759.1992 (C52H29N3O2S, 759.1980) | 240 | m/z=759.1978 (C52H29N3O2S, 759.1980) |
| 241 | m/z=635.1324 (C41H21N3O3S, 635.1304) | 242 | m/z=6223.1321 (C40H21N3O3S, 623.1304) |
| 243 | m/z=749.1788 (C50H27N3O3S, 749.1773) | 244 | m/z=711.1629(C47H25N3O3S, 711.1617) |
| 245 | m/z=683.1702(C46H25N3O2S, 683.1667) | 246 | m/z=685.2192 (C47H31N3OS, 685.2188) |
| 247 | m/z=746.2149(C51H30N4OS, 746.2140) | 248 | m/z=746.2160(C51H30N4OS, 746.2140) |
| 249 | m/z=777.1549(C51H27N3O2S2, 777.1545) | 250 | m/z=777.1553(C51H27N3O2S2, 777.1545) |
| 251 | m/z=777.1553(C51H27N3O2S2, 777.1545) | 252 | m/z=853.1860(C57H31N3O2S2, 853.1858) |
| 253 | m/z=777.1562 (C51H27N3O2S2, 777.1545) | 254 | m/z=827.1689(C55H29N3O2S2, 827.1701) |
| 255 | m/z=827.1685 (C55H29N3O2S2, 827.1701) | 256 | m/z=591.1681(C42H25NOS, 591.1657) |
| 257 | m/z=633.2133(C45H31NOS, 633.2126) | 258 | m/z=673.2443(C48H35NOS, 673.2439) |
| 259 | m/z=757.2448 (C55H35NOS, 757.2439) | 260 | m/z=795.2561(C58H37NOS, 795.2596) |
| 261 | m/z=617.1835 (C44H27NOS, 617.1813) | 262 | m/z=617.1835 (C44H27NOS, 617.1813) |
| 263 | m/z=667.1998 (C48H29NOS, 667.1970) | 264 | m/z=591.1682 (C42H25NOS, 591.1657) |
| 265 | m/z=667.1992 (C48H29NOS, 667.1970) | 266 | m/z=667.1971(C48H29NOS, 667.1970) |
| 267 | m/z=667.1957(C48H29NOS, 667.1970) | 268 | m/z=617.1835 (C44H27NOS, 617.1813) |
| 269 | m/z=743.2292 (C54H33NOS, 743.2283) | 270 | m/z=869.2753(C64H39NOS, 869.2752) |
| 271 | m/z=657.2161(C47H31NOS, 657.2126) | 272 | m/z=846.2692 (C61H38N2OS, 846.2705) |
| 273 | m/z=617.1824 (C44H27NOS, 617.1813) | 274 | m/z=565.1533(C40H23NOS, 565.1500) |
| 275 | m/z=641.1828 (C46H27NOS, 641.1813) | 276 | m/z=607.1982 (C43H29NOS, 607.1970) |
| 277 | m/z=679.1999(C49H29NOS, 679.1970) | 278 | m/z=755.2292 (C55H33NOS, 755.2283) |
| 279 | m/z=695.1925 (C49H29NO2S, 695.1919) | 280 | m/z=695.1987(C49H29NO2S, 695.1919) |
| 281 | m/z=571.1254 (C38H21NO3S, 571.1242) | 282 | m/z=559.1243(C37H21NO3S, 559.1242) |
| 283 | m/z=685.1700(C47H27NO3S, 685.1712) | 284 | m/z=647.1569(C44H25NO3S, 647.1555) |
| 285 | m/z=619.1624 (C43H25NO2S, 619.1606) | 286 | m/z=621.2133(C44H31NOS, 621.2126) |
| 287 | m/z=682.2077(C48H30N2OS, 682.2079) | 288 | m/z=682.2081(C48H30N2OS, 682.2079) |
| 289 | m/z=713.1472 (C48H27NO2S2, 713.1483) | 290 | m/z=713.1480(C48H27NO2S2, 713.1483) |
| 291 | m/z=713.1470(C48H27NO2S2, 713.1483) | 292 | m/z=789.1815 (C54H31NO2S2, 789.1796) |
| 293 | m/z=713.1491(C48H27NO2S2, 713.1483) | 294 | m/z=763.1665 (C52H29NO2S2, 763.1640) |
| 295 | m/z=763.1685 (C52H29NO2S2, 763.1640) | 296 | m/z=835.2415 (C57H33N5OS, 835.2406) |
| 297 | m/z=835.2421(C57H33N5OS, 835.2406) | 298 | m/z=835.2457(C57H33N5OS, 835.2406) |
| 299 | m/z=835.2416(C57H33N5OS, 835.2406) | 300 | m/z=835.2401(C57H33N5OS, 835.2406) |
| 301 | m/z=786.2509(C54H34N4OS, 786.2453) | 302 | m/z=786.2482 (C54H34N4OS, 786.2453) |
| 303 | m/z=786.2481(C54H34N4OS, 786.2453) | 304 | m/z=786.2455 (C54H34N4OS, 786.2453) |
| 305 | m/z=786.2461(C54H34N4OS, 786.2453) | 306 | m/z=911.2708(C63H37N5OS, 911.2719) |
| 307 | m/z=910.2782 (C64H38N4OS, 910.2766) | 308 | m/z=832.2301(C58H32N4OS, 832.2297) |
| 309 | m/z=720.1986(C49H28N4OS, 720.1984) | 310 | m/z=683.1774 (C45H25N5OS, 683.1780) |
| 311 | m/z=783.2086(C53H29N5OS, 783.2093) | 312 | m/z=809.2281(C55H31N5OS, 809.2249) |
| 313 | m/z=645.1532 (C43H23N3O2S, 645.1511) | 314 | m/z=645.1532 (C43H23N3O2S, 645.1511) |
| 315 | m/z=645.1582 (C43H23N3O2S, 645.1511) | 316 | m/z=661.1284 (C43H23N3OS2, 661.1283) |
| 317 | m/z=661.1284 (C43H23N3OS2, 661.1283) | 318 | m/z=661.1275 (C43H23N3OS2, 661.1283) |
| 319 | m/z=721.1815 (C49H27N3O2S, 721.1824) | 320 | m/z=695.1660(C47H25N3O2S, 695.1667) |
| 321 | m/z=721.1817(C49H27N3O2S, 721.1824) | 322 | m/z=721.1825 (C49H27N3O2S, 721.1824) |
| 323 | m/z=721.1825 (C49H27N3O2S, 721.1824) | 324 | m/z=673.1557(C46H27NOS2, 673.1534) |
| 325 | m/z=673.1562 (C46H27NOS2, 673.1534) | 326 | m/z=673.1562 (C46H27NOS2, 673.1534) |
| 327 | m/z=721.1845 (C49H27N3O2S, 721.1824) | 328 | m/z=721.1843(C49H27N3O2S, 721.1824) |
| 329 | m/z=673.1538 (C46H27NOS2, 673.1534) | 330 | m/z=673.1524 (C46H27NOS2, 673.1534) |
| 331 | m/z=737.1621(C49H27N3OS2, 737.1596) | 332 | m/z=737.1578 (C49H27N3OS2, 737.1596) |
| 333 | m/z=737.1640(C49H27N3OS2, 737.1596) | 334 | m/z=737.1594 (C49H27N3OS2, 737.1596) |
| 335 | m/z=737.1587(C49H27N3OS2, 737.1596) | 336 | m/z=737.1599(C49H27N3OS2, 737.1596) |
| 337 | m/z=710.1593(C48H26N2OS2, 710.1487) | 338 | m/z=584.1010(C38H20N2OS2, 584.1017) |
| 339 | m/z=583.1081(C39H21NOS2, 583.1065) | 340 | m/z=633.1205(C43H23NOS2, 633.1221) |
| 341 | m/z=721.1820(C49H27N3O2S, 721.1824) | 342 | m/z=721.1836(C49H27N3O2S, 721.1824) |
| 343 | m/z=721.1826(C49H27N3O2S, 721.1824) | 344 | m/z=721.1843(C49H27N3O2S, 721.1824) |
| 345 | m/z=721.1844 (C49H27N3O2S, 721.1824) | 346 | m/z=721.1844 (C49H27N3O2S, 721.1824) |
| 347 | m/z=694.1748 (C48H26N2O2S, 694.1715) | 348 | m/z=568.1240(C38H20N2O2S, 568.1245) |
| 349 | m/z=643.1621(C45H25NO2S, 643.1606) | 350 | m/z=617.1462 (C43H23NO2S, 617.1449) |
| 351 | m/z=68.1947(C48H28N2OS, 680.1922) | 352 | m/z=730.2081(C52H30N2OS, 730.2079) |
| 353 | m/z=730.2100(C52H30N2OS, 730.2079) | 354 | m/z=730.2086(C52H30N2OS, 730.2079) |
| 355 | m/z=539.1365 (C38H21NOS, 539.1344) | 356 | m/z=681.1889(C47H27N3OS, 681.1875) |
| 357 | m/z=756.2241(C54H32N2OS, 756.2235) | 358 | m/z=730.2081(C52H30N2OS, 730.2079) |
| 359 | m/z=732.1992 (C50H28N4OS, 732.1984) | 360 | m/z=808.2291(C56H32N4OS, 808.2297) |
| 361 | m/z=796.2308(C55H32N4OS, 796.2297) | 362 | m/z=758.2115 (C52H30N4OS, 758.2140) |
| 363 | m/z=858.2541(C60H34N4OS, 858.2453) | 364 | m/z=884.2643(C62H36N4OS, 884.2610) |
| 365 | m/z=807.2340(C57H33N3OS, 807.2344) | 366 | m/z=673.1549(C46H27NOS2, 673.1534) |
| 367 | m/z=673.1562 (C46H27NOS2, 673.1534) | 368 | m/z=673.1534 (C46H27NOS2, 673.1534) |
| 369 | m/z=673.1551(C46H27NOS2, 673.1534) | 370 | m/z=673.1538 (C46H27NOS2, 673.1534) |
| 371 | m/z=673.1530(C46H27NOS2, 673.1534) | 372 | m/z=623.1381(C42H25NOS2, 623.1378) |
| 373 | m/z=623.1418 (C42H25NOS2, 623.1378) | 374 | m/z=623.1415 (C42H25NOS2, 623.1378) |
| 375 | m/z=621.1234 (C42H23NOS2, 621.1221) | 376 | m/z=657.1782 (C46H27NO2S, 657.1762) |
| 377 | m/z=657.1768 (C46H27NO2S, 657.1762) | 378 | m/z=657.1752 (C46H27NO2S, 657.1762) |
| 379 | m/z=657.1782 (C46H27NO2S, 657.1762) | 380 | m/z=657.1760(C46H27NO2S, 657.1762) |
| 381 | m/z=657.1766(C46H27NO2S, 657.1762) | 382 | m/z=607.1646(C42H25NO2S, 607.1606) |
| 383 | m/z=607.1595 (C42H25NO2S, 607.1606) | 384 | m/z=607.1628 (C42H25NO2S, 607.1606) |
| 385 | m/z=605.1558 (C42H23NO2S, 605.1449) | 386 | m/z=680.1920(C48H28N2OS, 680.1922) |
| 387 | m/z=730.2097(C52H30N2OS, 730.2079) | 388 | m/z=730.2062 (C52H30N2OS, 730.2079) |
| 389 | m/z=730.2088 (C52H30N2OS, 730.2079) | 390 | m/z=539.1341(C38H21NOS, 539.1344) |
| 391 | m/z=673.1538 (C46H27NOS2, 673.1534) | 392 | m/z=673.1556(C46H27NOS2, 673.1534) |
| 393 | m/z=673.1555 (C46H27NOS2, 673.1534) | 394 | m/z=623.1389(C42H25NOS2, 623.1378) |
| 395 | m/z=623.1389(C42H25NOS2, 623.1378) | 396 | m/z=657.1778 (C46H27NO2S, 657.1762) |
| 397 | m/z=657.1775 (C46H27NO2S, 657.1762) | 398 | m/z=657.1773(C46H27NO2S, 657.1762) |
| 399 | m/z=607.1657(C42H25NO2S, 607.1606) | 400 | m/z=607.1620(C42H25NO2S, 607.1606) |
| 401 | m/z=439.1031(C30H17NOS, 439.1045) | 402 | m/z=515.1344 (C36H21NOS, 515.1348) |
| 403 | m/z=591.1657(C42H25NOS, 591.1712) | 404 | m/z=515.1344 (C36H21NOS, 515.1352) |
| 405 | m/z=591.1657(C42H25NOS, 591.1651) | 406 | m/z=667.1970(C48H29NOS, 667.1992) |
| 407 | m/z=515.1344 (C36H21NOS, 515.1352) | 408 | m/z=591.1657(C42H25NOS, 591.1628) |
| 409 | m/z=667.1970(C48H29NOS, 667.1992) | 410 | m/z=489.1187(C34H19NOS, 489.1193) |
| 411 | m/z=565.1500(C40H23NOS, 565.1531) | 412 | m/z=565.1500(C40H23NOS, 565.1528) |
| 413 | m/z=565.1500(C40H23NOS, 565.1528) | 414 | m/z=641.1813(C46H27NOS, 641.1804) |
| 415 | m/z=615.1657(C44H25NOS, 6165.1586) | 416 | m/z=515.1344 (C36H21NOS, 515.1331) |
| 417 | m/z=591.1657(C42H25NOS, 591.1628) | 418 | m/z=565.1500(C40H23NOS, 565.1483) |
| 419 | m/z=515.1344 (C36H21NOS, 515.1357) | 420 | m/z=591.1657(C42H25NOS, 591.1661) |
| 421 | m/z=591.1657(C42H25NOS, 591.1662) | 422 | m/z=591.1657(C42H25NOS, 591.1678) |
| 423 | m/z=591.1657(C42H25NOS, 591.1649) | 424 | m/z=591.1657(C42H25NOS, 591.1635) |
| 425 | m/z=515.1344 (C36H21NOS, 515.1331) | 426 | m/z=595.1413(C39H21N3O2S, 595.1354) |
| 427 | m/z=595.1289(C39H21N3O2S, 595.1354) | 428 | m/z=595.1361(C39H21N3O2S, 595.1354) |
| 429 | m/z=645.1524 (C43H23N3O2S, 645.1511) | 430 | m/z=645.1528 (C43H23N3O2S, 645.1511) |
| 431 | m/z=645.1531(C43H23N3O2S, 645.1511) | 432 | m/z=645.1552 (C43H23N3O2S, 645.1511) |
| 433 | m/z=645.1507(C43H23N3O2S, 645.1511) | 434 | m/z=645.1523(C43H23N3O2S, 645.1511) |
| 435 | m/z=645.1528 (C43H23N3O2S, 645.1511) | 436 | m/z=645.1507(C43H23N3O2S, 645.1511) |
| 437 | m/z=671.1679(C45H25N3O2S, 671.1667) | 438 | m/z=671.1674 (C45H25N3O2S, 671.1667) |
| 439 | m/z=671.1642 (C45H25N3O2S, 671.1667) | 440 | m/z=671.1655 (C45H25N3O2S, 671.1667) |
| 441 | m/z=671.1681(C45H25N3O2S, 671.1667) | 442 | m/z=671.1668 (C45H25N3O2S, 671.1667) |
| 443 | m/z=671.1690(C45H25N3O2S, 671.1667) | 444 | m/z=671.1661(C45H25N3O2S, 671.1667) |
| 445 | m/z=671.1629(C45H25N3O2S, 671.1667) | 446 | m/z=721.1840(C49H27N3O2S, 721.1824) |
| 447 | m/z=721.1833(C49H27N3O2S, 721.1824) | 448 | m/z=681.1896(C47H27N3OS, 681.1875) |
| 449 | m/z=731.2047(C51H29N3OS, 731.2031) | 450 | m/z=721.1831(C49H27N3O2S, 721.1824) |
| 451 | m/z=605.1542 (C41H23N3OS, 605.1562) | 452 | m/z=655.1673(C45H25N3OS, 655. 1718) |
| 453 | m/z=655.1689(C45H25N3OS, 655.1718) | 454 | m/z=681.1888 (C47H27N3OS, 681.1875) |
| 455 | m/z=731.2014 (C51H29N3OS, 731. 2031) | | |

### [Experimental Example 1]

### 1) Fabrication of Organic Light Emitting Devices (Red Host)

Glass substrates coated with a thin film of ITO to a thickness of 1,500 Å were washed with distilled water and ultrasonic waves. After washing the substrates with distilled water, ultrasonic cleaning was performed on the washed substrates with solvents such as acetone, methanol, isopropyl alcohol, etc., and the ultrasonic cleaned substrates were dried, and UVO-treated for 5 minutes using UV in a UV cleaner. Thereafter, after transferring the substrates to a plasma cleaner (PT), plasma treatment was performed in order to remove the ITO work function and residual film in a vacuum state, and then the substrates were transferred to thermal deposition equipment for organic deposition.

As common layers on the ITO transparent electrodes (anodes), a hole injection layer 2-TNATA (4,4',4"-Tris [2-naphthyl(phenyl)amino]triphenylamine) and a hole transport layer NPB (N,N'-Di (1-naphthyl)-N,N'-diphenyl-(1,1'-biphenyl) -4, 4'-diamine) were formed.

A light emitting layer was thermal vacuum deposited thereon as follows. The light emitting layer was deposited to a thickness of 500 Å by doping 3 wt% of (piq)₂(Ir)_{(acac)} on the host using the compounds shown in Table 8 below as a host and (piq)₂(Ir)_{(acac)} as a red phosphorescent dopant. Thereafter, BCP was deposited to a thickness of 60 Å as a hole blocking layer, and Alq₃ was deposited to a thickness of 200 Å thereon as an electron transport layer. Finally, after depositing lithium fluoride (LiF) to a thickness of 10 Å on the electron transport layer to form an electron injection layer, organic light emitting devices were manufactured by depositing an aluminum (Al) cathode to a thickness of 1,200 Å on the electron injection layer to form cathodes.

Meanwhile, all organic compounds required for fabricating OLED devices were vacuum sublimated and purified under 10⁻⁶ to 10⁻⁸ torr for each material respectively, and used for OLED fabrication.

### 2) Driving voltages and Luminous efficiencies of Organic Light Emitting Devices

Electroluminescence (EL) characteristics were measured for the organic light emitting devices fabricated as described above with M7000 of McScience, and T₉₀ values were measured with the measurement results when the reference luminance was 6,000 cd/m² through the device lifespan measuring system (M6000) manufactured by McScience. The characteristics of the organic light emitting devices of the present disclosure are as shown in Table 8 below.

**[Table 8]**

| | Compound | Driving voltage (V) | Efficiency (cd/A) | Color coordinates (x, y) | Lifespan (T₉₀) |
|---|---|---|---|---|---|
| Comparative Example 1 | A | 5.28 | 27.8 | (0.685, 0.315) | 30 |
| Comparative Example 2 | B | 5.67 | 18.1 | (0.685, 0.315) | 27 |
| Comparative Example 3 | C | 5.30 | 19.7 | (0.685, 0.315) | 31 |
| Comparative Example 4 | D | 5.33 | 26.9 | (0.685, 0.315) | 40 |
| Comparative Example 5 | E | 5.62 | 22.0 | (0.685, 0.315) | 25 |
| Comparative Example 6 | F | 5.54 | 22.5 | (0.685, 0.315) | 34 |
| Comparative Example 7 | G | 5.19 | 17.9 | (0.685, 0.315) | 38 |
| Comparative Example 8 | H | 5.27 | 16.8 | (0.685, 0.315) | 29 |
| Comparative Example 9 | I | 5.32 | 21.5 | (0.685, 0.315) | 42 |
| Example 1 | 1 | 4.66 | 31.8 | (0.685, 0.315) | 76 |
| Example 2 | 2 | 4.31 | 30.5 | (0.685, 0.315) | 78 |
| Example 3 | 4 | 4.41 | 33.5 | (0.685, 0.315) | 79 |
| Example 4 | 5 | 4.74 | 34.9 | (0.685, 0.315) | 131 |
| Example 5 | 6 | 4.80 | 34.2 | (0.685, 0.315) | 129 |
| Example 6 | 9 | 4.63 | 32.8 | (0.685, 0.315) | 135 |
| Example 7 | 10 | 4.79 | 33.2 | (0.685, 0.315) | 128 |
| Example 8 | 12 | 4.28 | 29.8 | (0.685, 0.315) | 88 |
| Example 9 | 14 | 4.71 | 32.6 | (0.685, 0.315) | 129 |
| Example 10 | 17 | 4.75 | 32.9 | (0.685, 0.315) | 154 |
| Example 11 | 23 | 4.81 | 34.5 | (0.685, 0.315) | 183 |
| Example 12 | 26 | 4.41 | 35.1 | (0.685, 0.315) | 201 |
| Example 13 | 36 | 4.74 | 36.4 | (0.685, 0.315) | 230 |
| Example 14 | 38 | 4.73 | 36.5 | (0.685, 0.315) | 249 |
| Example 15 | 41 | 4.78 | 35.5 | (0.685, 0.315) | 143 |
| Example 16 | 51 | 4.81 | 34.0 | (0.685, 0.315) | 234 |
| Example 17 | 67 | 4.11 | 33.8 | (0.685, 0.315) | 243 |
| Example 18 | 68 | 4.80 | 27.9 | (0.685, 0.315) | 155 |
| Example 19 | 70 | 4.55 | 31.3 | (0.685, 0.315) | 134 |
| Example 20 | 72 | 4.82 | 33.5 | (0.685, 0.315) | 120 |
| Example 21 | 80 | 4.26 | 35.9 | (0.685, 0.315) | 86 |
| Example 22 | 84 | 4.33 | 39.8 | (0.685, 0.315) | 145 |
| Example 23 | 95 | 4.10 | 34.6 | (0.685, 0.315) | 163 |
| Example 24 | 97 | 4.81 | 38.9 | (0.685, 0.315) | 279 |
| Example 25 | 106 | 4.94 | 41.6 | (0.685, 0.315) | 290 |
| Example 26 | 109 | 4.81 | 33.5 | (0.685, 0.315) | 132 |
| Example 27 | 118 | 4.69 | 37.9 | (0.685, 0.315) | 128 |
| Example 28 | 122 | 4.21 | 33.4 | (0.685, 0.315) | 89 |
| Example 29 | 126 | 4.88 | 31.8 | (0.685, 0.315) | 124 |
| Example 30 | 140 | 4.64 | 29.8 | (0.685, 0.315) | 243 |
| Example 31 | 141 | 4.81 | 27.1 | (0.685, 0.315) | 123 |
| Example 32 | 143 | 4.41 | 38.4 | (0.685, 0.315) | 95 |
| Example 33 | 147 | 4.21 | 37.9 | (0.685, 0.315) | 66 |
| Example 34 | 148 | 4.72 | 32.0 | (0.685, 0.315) | 118 |
| Example 35 | 149 | 4.69 | 35.9 | (0.685, 0.315) | 74 |
| Example 36 | 151 | 4.67 | 41.6 | (0.685, 0.315) | 118 |
| Example 37 | 152 | 4.74 | 40.2 | (0.685, 0.315) | 111 |
| Example 38 | 165 | 4.80 | 38.1 | (0.685, 0.315) | 188 |
| Example 39 | 172 | 4.67 | 37.8 | (0.685, 0.315) | 154 |
| Example 40 | 173 | 4.35 | 32.6 | (0.685, 0.315) | 126 |
| Example 41 | 201 | 4.10 | 34.4 | (0.685, 0.315) | 168 |
| Example 42 | 202 | 4.65 | 31.6 | (0.685, 0.315) | 80 |
| Example 43 | 208 | 4.50 | 32.0 | (0.685, 0.315) | 142 |
| Example 44 | 236 | 4.44 | 38.1 | (0.685, 0.315) | 171 |
| Example 45 | 249 | 4.31 | 39.8 | (0.685, 0.315) | 182 |
| Example 46 | 256 | 4.29 | 33.7 | (0.685, 0.315) | 123 |
| Example 47 | 258 | 4.50 | 31.8 | (0.685, 0.315) | 130 |
| Example 48 | 276 | 4.52 | 30.5 | (0.685, 0.315) | 135 |
| Example 49 | 310 | 4.15 | 38.4 | (0.685, 0.315) | 94 |
| Example 50 | 351 | 4.23 | 39.0 | (0.685, 0.315) | 143 |
| Example 51 | 384 | 4.33 | 34.5 | (0.685, 0.315) | 137 |
| Example 52 | 386 | 4.25 | 38.9 | (0.685, 0.315) | 142 |
| Example 53 | 426 | 4.38 | 34.5 | (0.685, 0.315) | 89 |
| Example 54 | 433 | 4.27 | 38.7 | (0.685, 0.315) | 94 |
| Example 55 | 437 | 4.33 | 40.1 | (0.685, 0.315) | 101 |
| Example 56 | 441 | 4.34 | 39.8 | (0.685, 0.315) | 104 |
| Example 57 | 448 | 4.29 | 41.6 | (0.685, 0.315) | 141 |
| Example 58 | 450 | 4.24 | 43.2 | (0.685, 0.315) | 159 |

### <Experimental Example 2> Fabrication of Organic Light Emitting Devices

Glass substrates coated with a thin film of ITO to a thickness of 1,500 Å were washed with distilled water and ultrasonic waves. After washing the substrates with distilled water, ultrasonic cleaning was performed on the washed substrates with solvents such as acetone, methanol, isopropyl alcohol, etc., and the ultrasonic cleaned substrates were dried, and UVO-treated for 5 minutes using UV in a UV cleaner. Thereafter, after transferring the substrates to a plasma cleaner (PT), plasma treatment was performed in order to remove the ITO work function and residual film in a vacuum state, and then the substrates were transferred to thermal deposition equipment for organic deposition.

As common layers on the ITO transparent electrodes (anodes), a hole injection layer 2-TNATA (4,4',4"-Tris [2-naphthyl(phenyl)amino]triphenylamine) and a hole transport layer NPB (N,N'-Di (1-naphthyl)-N,N'-diphenyl-(1,1'-biphenyl) -4, 4'-diamine) were formed.

A light emitting layer was thermal vacuum deposited thereon as follows. The light emitting layer was deposited to a thickness of 500 Å by doping 3 wt% of (piq)₂(Ir)_{(acac)} on the host using one of the compounds shown in Table 9 below as a host and (piq)₂(Ir)_{(acac)} as a red phosphorescent dopant. Thereafter, BCP was deposited to a thickness of 60 Å as a hole blocking layer, and Alq₃ was deposited to a thickness of 200 Å thereon as an electron transport layer. Finally, after depositing lithium fluoride (LiF) to a thickness of 10 Å on the electron transport layer to form an electron injection layer, organic light emitting devices were manufactured by depositing an aluminum (Al) cathode to a thickness of 1,200 Å on the electron injection layer to form cathodes.

Meanwhile, all organic compounds required for fabricating OLED devices were vacuum sublimated and purified under 10⁻⁶ to 10⁻⁸ torr for each material respectively, and used for OLED fabrication.

### 2) Driving voltages and Luminous efficiencies of Organic Light Emitting Devices

Electroluminescence (EL) characteristics were measured for the organic light emitting devices fabricated as described above with M7000 of McScience, and T₉₀ values were measured with the measurement results when the reference luminance was 6,000 cd/m² through the device lifespan measuring system (M6000) manufactured by McScience. The characteristics of the organic light emitting devices according to an embodiment of the present disclosure are as shown in Table 9.

**[Table 9]**

| | Compound | Ratio | Driving voltage (V) | Efficiency (cd/A) | Color coordinates (x, y) | Lifespan (T₉₀) |
|---|---|---|---|---|---|---|
| Comparative Example 10 | E : F | 1 : 2 | 5.86 | 28.7 | (0.685, 0.315) | 89 |
| Comparative Example 11 | | 1 : 1 | 5.72 | 29.9 | (0.685, 0.315) | 74 |
| Comparative Example 12 | | 2 : 1 | 5.91 | 27.1 | (0.685, 0.315) | 49 |
| Comparative Example 13 | E : 51 | 1 : 2 | 5.34 | 38.1 | (0.685, 0.315) | 40 |
| Comparative Example 14 | | 1 : 1 | 5.21 | 41.4 | (0.685, 0.315) | 51 |
| Comparative Example 15 | | 2 : 1 | 5.45 | 46.8 | (0.685, 0.315) | 39 |
| Comparative Example 16 | 6 : F | 1 : 2 | 4.98 | 44.7 | (0.685, 0.315) | 76 |
| Comparative Example 17 | | 1 : 1 | 4.92 | 52.9 | (0.685, 0.315) | 57 |
| Comparative Example 18 | | 2 : 1 | 5.02 | 51.1 | (0.685, 0.315) | 48 |
| Example 59 | 6 : 51 | 1 : 2 | 3.96 | 92.8 | (0.685, 0.315) | 79 |
| Example 60 | | 1 : 1 | 3.81 | 101.2 | (0.685, 0.315) | 78 |
| Example 61 | | 2 : 1 | 3.99 | 93.9 | (0.685, 0.315) | 67 |
| Example 62 | 5 : 67 | 1 : 2 | 3.89 | 97.8 | (0.685, 0.315) | 101 |
| Example 63 | | 1 : 1 | 3.75 | 99.3 | (0.685, 0.315) | 120 |
| Example 64 | | 2 : 1 | 3.87 | 102.9 | (0.685, 0.315) | 118 |
| Example 65 | 9 : 97 | 1 : 2 | 3.58 | 98.5 | (0.685, 0.315) | 129 |
| Example 66 | | 1 : 1 | 3.79 | 101.8 | (0.685, 0.315) | 135 |
| Example 67 | | 2 : 1 | 3.86 | 105.9 | (0.685, 0.315) | 127 |
| Example 68 | 12 : 51 | 1 : 8 | 4.31 | 101.2 | (0.685, 0.315) | 261 |
| Example 69 | | 1 : 5 | 4.25 | 104.9 | (0.685, 0.315) | 248 |
| Example 70 | | 1 : 2 | 4.40 | 105.7 | (0.685, 0.315) | 224 |
| Example 71 | | 1 : 1 | 4.45 | 108.6 | (0.685, 0.315) | 181 |
| Example 72 | | 2 : 1 | 4.01 | 110.8 | (0.685, 0.315) | 155 |
| Example 73 | | 5 : 1 | 3.83 | 106.0 | (0.685, 0.315) | 138 |
| Example 74 | | 8 : 1 | 3.89 | 103.3 | (0.685, 0.315) | 119 |
| Example 75 | 23 : 95 | 1 : 2 | 4.23 | 111.8 | (0.685, 0.315) | 254 |
| Example 76 | | 1 : 1 | 3.97 | 115.5 | (0.685, 0.315) | 235 |
| Example 77 | | 2 : 1 | 3.78 | 120.7 | (0.685, 0.315) | 213 |
| Example 78 | 41 : 172 | 1 : 8 | 4.17 | 101.1 | (0.685, 0.315) | 189 |
| Example 79 | | 1 : 5 | 4.09 | 109.5 | (0.685, 0.315) | 182 |
| Example 80 | | 1 : 2 | 4.12 | 114.1 | (0.685, 0.315) | 202 |
| Example 81 | | 1 : 1 | 3.98 | 119.9 | (0.685, 0.315) | 198 |
| Example 82 | | 2 : 1 | 3.87 | 121.7 | (0.685, 0.315) | 172 |
| Example 83 | | 5 : 1 | 3.85 | 114.4 | (0.685, 0.315) | 143 |
| Example 84 | | 8 : 1 | 3.96 | 100.6 | (0.685, 0.315) | 126 |
| Example 85 | 202:256 | 1 : 8 | 4.05 | 99.8 | (0.685, 0.315) | 183 |
| Example 86 | | 1 : 5 | 3.98 | 104.7 | (0.685, 0.315) | 202 |
| Example 87 | | 1 : 2 | 3.99 | 107.4 | (0.685, 0.315) | 188 |
| Example 88 | | 1 : 1 | 4.02 | 108.5 | (0.685, 0.315) | 194 |
| Example 89 | | 2 : 1 | 3.81 | 112.6 | (0.685, 0.315) | 142 |
| Example 90 | | 5 : 1 | 3.87 | 114.9 | (0.685, 0.315) | 133 |
| Example 91 | | 8 : 1 | 4.01 | 116.2 | (0.685, 0.315) | 128 |
| Example 92 | 433:351 | 1 : 2 | 4.12 | 104.7 | (0.685, 0.315) | 260 |
| Example 93 | | 1 : 1 | 3.96 | 103.6 | (0.685, 0.315) | 250 |
| Example 94 | | 2 : 1 | 3.89 | 107.2 | (0.685, 0.315) | 228 |

Compounds A to I used in Tables 8 and 9 above are as follows.

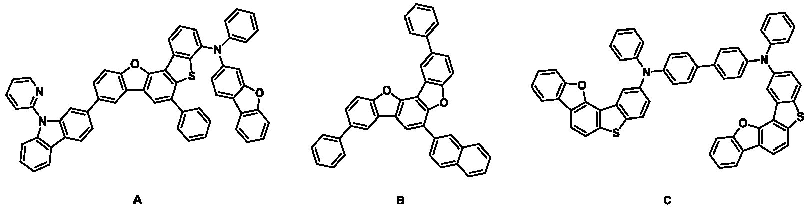

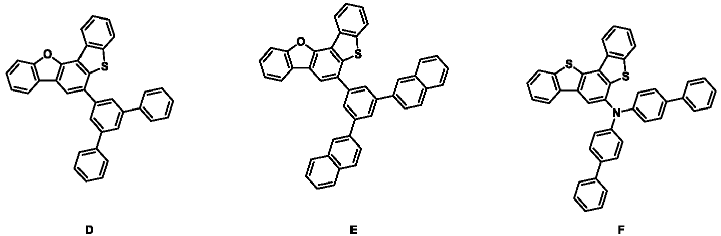

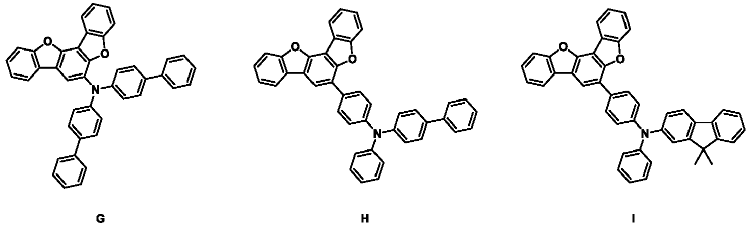

As can be seen from the results of Tables 8 and 9 above, in the case of the organic light emitting devices of Examples 1 to 94 in which the heterocyclic compound of Chemical Formula 1 above of the present application was used as a host for the organic material layer, particularly the light emitting layer, of the organic light emitting device, it could be confirmed that the driving voltage and efficiency could be improved compared to those of the organic light emitting devices of Comparative Examples 1 to 18.

That is, when compared with Compounds A to C used in Comparative Examples 1 to 3, it could be confirmed that the heterocyclic compound of Chemical Formula 1 above of the present application used in Examples 1 to 94 has a steric arrangement by fixing the position of an amine group or a heteroaryl group corresponding to the substituent Z of Chemical Formula 1 above to a specific position as in Chemical Formula 1 above, the heterocyclic compound is suitable as a red host since a strong charge transfer is possible by spatially separating Highest Occupied Molecular Orbital (HOMO) and Lowest Unoccupied Molecular Orbital (LUMO), and high efficiency may be expected when it is used as an organic material in an organic light emitting device.

Further, HOMO, LUMO, Eg, S1, T1, and dipole moment values were respectively calculated for the molecules of Compounds D to I used in Comparative Examples 4 to 18 above and Compounds 1, 6, 51, 95, and 100 included in the heterocyclic compounds of Chemical Formula 1 above of the present application, and the results are shown in Table 10 below.

Eg, T1, and S1 denote a bandgap, a triplet excited state (T1 level), and a singlet excited state (S1 level) respectively.

**[Table 10]**

| Compound | HOMO | LUMO | Eg | S1 | T1 | dipole moment |
|---|---|---|---|---|---|---|
| 1 | -5.98 | -2.26 | 3.71 | 3.16 | 2.74 | 0.85 |
| 6 | -5.96 | -2.34 | 3.63 | 3.11 | 2.47 | 1.02 |
| 51 | -5.29 | -1.66 | 3.63 | 3.07 | 2.74 | 1.15 |
| 95 | -5.27 | -1.54 | 3.73 | 3.29 | 2.75 | 0.91 |
| 100 | -5.19 | -1.53 | 3.66 | 3.23 | 2.64 | 1.35 |
| D | -5.87 | -1.58 | 4.29 | 3.71 | 2.92 | 0.64 |
| E | -5.87 | -1.66 | 4.21 | 3.70 | 2.59 | 0.64 |
| F | -5.30 | -1.65 | 3.65 | 3.08 | 2.73 | 0.84 |
| G | -5.24 | -1.61 | 3.63 | 3.06 | 2.72 | 0.97 |
| H | -5.22 | -1.55 | 3.66 | 3.21 | 2.67 | 0.59 |
| I | -5.14 | -1.55 | 3.59 | 3.15 | 2.60 | 0.85 |

It can be confirmed from the molecular calculation results of Table 10 above that the band gap is smaller when Z in Chemical Formula 1 above of the present application is an amine group or a heteroaryl group than when it is an aryl group (Compounds D and E).

That is, the compounds (Compounds 1 and 6) in which Z of Chemical Formula 1 above is a heteroaryl group having N-type characteristics have a lower LUMO than the compounds (Compounds D and E) in which Z of Chemical Formula 1 above is an aryl group, thereby facilitating electron injection.

Further, the compounds (Compounds 51, 95, and 100) in which Z of Chemical Formula 1 above is an amine group having P-type characteristics has a higher HOMO than the compounds (Compounds D and E) in which Z of Chemical Formula 1 above is an aryl group, thereby facilitating hole injection.

The cause of such results is that an exciplex phenomenon, which is a phenomenon of emission by an energy difference between the LUMO level of the acceptor (N-type host) and the HOMO level of the donor (P-type host) due to electron exchange between two molecules, is more likely to occur in the heterocyclic compound represented by Chemical Formula 1 according to the present application when the heterocyclic compound represented by Chemical Formula 1 according to the present application is used in a device by having as substituents a heteroaryl group having an N-type characteristic rather than an aryl group that is the substituent Z of Chemical Formula 1 above, and an amine group having a P-type characteristic rather than the aryl group.

When a donor (P-type host) having a good hole transport ability and an acceptor (N-type host) having a good electron transport ability are used as a host of the light emitting layer by the exciplex phenomenon, since holes are injected into the P-type host and electrons are injected into the N-type host, the electrons and holes are easily combined in the light emitting layer.

That is, when the heterocyclic compound represented by Chemical Formula 1 above of the present application is used in a device, it can be confirmed that there is an effect of improving the driving voltage and efficiency compared to when Z of Chemical Formula 1 above is an aryl group.

Further, as can be seen from the results of Table 9 above, it can be confirmed that effects of improving the driving voltage and efficiency are excellent when X1 and X2 above are different compared to when X1 and X2 of Chemical Formula 1 above of the present application are the same (Compounds F to I).

It is judged that this is since, when looking at the molecular calculation results of Table 10 above, as the HOMO of the heteroskeleton composed of two or more atoms is formed lower than the HOMO of the heteroskeleton composed of one atom, energy may be transferred more efficiently without energy loss.

Further, as can be seen from the molecular calculation results of Table 10 above, it is judged that, as the dipole moment of the heteroskeleton composed of two or more atoms is formed higher than the dipole moment of the heteroskeleton composed of one atom, the recombination ratio of the electrons and holes is increased, and the driving voltage and lifespan characteristics of the device are excellent.

### <Reference Numeral>

- 100:: Substrate
- 200:: Anode
- 300:: Organic Material Layer
- 301:: Hole Injection Layer
- 302:: Hole Transport Layer
- 303:: Light Emitting Layer
- 304:: Hole Blocking Layer
- 305:: Electron Transport Layer
- 306:: Electron Injection Layer
- 400:: Cathode

## Claims

1. A heterocyclic compound represented by the following Chemical Formula 1: 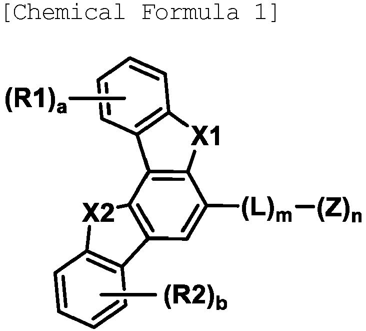 In Chemical Formula 1,
X1 and X2 are each independently O; or S, wherein X1 and X2 are different from each other,
L is a direct bond; a substituted or unsubstituted C₆-C₆₀ arylene group; or a substituted or unsubstituted C₂-C₆₀ heteroarylene group,
Z is a substituted or unsubstituted amine group; or a substituted or unsubstituted C₂-C₆₀ heteroaryl group,
R1 and R2 are the same as or different from each other, and are each independently hydrogen; heavy hydrogen; a cyano group; a nitro group; a substituted or unsubstituted C₁-C₆₀ alkyl group; a substituted or unsubstituted C₁-C₂₀ alkoxy group; a substituted or unsubstituted C₃-C₆₀ cycloalkyl group; a substituted or unsubstituted C₂-C₆₀ alkenyl group; a substituted or unsubstituted C₂-C₆₀ alkynyl group; a substituted or unsubstituted C₁-C₆₀ aryl group; a substituted or unsubstituted C₂-C₆₀ heterocycloalkyl group; a substituted or unsubstituted C₂-C₆₀ heteroaryl group; a substituted or unsubstituted phosphine oxide group; or a substituted or unsubstituted silyl group,
a and b are each independently an integer of 0 to 4, and when a and b are each 2 or more, the substituents in parentheses are the same as or different from each other, and
m and n are each independently an integer of 0 to 6, and when m and n are each 2 or more, the substituents in parentheses are the same as or different from each other.

2. The heterocyclic compound of claim 1, wherein the "substituted or unsubstituted" means that it is substituted or unsubstituted with one or more substituents selected from the group consisting of: a C₁-C₆₀ linear or branched alkyl group; a C₂-C₆₀ linear or branched alkenyl group; a C₂-C₆₀ linear or branched alkynyl group; a C₃-C₆₀ monocyclic or polycyclic cycloalkyl group; a C₂-C₆₀ monocyclic or polycyclic heterocycloalkyl group; a C₆-C₆₀ monocyclic or polycyclic aryl group; a C₂-C₆₀ monocyclic or polycyclic heteroaryl group; a silyl group; a phosphine oxide group; and an amine group, or is substituted or unsubstituted with a substituent to which two or more substituents selected from the above-exemplified substituents are connected.

3. The heterocyclic compound of claim 1, wherein Chemical Formula 1 above is represented by the following Chemical Formula 1-1 or 1-2: 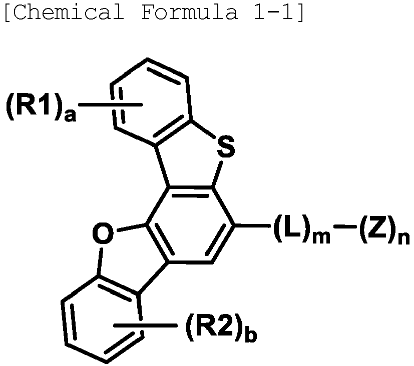 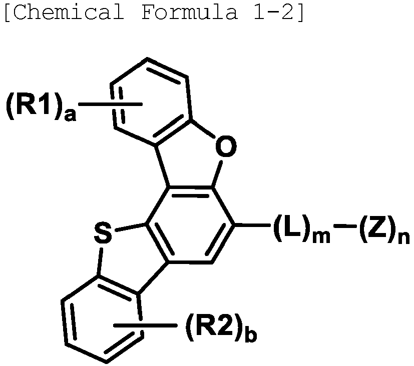 In Chemical Formulas 1-1 and 1-2, the definition of each substituent is the same as in Chemical Formula 1.

4. The heterocyclic compound of claim 1, wherein Z of Chemical Formula 1 above is a group represented by any one of the following Chemical Formulas 2 to 4: 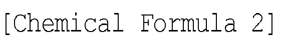 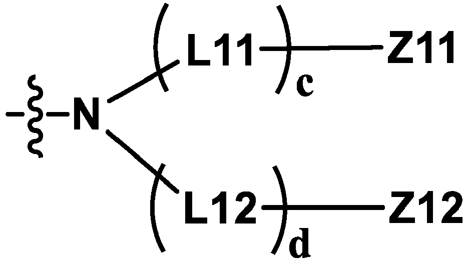 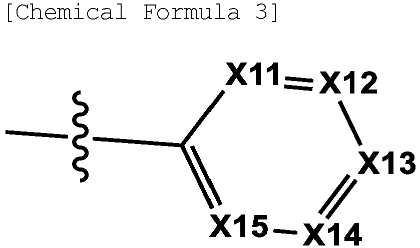 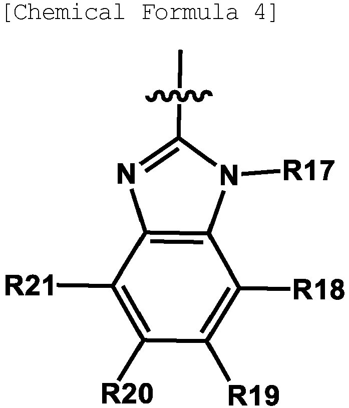 In Chemical Formulas 2 to 4,
L11 and L12 are the same as or different from each other, and are each independently a direct bond; and a substituted or unsubstituted C₆-C₄₀ arylene group or a substituted or unsubstituted C₂-C₄₀ heteroarylene group, c and d are each 0 or 1,
Z11 and Z12 may be the same as or different from each other, and may be each independently a substituted or unsubstituted C₆-C₄₀ aryl group or a substituted or unsubstituted C₂-C₄₀ heteroaryl group, and Z11 and Z12 may be bonded to each other to form a substituted or unsubstituted C₆-C₆₀ aromatic hydrocarbon ring or a substituted or unsubstituted C₂-C₆₀ heterocycle,
X11 is CR11 or N, X12 is CR12 or N, X13 is CR13 or N, X14 is CR14 or N, X15 is CR15 or N, and at least one of X11 to X15 is N, and
R11 to R15 and R17 to R21 may be the same as or different from each other, and may be each independently selected from the group consisting of: hydrogen; heavy hydrogen; halogen; a cyano group; a substituted or unsubstituted C₁-C₆₀ alkyl group; a substituted or unsubstituted C₂-C₆₀ alkenyl group; a substituted or unsubstituted C₂-C₆₀ alkynyl group; a substituted or unsubstituted C₁-C₂₀ alkoxy group; a substituted or unsubstituted C₃-C₆₀ cycloalkyl group; a substituted or unsubstituted C₂-C₆₀ heterocycloalkyl group; a substituted or unsubstituted C₆-C₆₀ aryl group; a substituted or unsubstituted C₂-C₆₀ heteroaryl group; a substituted or unsubstituted phosphine oxide group; and a substituted or unsubstituted amine group, or two or more groups adjacent to each other may be bonded to each other to form a substituted or unsubstituted C₆-C₆₀ aromatic hydrocarbon ring or a substituted or unsubstituted C₂-C₆₀ heterocycle, and 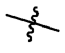 denotes a position bonded to Chemical Formula 1 above.

5. The heterocyclic compound of claim 4, wherein Chemical Formula 2 above is represented by any one of the following Chemical Formulas 2-1 to 2-4: 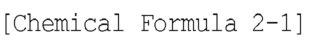 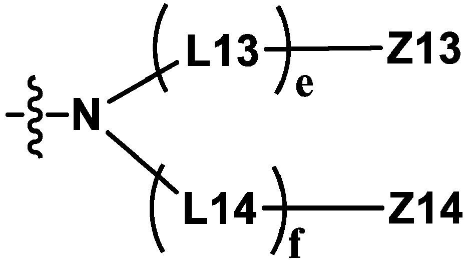 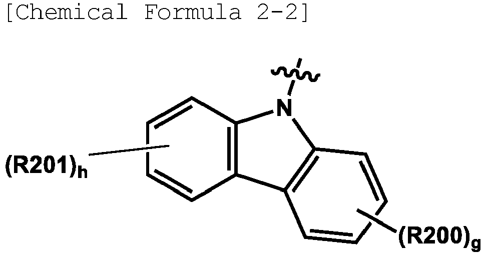 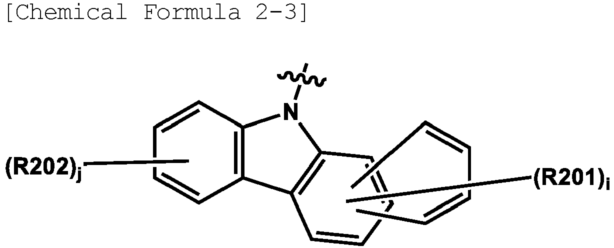 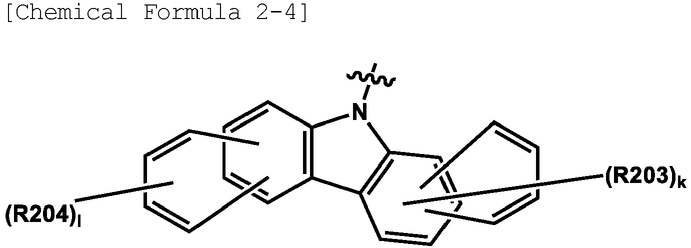 In Chemical Formulas 2-1 to 2-4,
L13 and L14 are the same as or different from each other, and are each independently a direct bond; a substituted or unsubstituted C₆-C₄₀ arylene group; or a substituted or unsubstituted C₂-C₄₀ heteroarylene group,
Z13 and Z14 are the same as or different from each other, and are each independently a substituted or unsubstituted C₆-C₄₀ aryl group; or a substituted or unsubstituted C₂-C₄₀ heteroaryl group,
R200 to R204 are each independently hydrogen; heavy hydrogen; a halogen group; a cyano group; a substituted or unsubstituted C₆-C₂₀ aryl group; or a substituted or unsubstituted C₂-C₂₀ heteroaryl group,
e and f are each 0 or 1,
g, h, j, and l are each an integer of 0 to 4, i and k are integers of 0 to 6, and when g to l are each 2 or more, the substituents in parentheses are the same as or different from each other, and 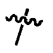
denotes a position bonded to L1 of Chemical Formula 1 above.

6. The heterocyclic compound of claim 4, wherein Chemical Formula 3 above is represented by one of the following Chemical Formulas 3-1 to 3-4: 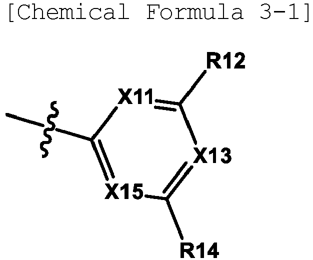 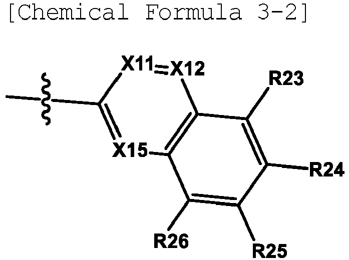 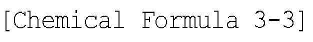 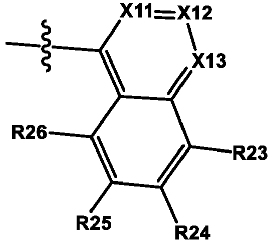 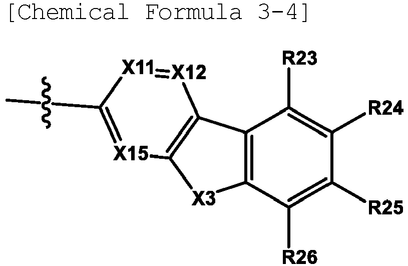
In Chemical Formula 3-1, one or more of X11, X13, and X15 are N, and the rest are as defined in Chemical Formula 3,
In Chemical Formula 3-2, one or more of X11, X12, and X15 are N, and the rest are as defined in Chemical Formula 3,
In Chemical Formula 3-3, one or more of X11 to X13 are N, and the rest are as defined in Chemical Formula 3,
In Chemical Formula 3-4, one or more of X11, X12, and X15 are N, and the rest are as defined in Chemical Formula 3,
X3 is O; or S,
R12, R14, and R23 to R26 are the same as or different from each other, and are each independently selected from the group consisting of: hydrogen; heavy hydrogen; halogen; a cyano group; a substituted or unsubstituted C₁-C₆₀ alkyl group; a substituted or unsubstituted C₂-C₆₀ alkenyl group; a substituted or unsubstituted C₂-C₆₀ alkynyl group; a substituted or unsubstituted C₁-C₂₀ alkoxy group; a substituted or unsubstituted C₃-C₆₀ cycloalkyl group; a substituted or unsubstituted C₂-C₆₀ heterocycloalkyl group; a substituted or unsubstituted C₆-C₆₀ aryl group; a substituted or unsubstituted C₆-C₆₀ heteroaryl group; a substituted or unsubstituted phosphine oxide group; and a substituted or unsubstituted amine group, or two or more groups adjacent to each other are bonded to each other to form a substituted or unsubstituted aliphatic or aromatic hydrocarbon ring or heterocycle, and 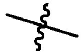
denotes a position bonded to Chemical Formula 1 above.

7. The heterocyclic compound of claim 1, wherein Chemical Formula 1 above is represented by any one of the following compounds:

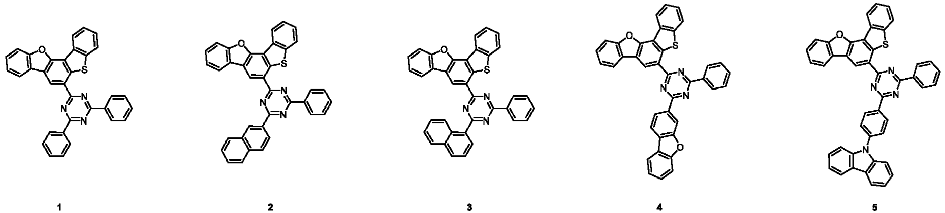

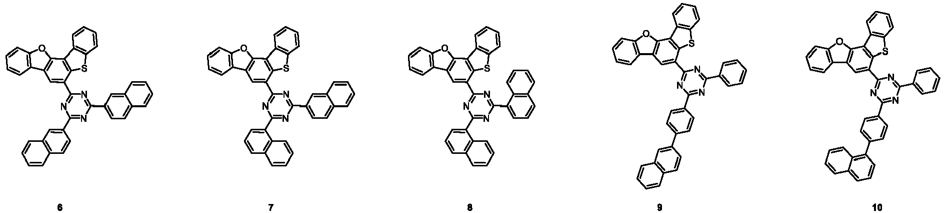

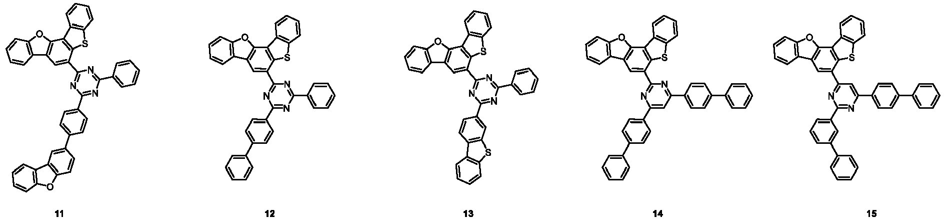

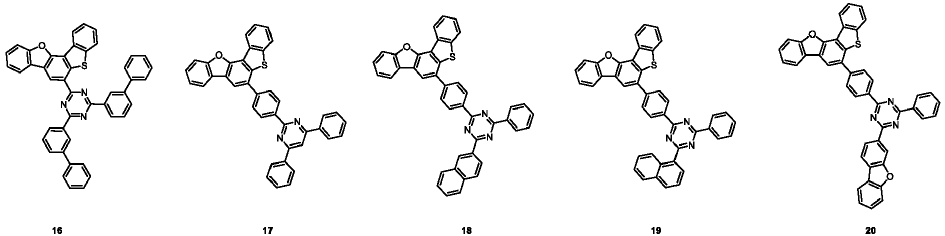

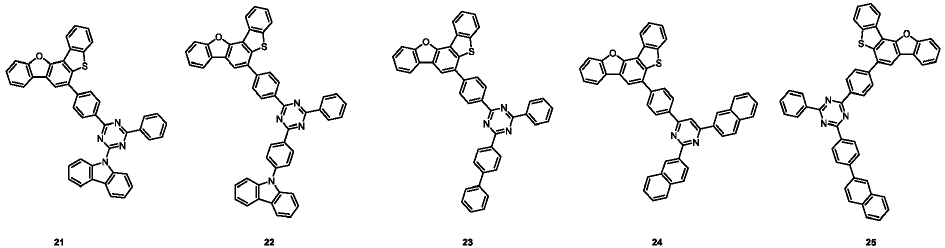

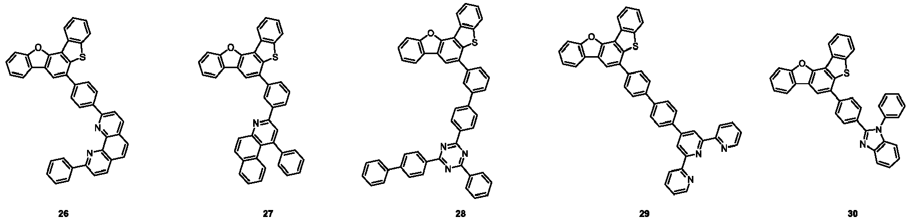

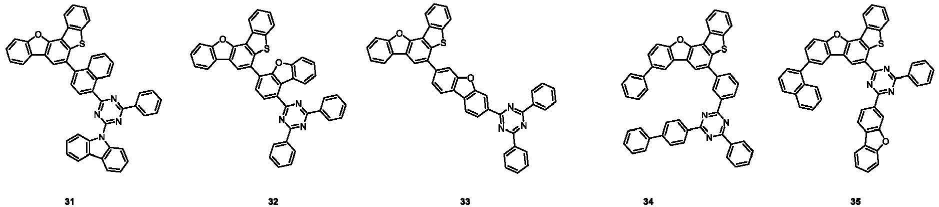

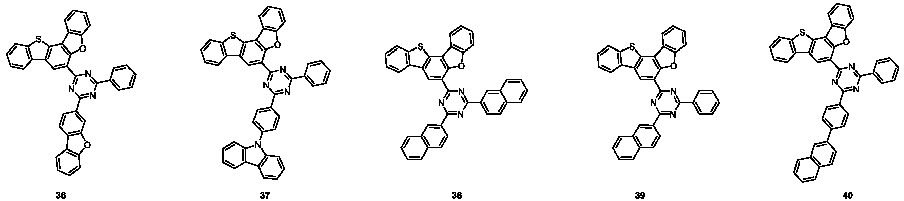

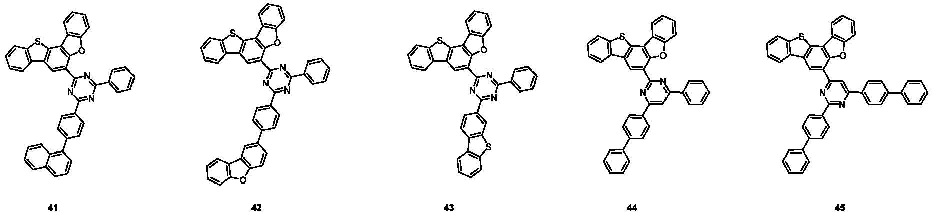

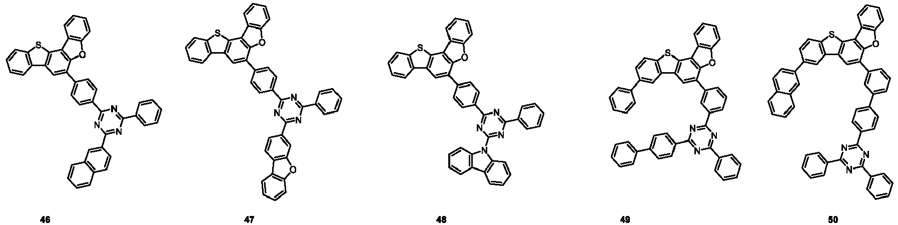

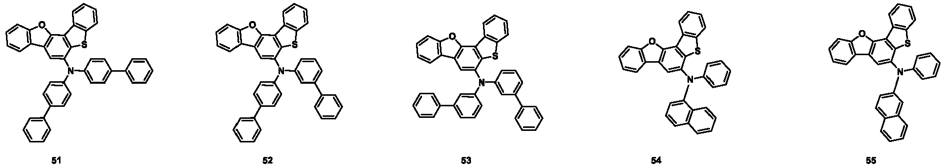

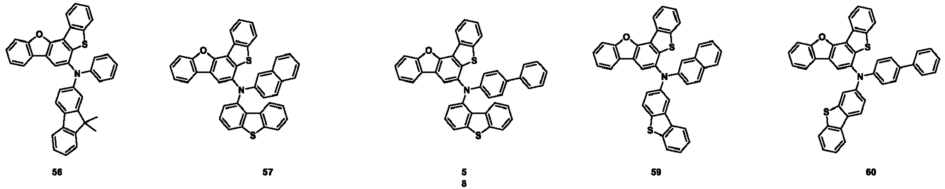

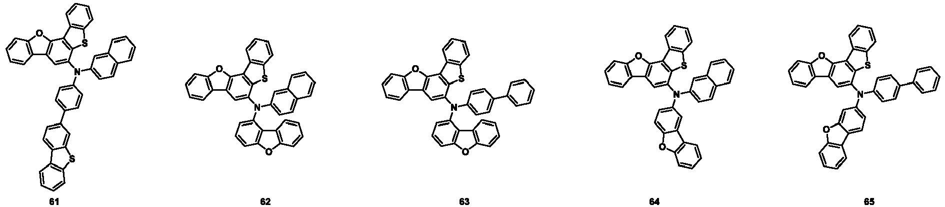

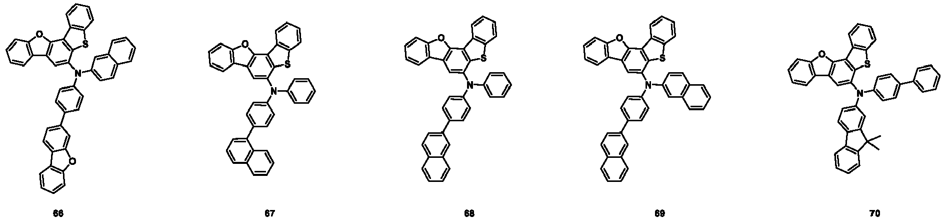

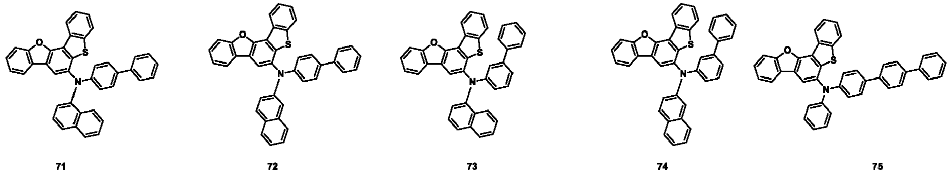

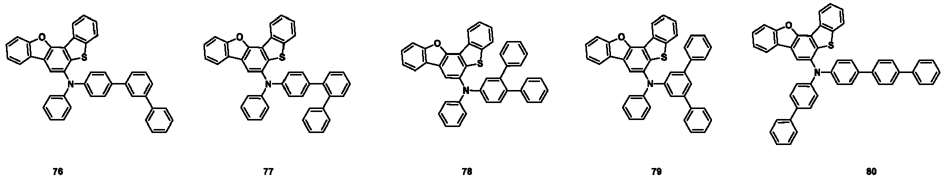

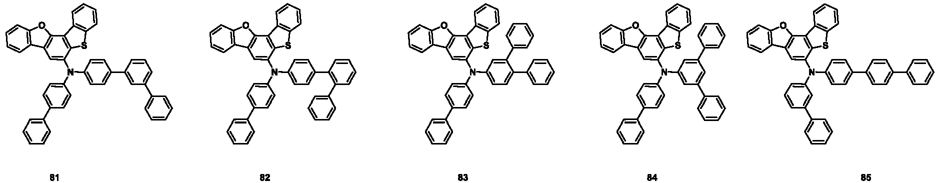

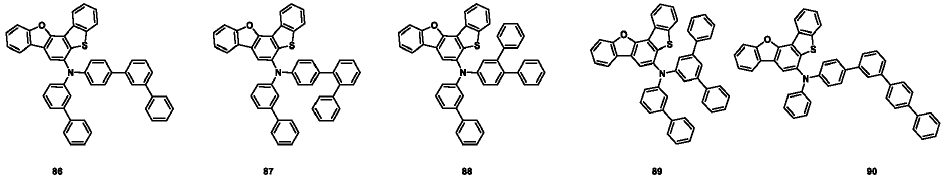

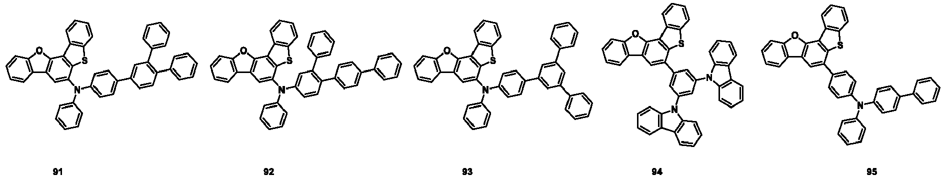

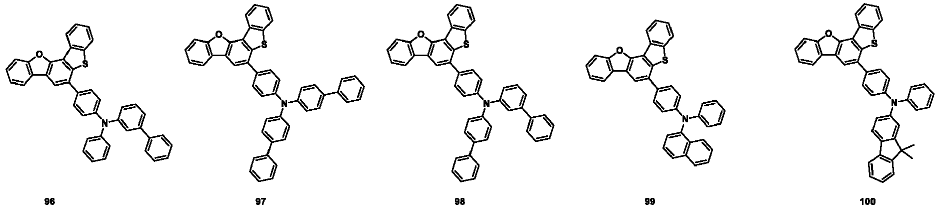

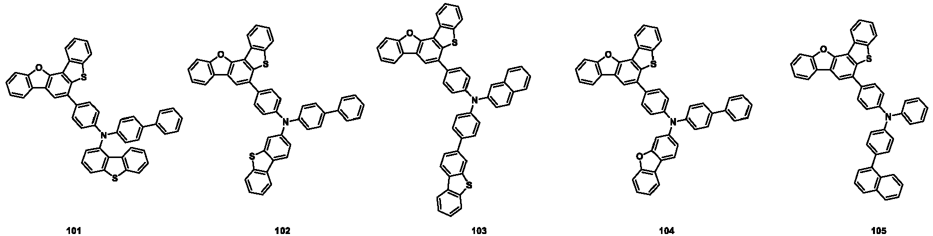

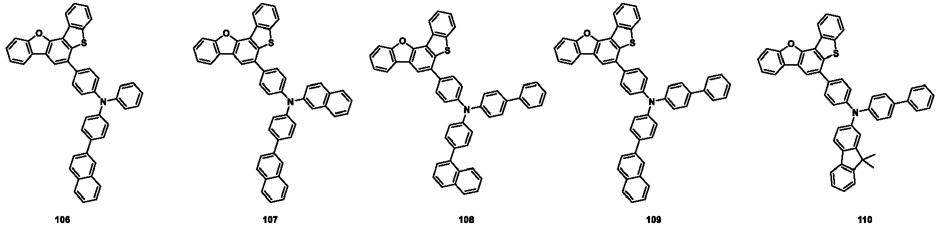

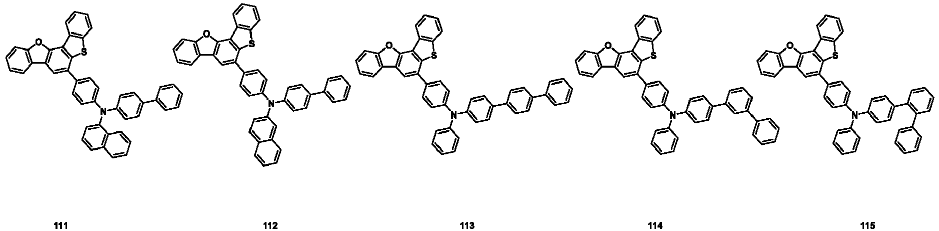

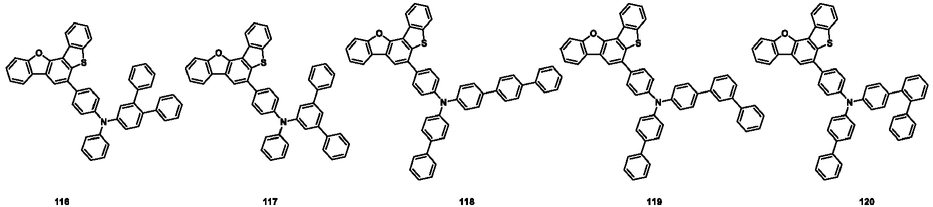

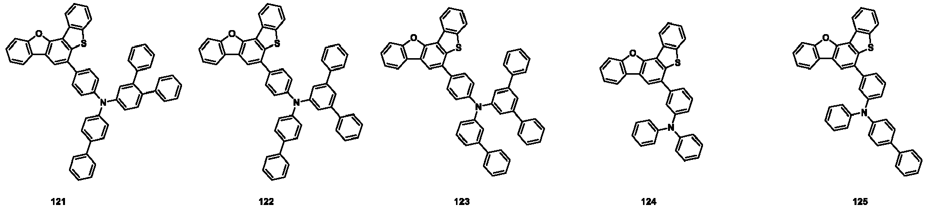

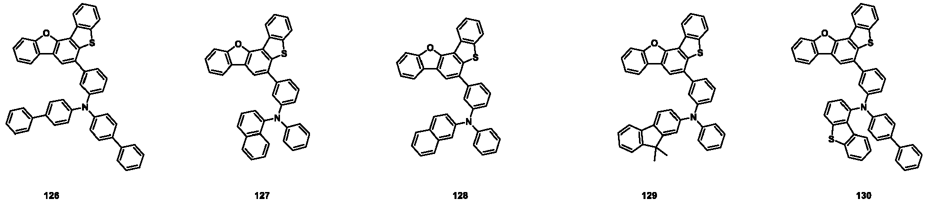

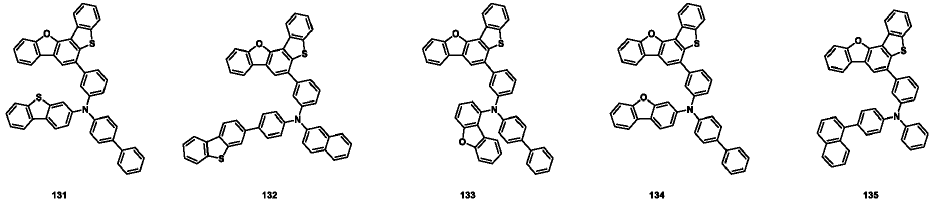

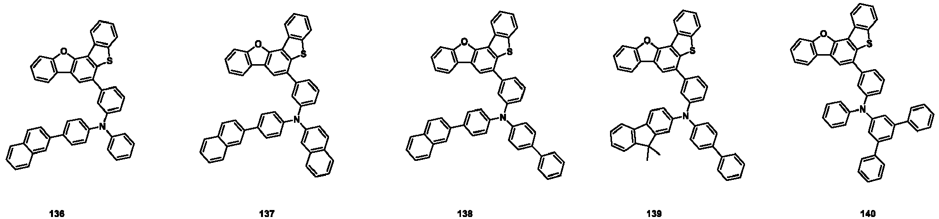

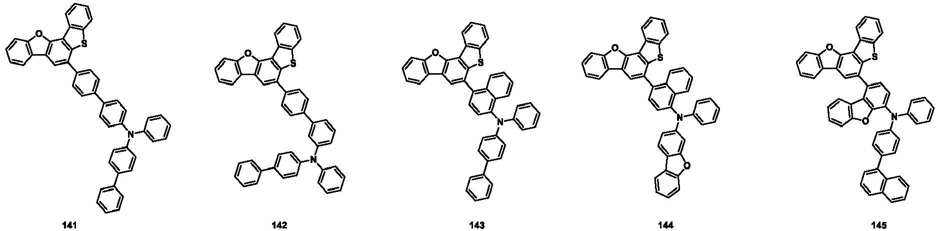

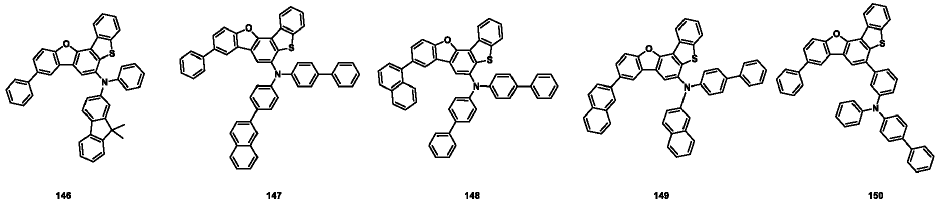

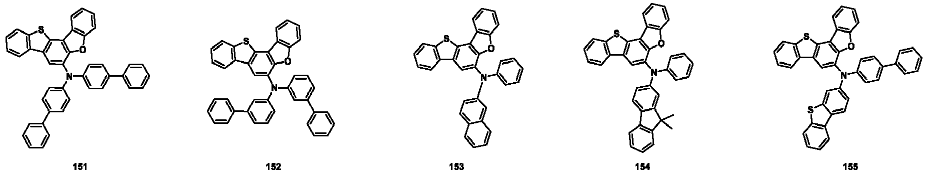

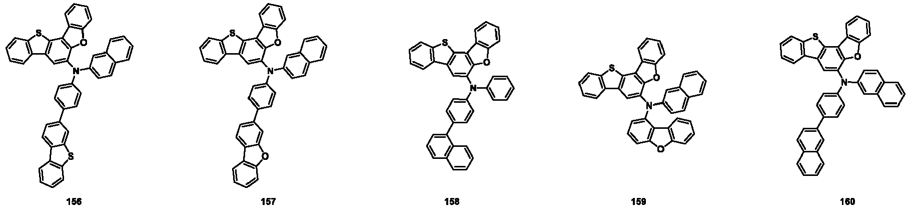

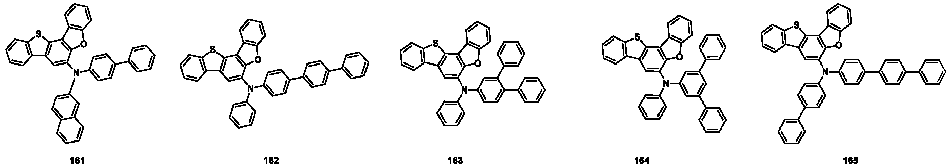

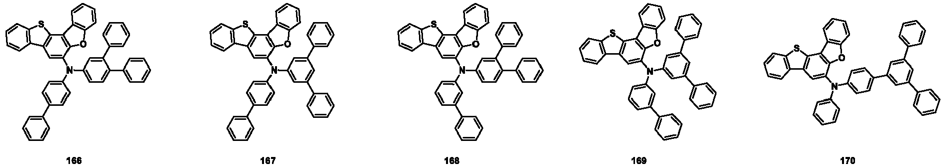

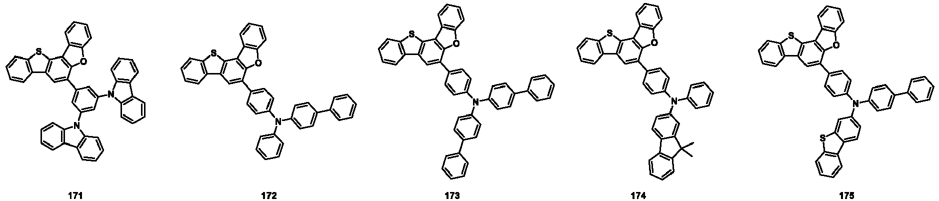

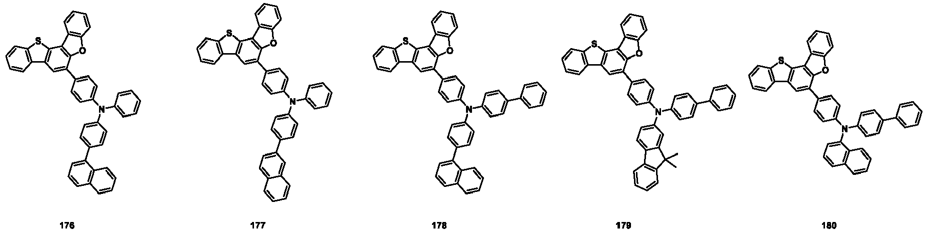

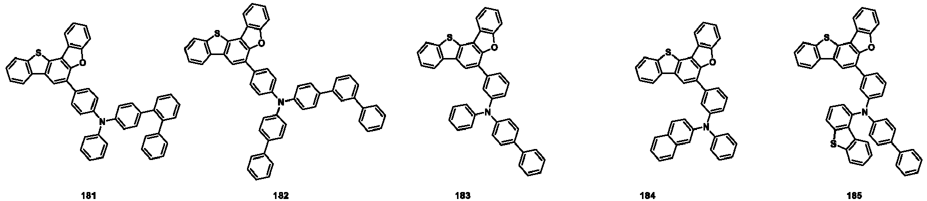

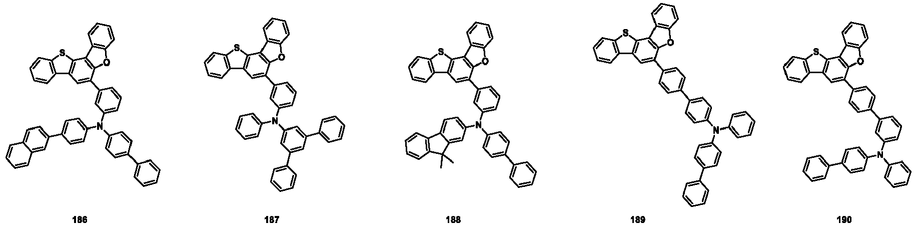

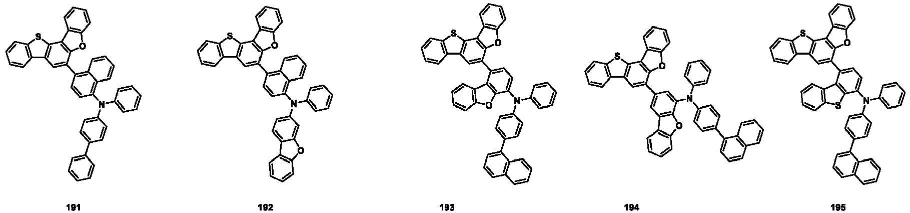

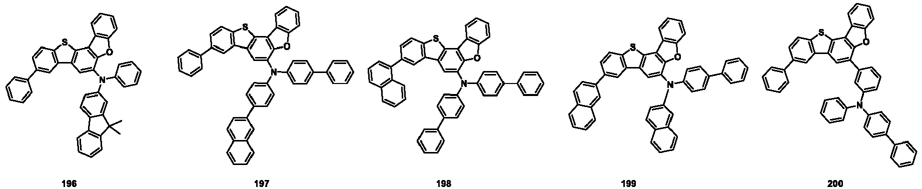

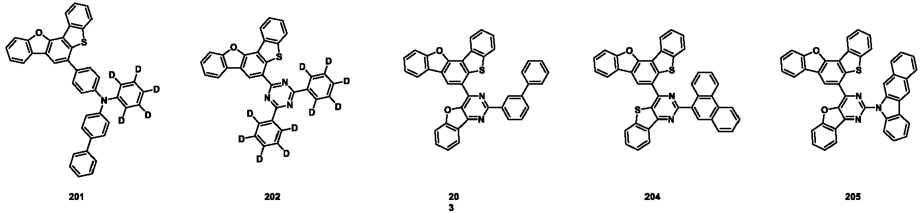

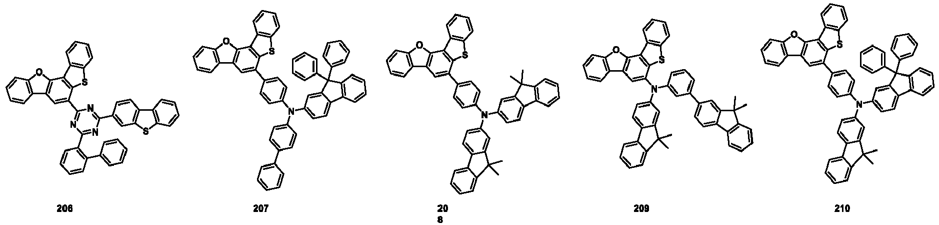

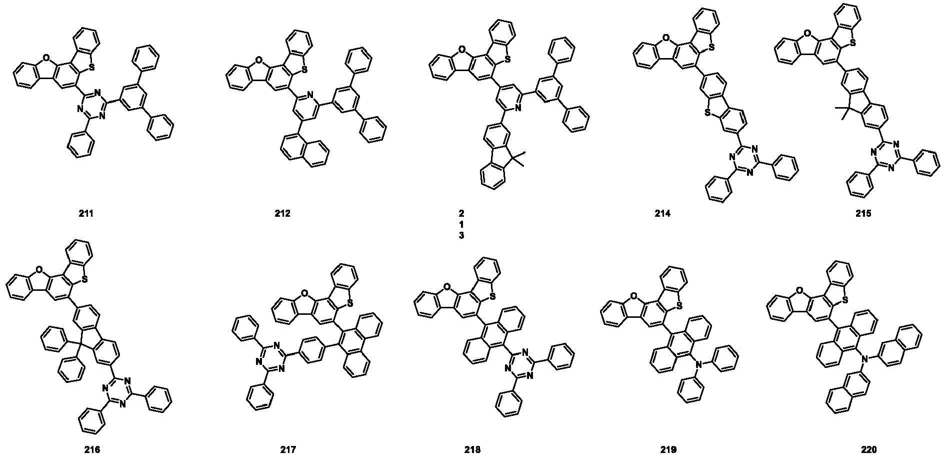

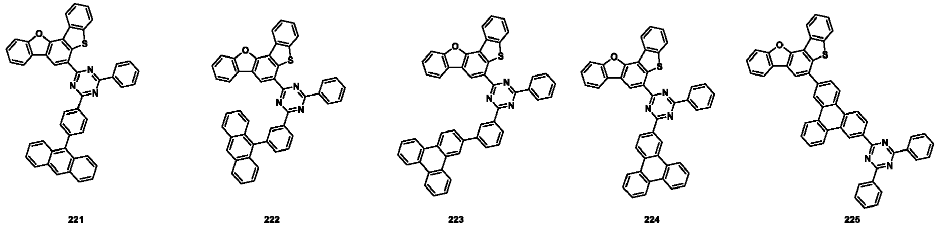

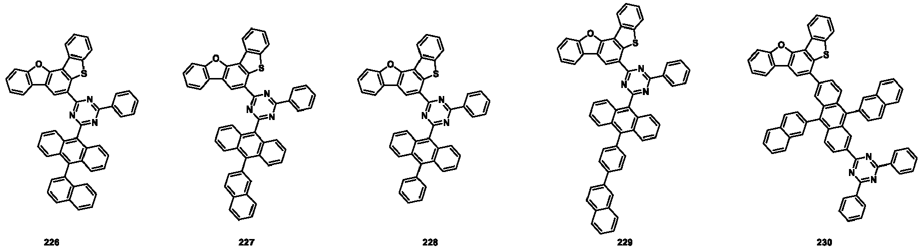

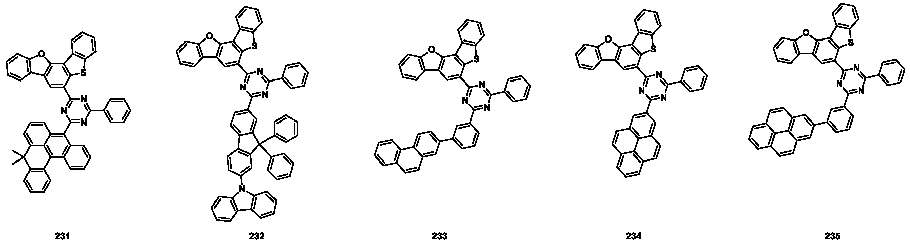

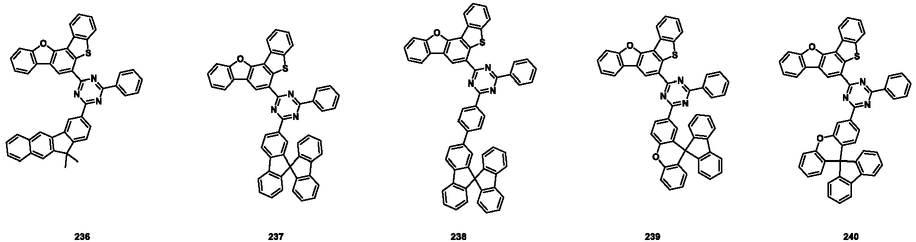

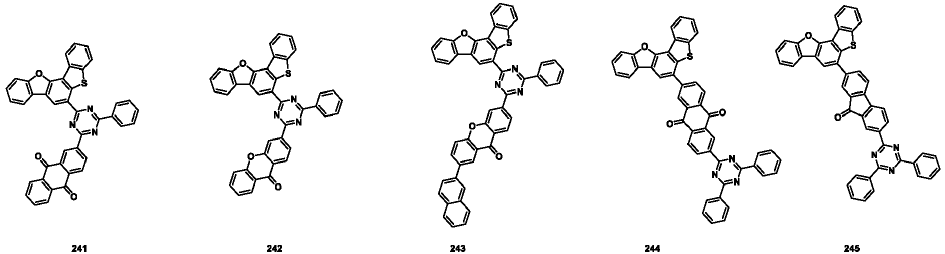

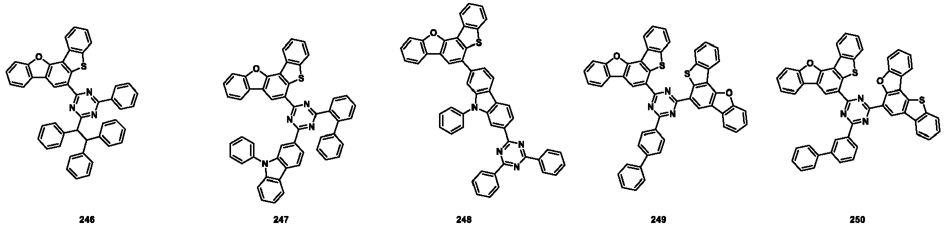

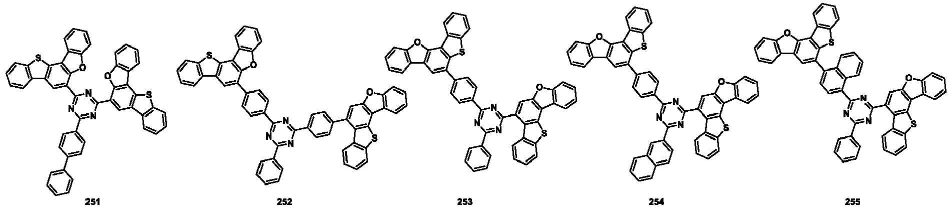

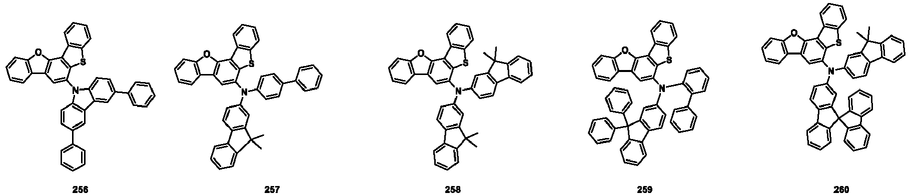

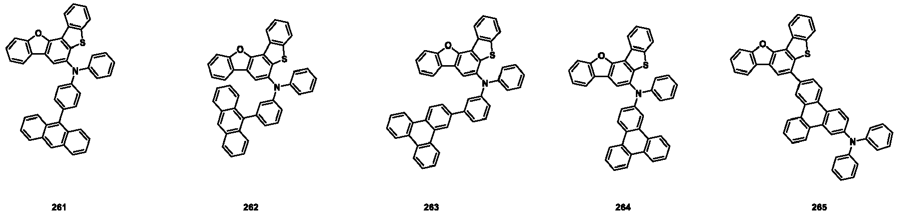

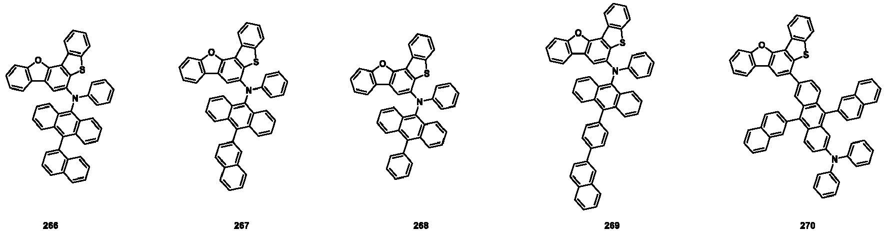

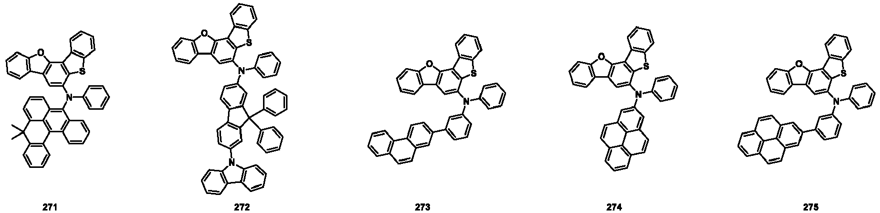

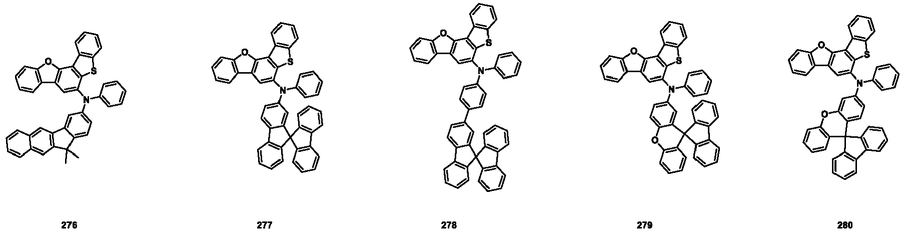

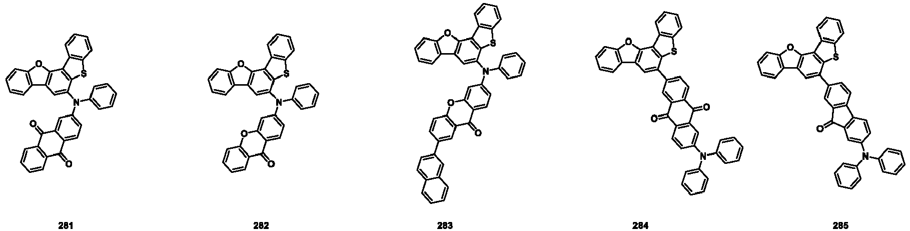

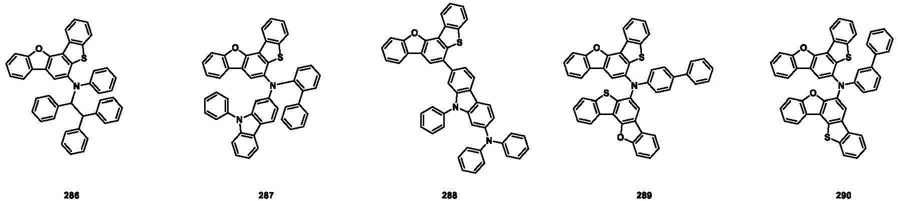

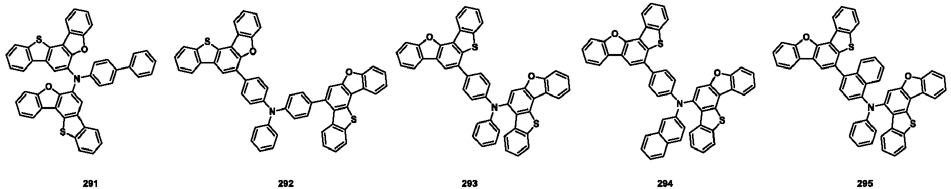

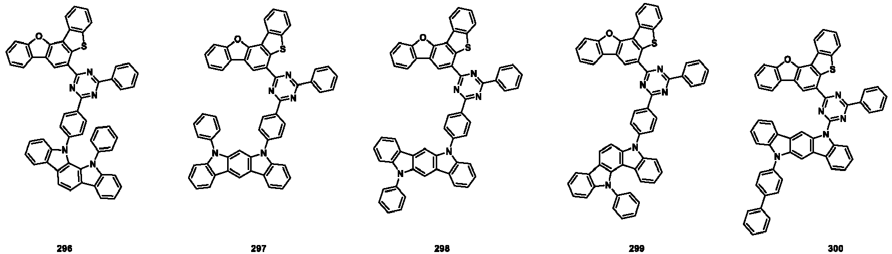

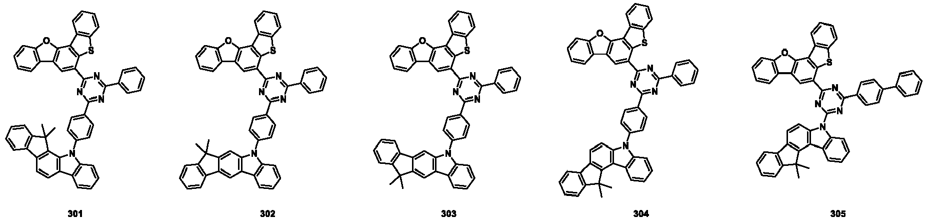

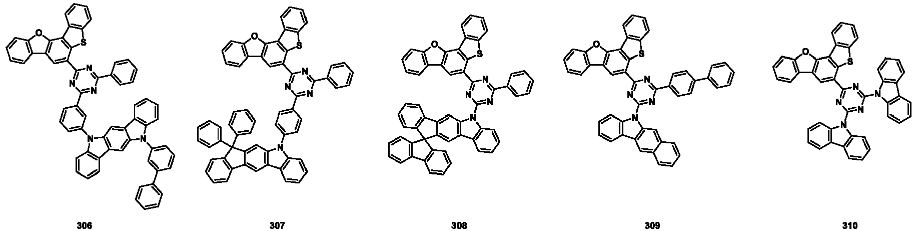

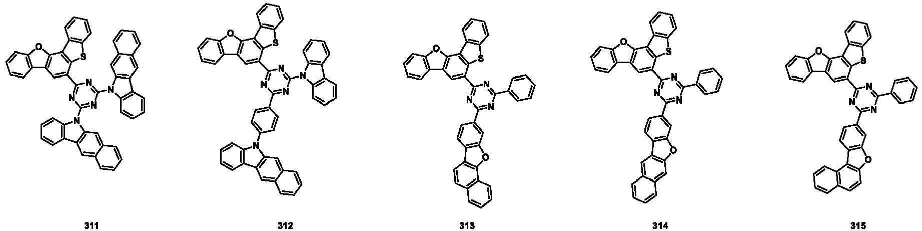

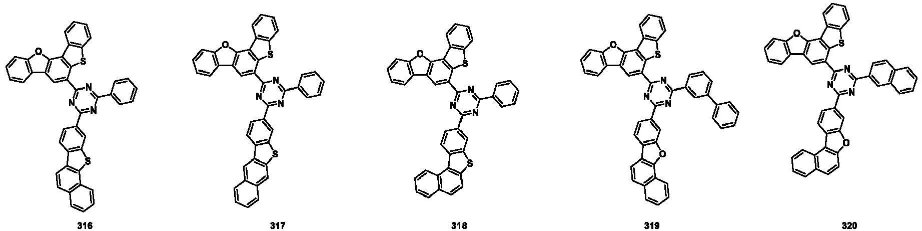

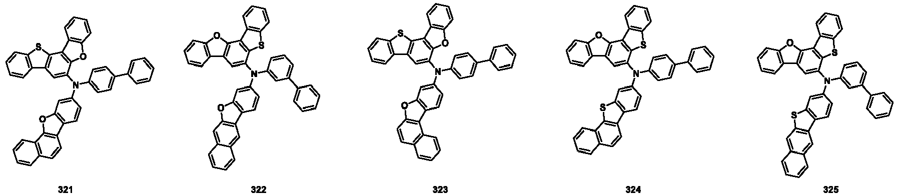

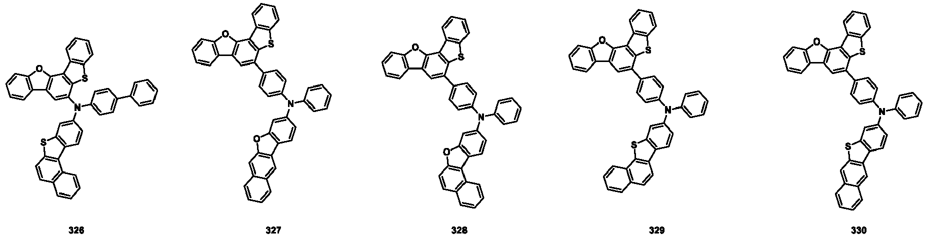

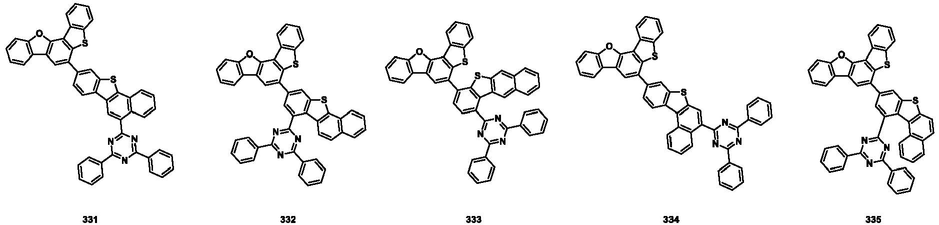

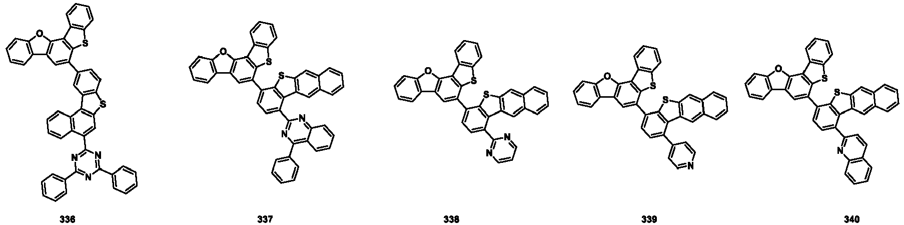

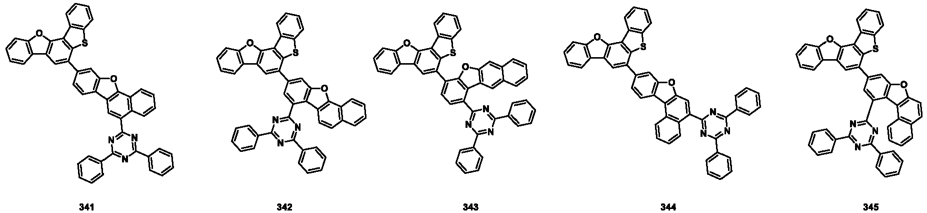

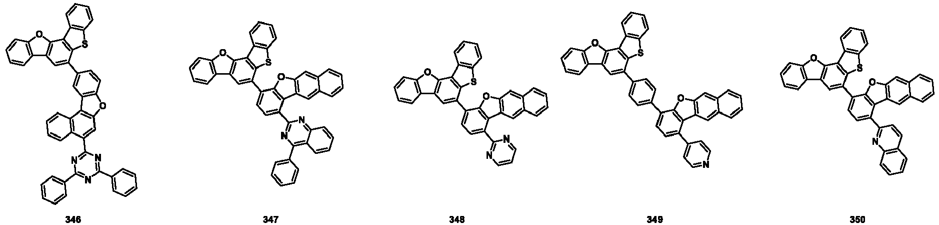

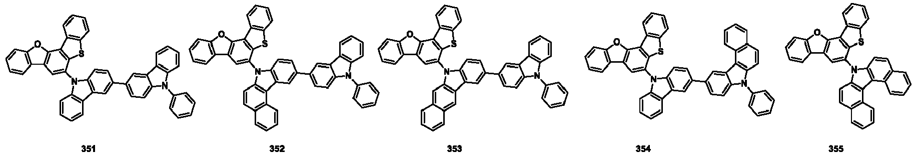

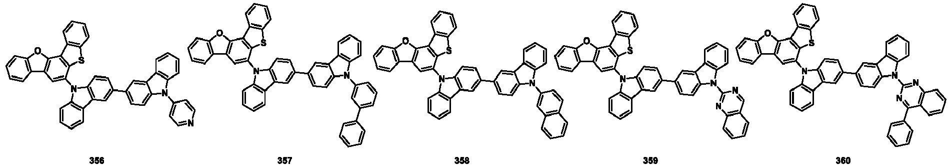

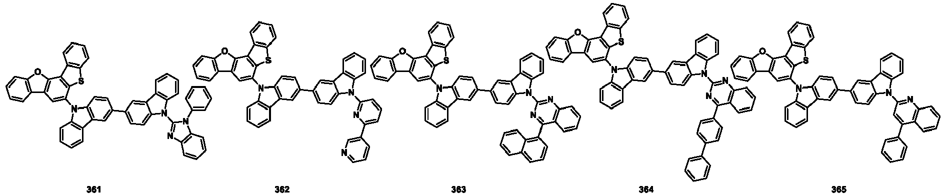

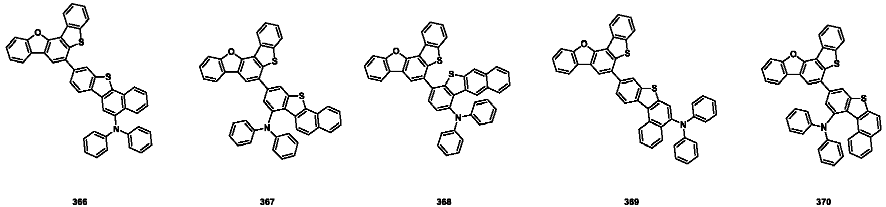

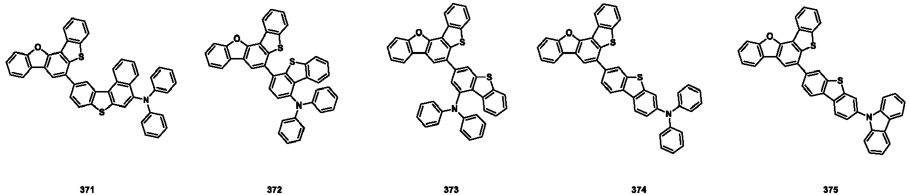

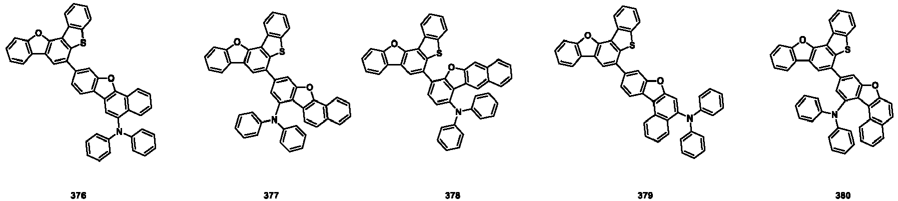

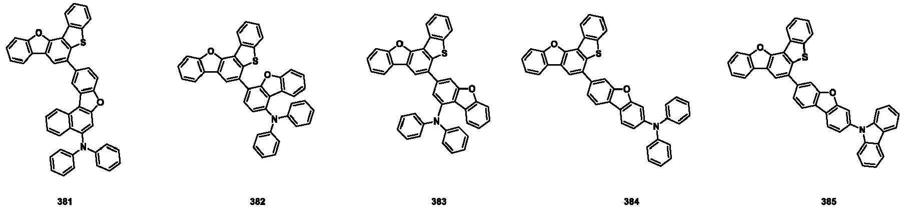

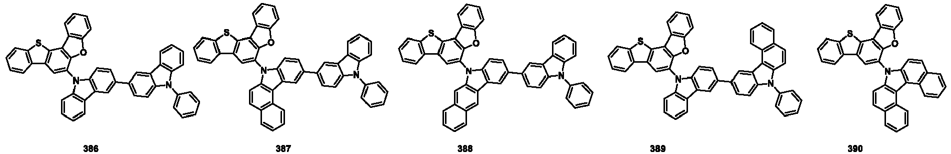

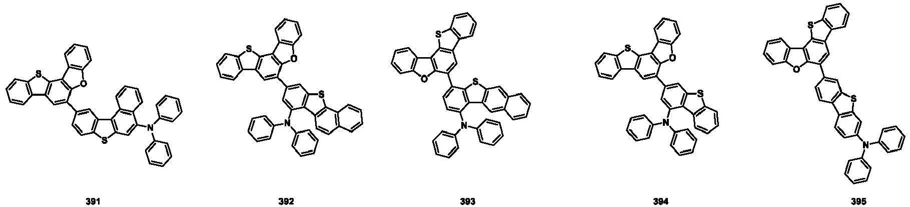

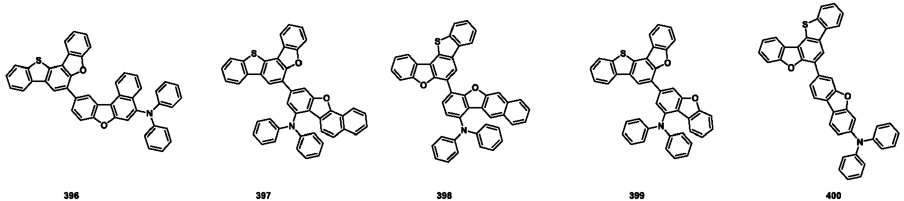

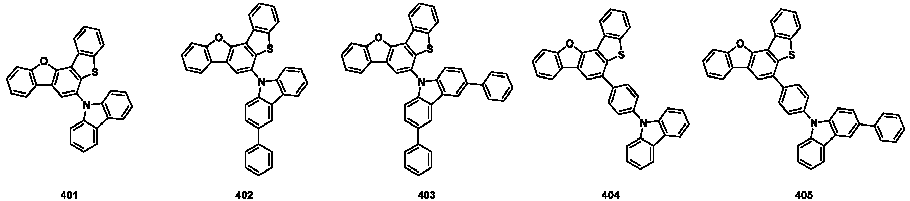

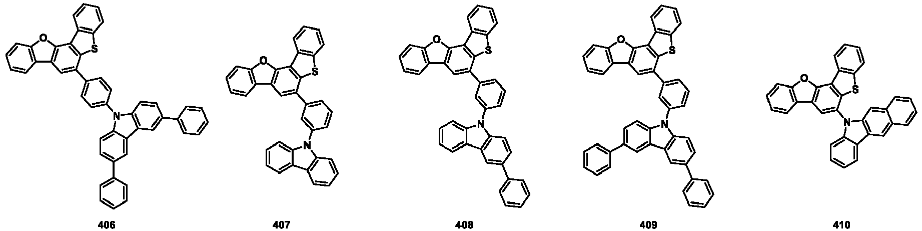

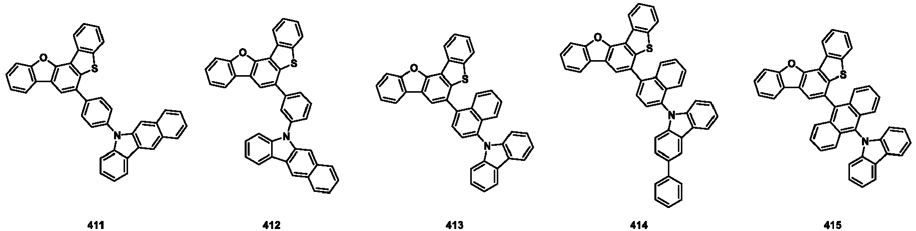

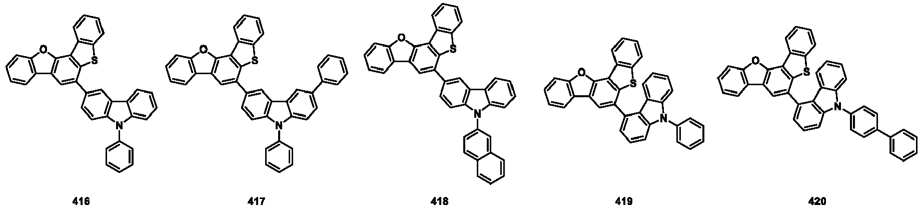

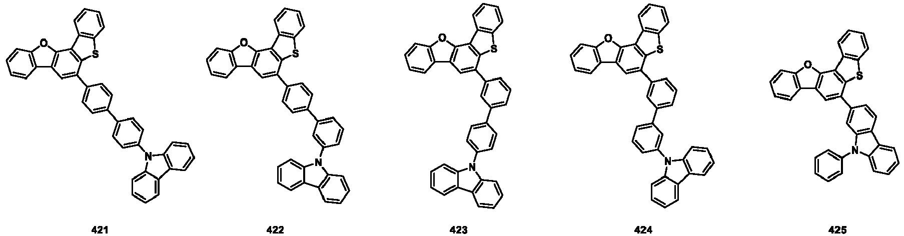

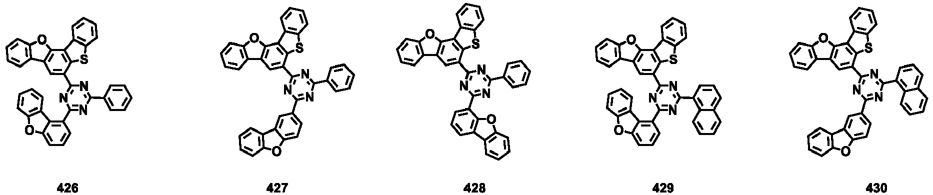

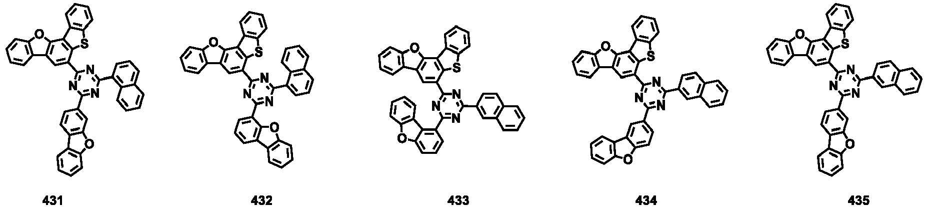

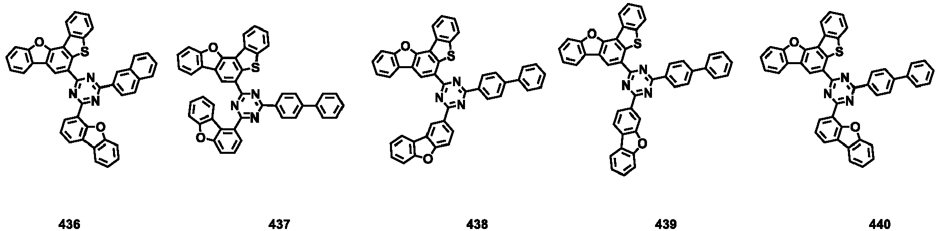

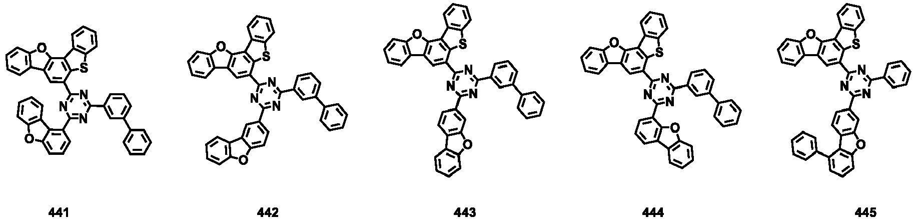

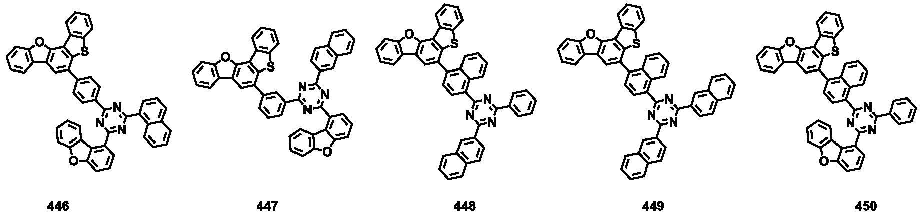

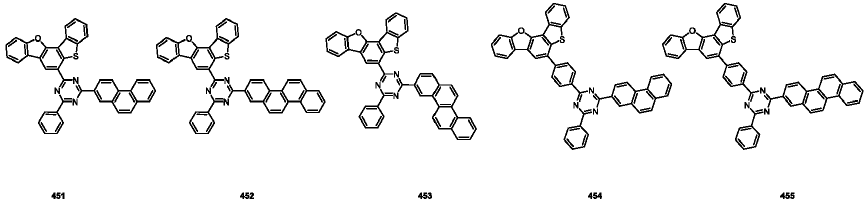

8. An organic light emitting device comprising:
a first electrode;
a second electrode; and
an organic material layer with one or more layers provided between the first electrode and the second electrode,
wherein one or more layers of the organic material layer comprise the heterocyclic compound according to any one of claims 1 to 7.

9. The organic light emitting device of claim 8, wherein the organic material layer includes a light emitting layer, and the light emitting layer comprises the heterocyclic compound.

10. The organic light emitting device of claim 8, wherein the organic material layer includes a light emitting layer, and the light emitting layer comprises the heterocyclic compound as a host material of the light emitting material.

11. The organic light emitting device of claim 8, further comprising one or more layers selected from the group consisting of a light emitting layer, a hole injection layer, a hole transport layer, an electron injection layer, an electron transport layer, an electron blocking layer, a hole auxiliary layer, and a hole blocking layer.
